Europäisches Patentamt

European Patent Office

Office européen des brevets

(19)

(11) Publication number: **0 504 914 A2**

## EUROPEAN PATENT APPLICATION

(12)

(21) Application number: **92104872.4**

(22) Date of filing: **20.03.92**

(51) Int. Cl.⁵: **G01N 33/68**, G01N 33/74

(30) Priority: **21.03.91 US 674285**
**08.07.91 US 725332**
**25.02.92 US 837814**

(43) Date of publication of application:
**23.09.92 Bulletin 92/39**

(84) Designated Contracting States:
**AT BE CH DE DK ES FR GB GR IT LI LU MC NL PT SE**

(71) Applicant: **E.R. SOUIBB & SONS, INC.**
**P.O.Box 4000**
**Princeton New Jersey 08543-4000(US)**

(72) Inventor: **Barbacid, Mariano**
**5 Stoney Creek Place**
**Lawrenceville, NJ(US)**
Inventor: **Klein, Rudiger**
**28 Linden Lane S.**
**Plainsboro, NJ(US)**

(74) Representative: **Vossius & Partner**
**Siebertstrasse 4 P.O. Box 86 07 67**
**W-8000 München 86(DE)**

(54) **Methods for detection of neuroactive substances.**

(57) Methods for the detection of agonists or antagonists of neurotrophic factors.

This invention relates to methods for detecting an agonist or an antagonist of a neutrophic factor.

The family of cell surface proteins with tyrosine protein kinase activity includes the receptors for known peptide growth factors such as insulin, IGF-I, EGF, PDGFs, FGFs, M-CSF, etc. The function of other members of this family remains to be elucidated since their putative ligands have not yet been identified. Among the latter is the *trk* subfamily of tyrosine protein kinases, [Barbacid, M., et al., Biochem. Biophys. Acta Rev. on Cancer (1991)] which so far includes products of the mamalian *trk* [Martin-Zanca, D. et al., Mol. Cell. Biol. 9, 24-33 (1989)], *trk*B [Klein, R., et al, EMBO J. 8, 3701-3709 (1989)] and *trk*C [Lamballe, F. et al., Cell 66, 967-979 (1991)] proto-oncogenes and the *Drosophila Dtrk* protein (Pulido et al., EMBO J., in press). Transforming alleles of the human *trk* locus have been identified in colon and thyroid papillary carcinomas [Martin-Zanca, D. et al., Nature 319, 743-748 (1986); Bongarzone, I., et al., Oncogene 4, 1457-1462 (1989)].

The members of the *trk* gene family appear to be preferentially expressed in neural tissues [Barbacid, M., et al., Biochem. Biophys. Acta Rev. on Cancer, (1991)]. *trk* gene transcripts have been identified in dorsal root ganglia as well as in visceral sensory ganglia such as the trigeminal, superior and jugular cranial ganglia [Martin-Zanca, D. et al., Genes Dev. 4, 683-694 (1990)]. Transcripts derived from the highly related tyrosine kinase gene *trk*B have been observed in multiple structures of the central (CNS) and peripheral (PNS) nervous system [Klein, R. et al., Cell 61, 647-656 (1990); Klein, R. et al., Development 109, 845-850 (1990)]. The *trk*B locus encodes at least two different gene products of 145,000 daltons (gp145$^{trkB}$) and 95,000 daltons (gp95$^{trkB}$) which possess identical putative extracellular domains. However, only gp145$^{trkB}$ has a cytoplasmic tyrosine kinase domain [Klein, R. et al., Cell 61, 647-656 (1990)]. Transcripts encoding this putative receptor have been detected in some of the major brain structures including the cerebral cortex and the hippocampus. In contrast, expression of its non-catalytic isoform gp95$^{trkB}$ within the brain appears to be restricted to specialized structures such as the choroid plexus and the ependymal lining of certain ventricles [Klein, R. et al., Cell 61, 647-656 (1990)]. In *Drosophila*, the *Dtrk* protein is widely expressed in the CNS and PNS (Pulido et al., EMBO J., in press), suggesting that at least some of the functions of these tyrosine kinases in the nervous system may have been conserved during evolution.

Nerve growth factor (NGF) [Levi-Montalcini, R., EMBO J. 6, 1145-1154 (1987)] is a neurotrophic factor whose receptor has not been well-characterized. NGF isolated from the male mouse submaxillary gland exists as a 7S complex formed by three subunits (designated $\alpha$, $\beta$ and $\gamma$), all of which have been molecularly characterized [Pintar, J.E., Oncogenes, Genes and Growth Factors, 103-131 (1987)]. The biologically active form of NGF appears to be the $\beta$ subunit which exists as a 26,000 d. homodimer. $\beta$NGF is synthesized as a pre-pro-molecule that undergoes subsequent proteolytic cleavage at both the amino and carboxy termini to liberate the mature hormone [Pintar, J.E., Oncogenes, Genes and Growth Factors, pp. 103-131 (1987)]. BDNF and NT-3 are structurally related to NGF. Both of them are synthesized as pre-pro-hormones and are subsequently processed in similar manner as $\beta$NGF [Barde, Y.A. et al., EMBO J. 1, 549-553 (1982); Leibrock, J. et al., Nature 341, 149-152 (1989); Hohn, A. et al., Nature 344, 339-341 (1990); Maisonpierre, P.C. et al., Science 247, 1446-1451 (1990); Jones, K.R. and Reichardt, L.F., Proc. Natl. Acad. Sci. USA 87, 8060-8064 (1990)].

Previous studies have indicated that $\beta$NGF induces rapid phosphorylation of tyrosine residues when added to NGF-responsive PC12 rat pheochromocytoma cells [Maher, P.A., Proc. Natl. Acad. Sci. USA 85, 6788-6791 (1988)]. More recently, it has been reported that high-affinity NGF receptors could be immunoprecipitated with antiphosphotyrosine antibodies [Meakin, S.G. and Shooter, E.M., Neuron 6, 153-163 (1991)]. These receptors are distinct from the previously characterized low affinity NGF receptor molecule (LNGFR) [Chao, M.V. et al., Science 232, 518-521 (1986); Johnson, D. et al., Cell 47, 545-554 (1986); Radeke, M.J. et al., Nature 325, 593-597 (1987)]. These phosphotyrosine-containing NGF receptors exhibited a molecular weight of around 140,000-150,000 [Massague, J. et al., J. Biol. Chem. 256, 9419-9424 (1981); Hosang, M. and Shooter, E.M. J. Biol. Chem. 260, 655-662 (1985)].

The present invention concerns a method for detecting an agonist or an antagonist of a neurotrophic factor comprising:

(a) contacting a *trk* family receptor with a test substance which may contain the agonist or antagonist; and

(b) determining whether the agonist or antagonist binds to the *trk* family receptor.

The present invention concerns a method for detecting an agonist or an antagonist of a neurotrophic factor comprising:

(a) contacting a *trk* family receptor with the neurotrophic factor and a test substance which may contain the agonist or antagonist; and

(b) determining whether the agonist or antagonist competes with the neurotrophic factor in binding to the *trk* family

receptor.

The present invention further concerns a method for detecting an agonist of a neurotrophic factor comprising:

(a) contacting a *trk* family receptor with a test substance which may contain the agonist; and

(b) determining whether the test substance activates one or more biological activities of the *trk* family receptor.

The present invention also concerns a method for detecting an antagonist of a neurotrophic factor comprising:

(a) contacting a *trk* family receptor with the neurotrophic factor and a test substance which may contain the antagonist; and

(b) determining whether the test substance affects the activation by the neutrophic factor of one or more of the biological activities of the *trk* receptor.

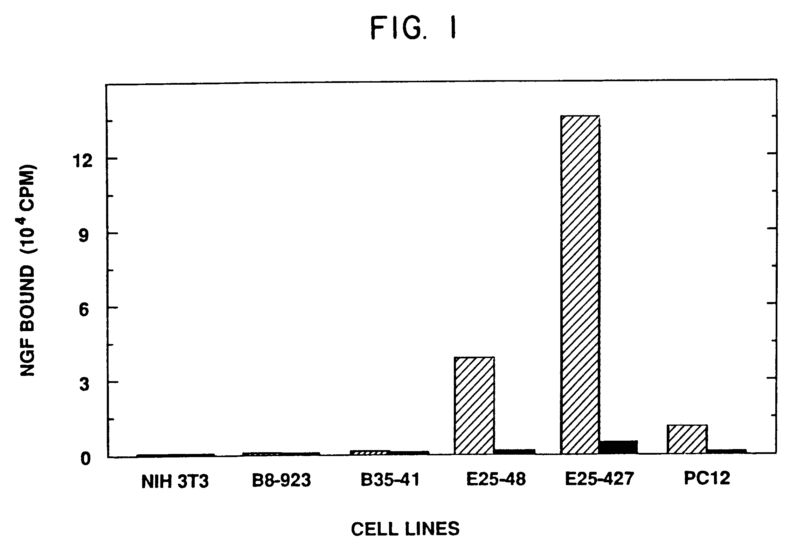

FIGURE 1. Binding of NGF to cells expressing *trk*B and *trk* tyrosine protein kinases. Cells, including NIH3T3 mouse fibroblasts, NIH3T3 cells expressing the mouse *trk*B (B8-923 and B35-41 cell lines) and human *trk* (E25-48 and E25-47 cell lines) proto-oncogenes and PC12 rat pheochromocytoma cells, were incubated with 1 nM of [$^{125}$I]- labeled $\beta$NGF at 4°C for 2 hours, with (filled bars) or without (hatched bars) the addition of 100 nM unlabeled $\beta$NGF. The unbound $\beta$NGF was removed at the end of the incubation and the radioactivity associated with the cells was determined as described herein below. The data shown here represents the average of three experiments and is expressed on the basis of the amount of [$^{125}$]-labeled $\beta$NGF associated with $10^5$ cells.

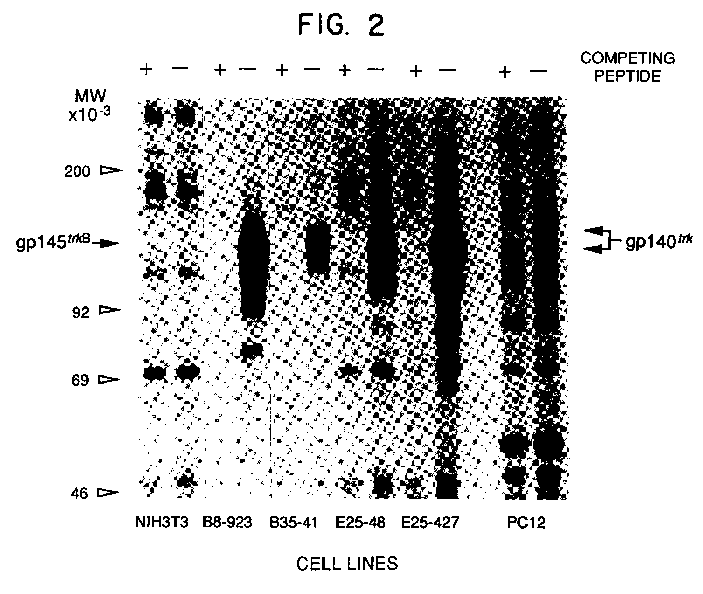

FIGURE 2. Expression levels of *trk*B and *trk* gene products. [$^{35}$S] methionine-labeled extracts of NIH3T3 cells expressing the mouse *trk*B proto-oncogene (B8-923 and B35-41 cell lines) were incubated with rabbit anti-*trk*B antibodies [Klein, R. et al., EMBO J. 8, 3701-3709 (1989)] either in the presence (+) or absence (-) of 5$\mu$g of competing peptide. [$^{35}$S] methionine-labeled extracts of normal NIH3T3 cells, NIH3T3 cells expressing the human *trk* proto-oncogene (E25-48 and E25-427 cell lines) and rat PC12 cells were incubated with rabbit anti *trk* antibodies [Martin-Zanca, D. et al., Mol. Cell. Biol. 9, 24-33 (1989)] either in the presence (+) or absence (-) of 5 $\mu$g of competing peptide. Each of the immunoprecipitation reactions contained identical amounts (2.3 x 10$^7$ cpm) of [$^{35}$S] methionine-labeled cell extracts. The resulting immunoprecipitates were analyzed by 7.5% SDS-polyacrylamide gel electrophoresis. Gel were fixed, dried and exposed to Kodak X-OMAT film for 18 hours at -70°C in the presence of intensifier screens. The migration of the mature *trk*B (gp145$^{trkB}$) and *trk* (gp140$^{trk}$) gene products are indicated by arrows. The slower electrophoretical migration of the product of the *trk* proto-oncogene in PC12 cells is likely to be due to differential glycosylation. Co-electrophoresed molecular weight markers included myosin (200,000), phosphorylase B(92,000), albumin (69,000) and ovalbumin (46,000).

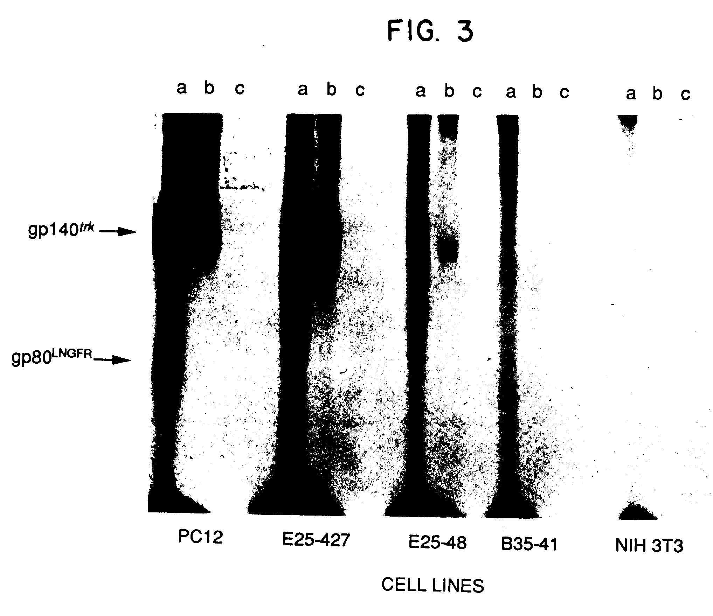

FIGURE 3. Chemical cross-linking of [$^{125}$I]-labeled $\beta$NGF. Cells, including rat PC12 cells, NIH3T3 cells expressing the human *trk* (E25-427 and E-25-48) and mouse *trk*B (B35-41) tyrosine protein kinases and parental NIH3T3 cells, were incubated with 1 nM of [$^{125}$I]-labeled $\beta$NGF at 4°C for 2 hours and cross-linked as described herein below. [$^{125}$I]-labeled cells were lysed either (a) in sample buffer and directly loaded onto 7.5% SDS-polyacrylamide gels, or (b,c) in lysis buffer and incubated with an antiserum elicited against the carboxy-terminal domain of the gp140$^{trk}$ either (b) in the absence or (c) in the presence of 5 $\mu$g of the immunizing peptide. The resulting immunoprecipitates were loaded onto 7.5% SDS-polyacrylamide gels in parallel with the corresponding [$^{125}$I]-labeled cell extracts. Gels were fixed, dried and exposed to Kodak X-OMAT film for 15 hours at -70°C in the presence of intensifier screens. The migration of the cross-linked *trk* proto-oncogene product gp140$^{trk}$ and the low affinity NGF receptor gp80$^{LNGFR}$ is indicated by arrows. It should be noted that the *trk* proto-oncogene product of PC12 cells has a slightly slower migration than that present in E25-427 and E25-48 cells (see Fig. 2).

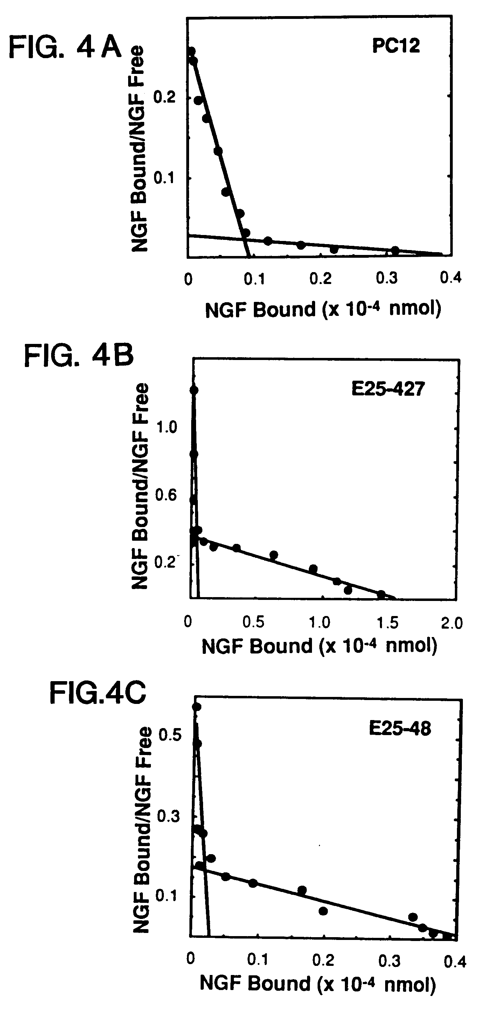

FIGURE 4. Scatchard plot analysis of the binding of [$^{125}$I]-labeled $\beta$NGF to the surface of PC12, and NIH3T3-derived E25-427 and E25-48 cells. Cells were incubated with various concentrations (0.02 nM-50 nM) of [$^{125}$I]-labeled $\beta$NGF at 4°C for 2 hours under the conditions described herein below. Nonspecific binding of [$^{125}$I]-labeled $\beta$NGF at each concentration was determined by addition of a 100-fold excess of unlabeled NGF. The values of the dissociation constants and the number of high and low affinity binding sites for each of these cell lines are summarized in Table 1.

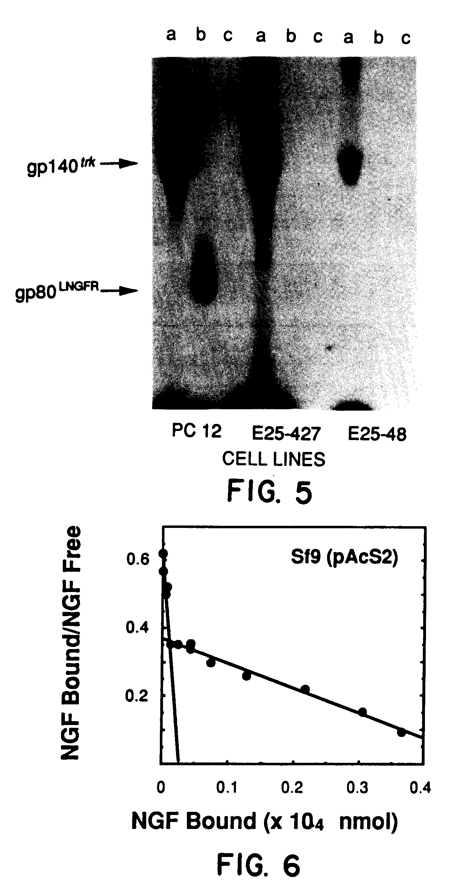

FIGURE 5. Identification of low affinity gp$^{80LNGFR}$ receptors in cells expressing the gp140$^{trk}$ tyrosine protein kinase. PC12 cells and NIH3T3-derived E25-427 and E25-48 cells were cross-linked to [$^{125}$I]-labeled $\beta$NGF as indicated in the legend to Figure 3. [$^{125}$I]-labeled cell extracts were incubated with (a) anti gp140$^{trk}$ antibodies; (b) anti rat gp80$^{LNGFR}$ antibodies; and (c) normal rabbit serum and the resulting immunoprecipitates analyzed by 7.5% SDS-polyacrylamide gel electrophoresis. Gels were fixed, dried and exposed to Kodak X-OMAT film for 2 days at -70°C in the presence of intensifier screens. The migration of gp140$^{trk}$ and gp80$^{LNGFR}$ is indicated by arrows.

FIGURE 6. Scatchard plot analysis of the binding of [$^{125}$I]-labeled $\beta$NGF to the surface of Sf9 cells expressing the human *trk* proto-oncogene. Binding of $\beta$NGF was performed as described in the legend to Figure 4. A total of 31,200 high affinity ($K_d = 0.42$nM) and 61,000 low affinity ($K_d$ = 13.7 nM) NGF receptors were identified (see Table 1).

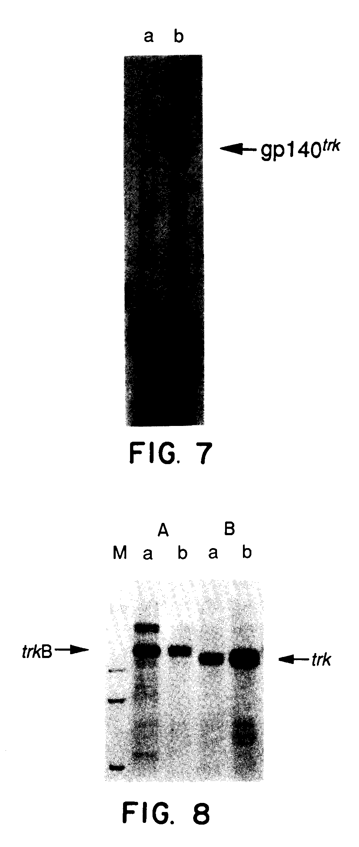

FIGURE 7. NGF-induced tyrosine phosphorylation of the endogenous *trk* proto-oncogene product of PC12 cells. PC12 cells were incubated at 37°C for 4 minutes either (a) without or (b) with 100 ng/ml of $\beta$NGF, lysed, and immunoprecipitated with rabbit anti *trk* antibodies. The resulting immunoprecipitates were separated by 7.5% SDS-polyacrylamide gel electrophoresis, blotted onto nitrocellulose and incubated with rabbit anti-phosphotyrosine antibodies as described in Experimental Procedures. Filters were exposed to Kodak X-OMAT film for 24 hours at -70°C in the presence of intensifier screens. The migration of gp140$^{trk}$ is indicated by an arrow.

FIGURE 8. Effect of $\beta$NGF on the tyrosine protein kinase activity of the *trk* proto-oncogene product. Membrane fractions isolated from Sf9 insect cells infected with recombinant baculoviruses capable of directing the expression of (A) *trk*B (pAcS1) and (B) *trk* (pAcS2) genes were immunoprecipitated with specific antibodies and the resulting immunoprecipitates incubated with [$\gamma^{32}$P]-labeled ATP in either the (a) absence or (b) presence of 1 $\mu$g/ml of $\beta$NGF as described herein below. Samples were analyzed by 8% SDS-polyacrylamide gel electrophoresis. Gels were fixed, dried and exposed to Kodak X-OMAT film for 2 hours at -70°C in the presence of intensifier screens. The migration of the mouse *trk*B and the human *trk* proto-oncogene products is indicated by arrows. It should be noted that these proteins exhibit lower apparent molecular weight in insect Sf9 cells (115,000 for the *trk*B protein and 106,000 for the *trk* protein) than in mammalian cells (145,000 for the *trk*B protein and 140,000 for the *trk* protein), presumably due to differential glycosylation. Molecular weight markers (lane M) were those described in the legend to Figure 2.

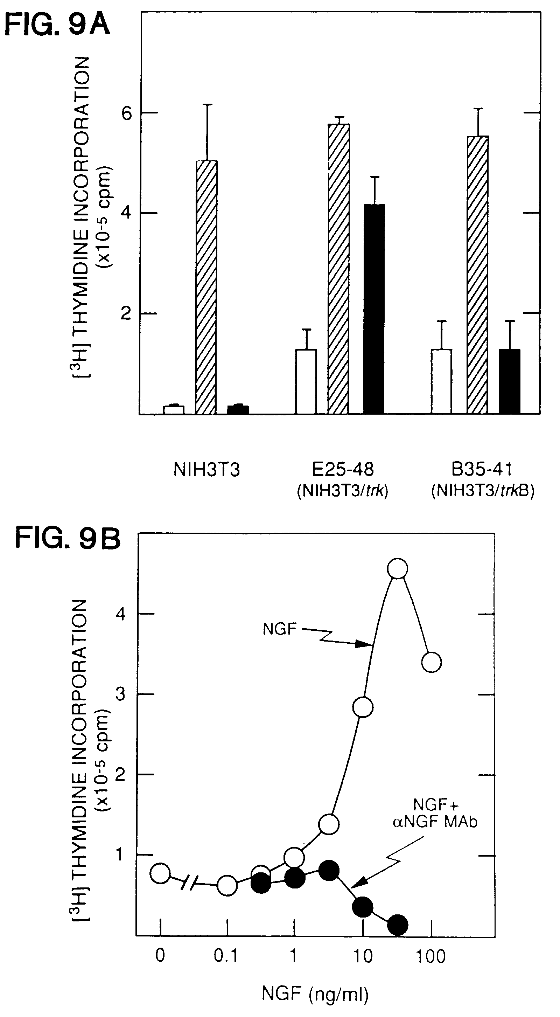

FIGURE 9. Stimulation of [$^{3}$H] thymidine incorporation by NGF. A) Quiescent NIH3T3, E25-48 and B35-41 cells were incubated with DMEM containing 5 $\mu$g/ml of insulin (open bars), 20% calf serum (hatched bars) or 30 ng/ml of NGF + 5 $\mu$g/ml of insulin (filled bars). Results are expressed as the mean of three independent experiments assayed in duplicate (+SEM). (B) E25-48 cells were stimulated by the indicated amounts of NGF either in the absence (open circles) or presence (closed circles) of a 10-fold molar excess of anti-NGF monoclonal antibody clone 27/21 (Boehringer Mannheim).

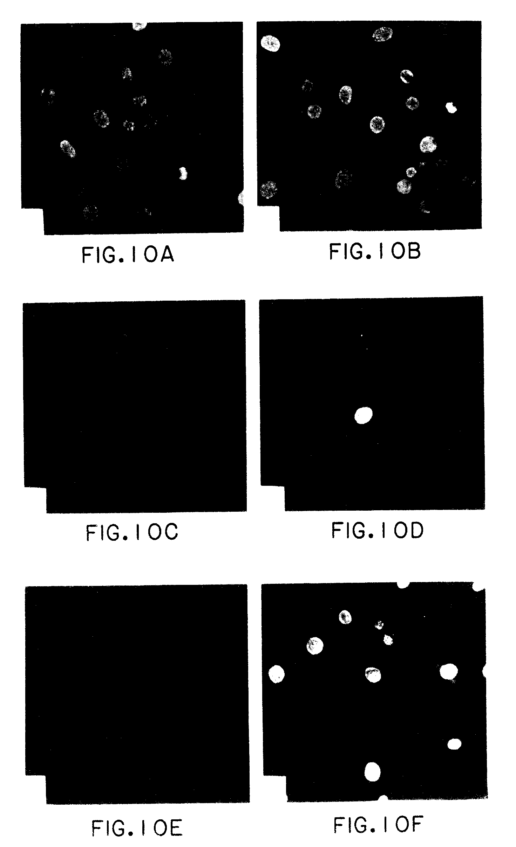

FIGURE 10. Induction of DNA synthesis by NGF. Quiescent NIH3T3 (A,C,E) and E25-48 (B,D,F) cells were incubated in the presence of 100 $\mu$M 5-bromodeoxyuridine (BrdURd) plus either 20% calf serum (A,B); 5$\mu$g/ml of insulin (C,D); or 10 ng/ml of NGF (E,F). Cells were fixed after 24 hours and analyzed for immunofluorescence using a mouse anti-BrdUrd monoclonal antibody and donkey anti-mouse IgG conjugated with Texas-Red (400x magnification).

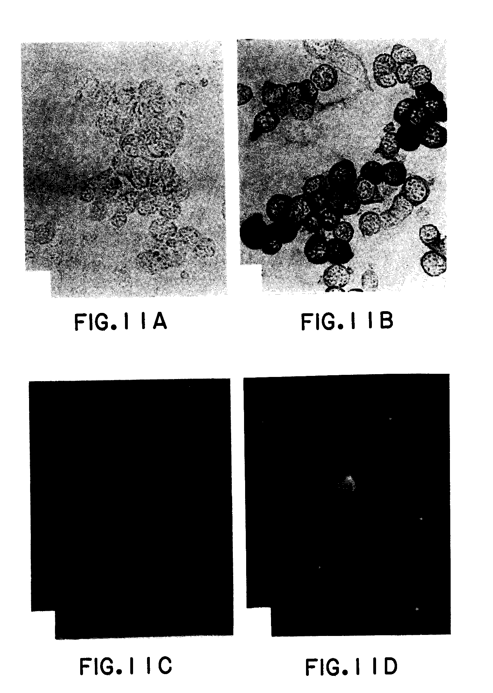

FIGURE 11. Reponsiveness to NGF correlates with expression of the *trk* proto-oncogene product. E25-48 cells were assayed for BrdUrd incorporation as described in the legend to Figure 10 and the results photographically recorded. Microslides containing BrdUrd-stained E25-48 cells were then used to immunocytochemically visualize gp140$^{trk}$ expression using the avidin-biotin immunoperoxidase technique. (A) preimmune rabbit serum, (B,C) rabbit polyclonal anti-*trk* antiserum (D) BrdUrd incorporation in the same field as shown in C. (A,B 400 x magnification; C,D 600 x magnification). Asterisks indicate cells without (C) detectable gp140*trk* expression and (D) BrdUrd incorporation.

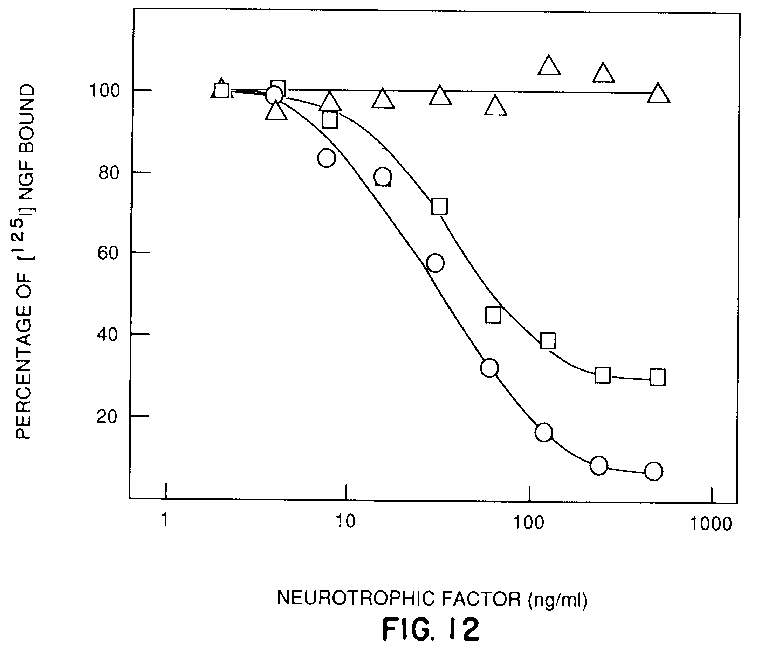

FIGURE 12. NT-3 competes with [$^{125}$I]-labeled NGF for binding to the *trk* receptors expressed in E25-48 cells. E25-48 cells were pre-incubated with unlabeled NGF (open circles), NT-3 (open squares), or BDNF (open triangles) at 4°C for 1 hour. [$^{125}$I]-labeled NGF bound to the cells was determined as described herein below in Experimental Procedures (Example 2). Results are expressed as the mean of duplicate samples.

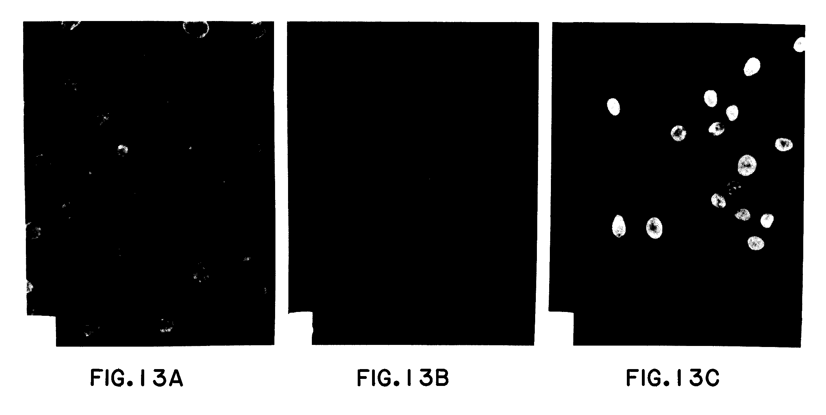

FIGURE 13. NT-3 induces DNA synthesis in E25-48 cells. The mitogenic activity of NT-3 was measured by immunofluorescence after BrdUrd incorporation. Cells were stimulated with DMEM containing (A) 20% calf serum, (B) 5 $\mu$g/ml insulin, or (C) 50 ng/ml NT-3 (400x magnification).

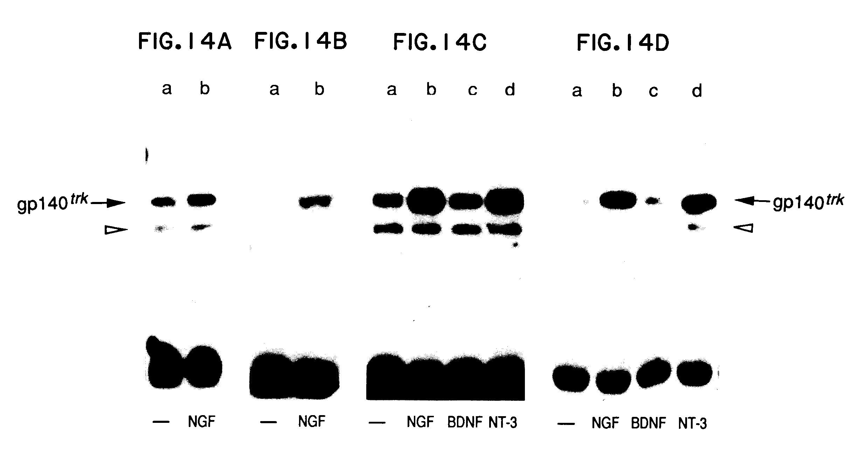

FIGURE 14. NGF and NT-3 neurotrophic factors stimulate tyrosine phosphorylation of the *trk* proto-oncogene product. Quiescent E25-48 cells were incubated for 10 minutes at 37°C with (a) DMEM alone or with DMEM containing 50 ng/ml of (b) NGF, (c) BDNF, or (d) NT-3. Cells were lysed in RIPAE buffer containing 0.5 mM sodium orthovanadate, immunoprecipated with a rabbit polyclonal antibody elicited against a peptide corresponding to the carboxy terminal domain of gp140$^{trk}$ [Martin-Zanca, D. et al., Mol. Cell. Biol. 9, 24-33(1989)], and analyzed by 8% SDS-polyacrylamide gel electrophoresis. The electrophoresed̄ samples were transferred to nitrocellulose filters and blotted with (A) anti-trk antiserum of (B,C,D) anti-phosphotyrosine monoclonal antibody 4G10(UBI) as described herein below in Experimental Procedures (Example 2). Panel C depicts tyrosine phosphorylation of gp140$^{trk}$ after stimulation with baculovirus-expressed BDNF and NT-3, Panel D after stimulation with COS cell expressed BNDF and NT-3. The resulting nitrocellulose filters were exposed to Kodak X-OMAT film for 8 hours at 70°C with the help of an intensifier screen. Co-electrophoresed molecular weight markers include myosin (200,000), phosphorylase B (92,000), albumin (69,000) and ovalbumin (46,000).

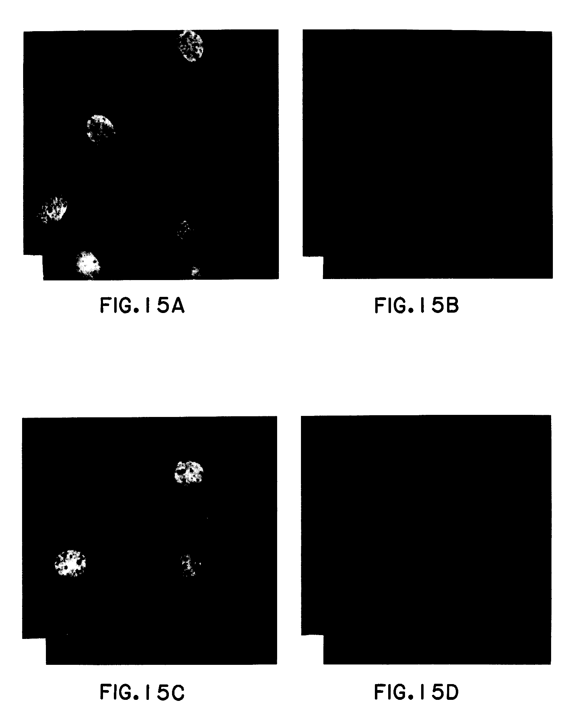

FIGURE 15. NGF and NT-3 induce expression of c-Fos protein in E25-48 cells. Quiescent E25-48 cells were incubated at 37°C for 90 minutes in DMEM containing either (A) 20% calf serum, (B) 5 $\mu$g/ml insulin, (C) 50 ng/ml NGF, or (D) 50 ng/ml NT-3. After fixation in methanol, immunofluorescence was performed using anti c-Fos specific antibodies and anti-rabbit IgG conjugated with rhodamine (600x magnification).

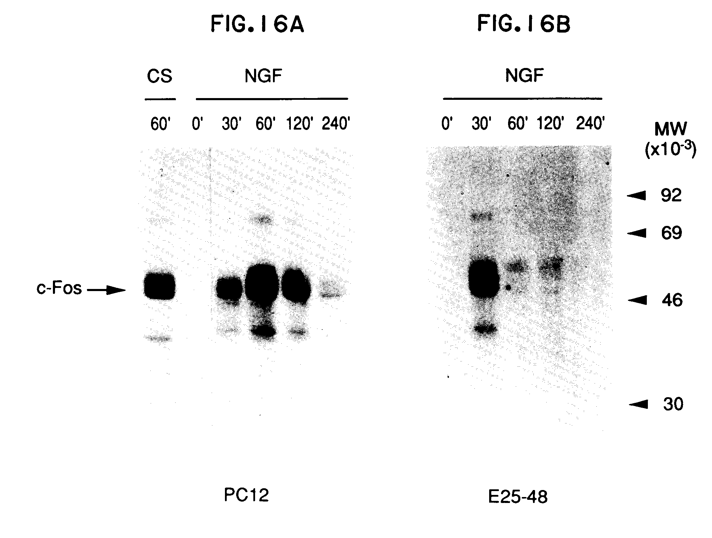

FIGURE 16. Kinetics of c-Fos induction by NGF. Quiescent (A) PC12 and (B) E25-48 cells were stimulated with 100 ng/ml of NGF for the indicated times, pulse labeled for 30 minutes with 400 $\mu$Ci/ml of [$^{35}$S]-labeled methionine and the cell lysates immunoprecipitated with rabbit polyclonal anti-c-Fos antibodies. Immunoprecipitates were analyzed by 12% SDS-polyacrylamide gel electrophoresis. Gels were subjected to fluorography, dried and exposed to Kodak X-OMAT film for 3 days with the help of an intensifier screen. Molecular weight markers are those described in the legend to Figure 14.

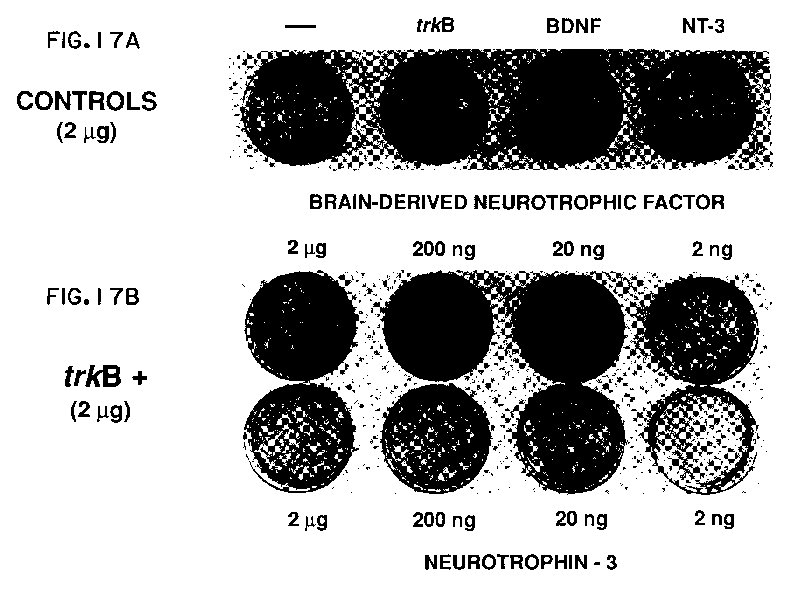

FIGURE 17. Transformation of NIH3T3 cells by co-transfection of *trk*B with BDNF or NT-3. NIH3T3 cells were transfected with saturating amounts (2$\mu$g) of the *trk*B kinase expressing plasmid pFRK44 either in the absence or presence of various amounts (2 $\mu$g to 2 ng) of pLL42 (BDNF) or pLL43 (NT-3) expression plasmids. As controls, 2 $\mu$g of pLL42 and pLL43 DNAs were co-transfected in the absence of pFRK44 DNA. Foci of transformed cells were counted under the microscope 12 days after transfection (Table 3) and the plates subsequently stained with Giemsa.

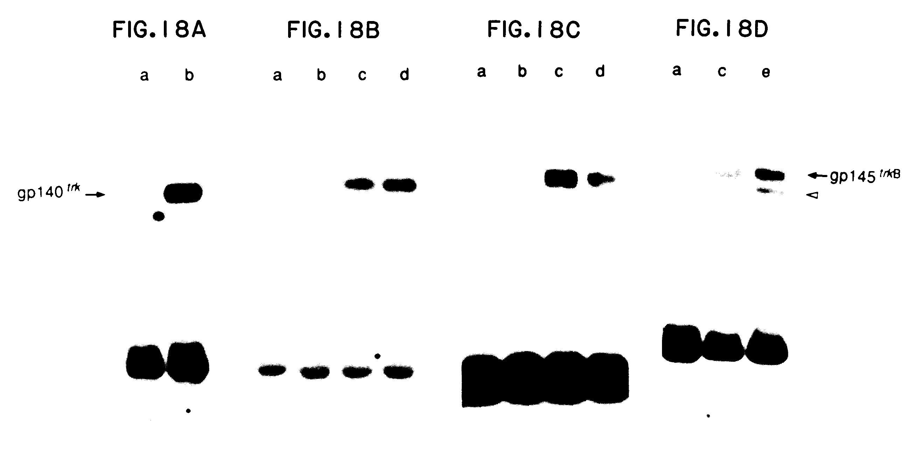

FIGURE 18. BDNF and NT-3 stimulate phosphorylation of tyrosine residues in the *trk*B tyrosine kinase receptor gp145$^{trkB}$. (A) gp140$^{trk}$-expressing E25-48 cells incubated in the (a) absence or (b) presence of 50 ng/ml of NGF. (B) gp145$^{trkB}$-expressing Z52-17 cells incubated with (a) 1:10 dilution of supernatant from mock-transfected COS cells; (b) 50 ng/ml of NGF; (c) 1:10 dilution of supernatant derived from COS cells transfected with pVN1 (BDNF); or (d) 1:10 dilution of supernatant derived from COS cells transfected with pVN2 (NT-3). (C) gp145$^{trkB}$-expressing Z52-17 cells incubated with (a) supernatant from Sf9 cells infected with wild type baculovirus; or 50 ng/ml of either (b) NGF; (c) baculovirus-derived BDNF or (d) baculovirus-derived NT-3. (D) (a,c) Z52-17 cells incubated with (a) supernatant from Sf9 cells infected with wild type baculovirus; or (c) 50 ng/ml of baculovirus-derived BDNF. (D) (e) Z82-41 cells transformed by co-transfection of pFRK44 (*trk*B) and pLL42 (BDNF) DNAs. All incubations were performed for 10 minutes at 37°C. Cells were lysed and incubated with rabbit polyclonal antibodies elicited against (A) gp140$^{trk}$ [Martin-Zanca, D. et al., Mol. Cell.Biol. 9, 24-33 (1989)] or (B,C,D) gp145$^{trkB}$ [Klein, R. et al., Cell. 61, 647-656 (1990)]. Immunoprecipitates were analyzed by 8% SDS-polyacrylamide gel electrophoresis, transferred to nitrocellulose filters and blotted with the anti-phosphotyrosine monoclonal antibody 4G10 (UBI) as described herein below in Experimental Procedures (Example 3). Filters were exposed to Kodak X-Omat film for 24 hours at -70°C with the help of an intensifier screen. The migration of gp140$^{trk}$ and gp145$^{trkB}$ receptors is indicated by arrows. The open arrow indicates the migration of a phosphorylated precursor of gp145$^{trkB}$.

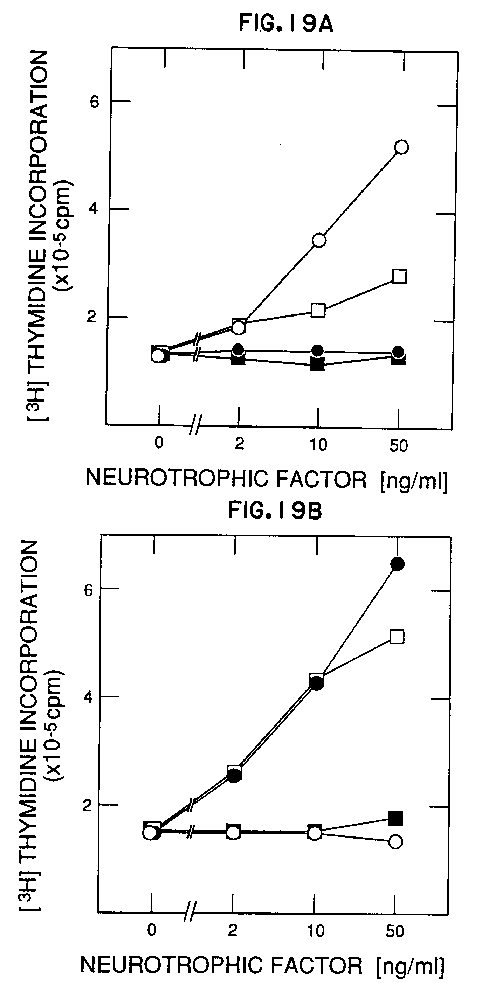

FIGURE 19. Stimulation of [$^3$H]-labeled thymidine incorporation by BDNF and NT-3. Quiescent (A) gp140$^{trk}$-expressing E25-48 cells and (B) gp145$^{trkB}$-expressing Z52-17 cells were incubated in DMEM containing 5 ug/ml of insulin and the indicated amounts of NGF (open circles); baculovirvs-derived BDNF (closed circles); baculovirus-derived NT-3 (open squares), or supernatant from wild-type baculovirus-infected Sf9 insect cells (closed squares). Incorporation of [$^3$H]-labeled thymidine into DNA was measured in duplicate samples 22 hours after stimulation as described herein below in Experimental Procedures (Example 3). Values are not corrected for DNA synthesis in untreated resting cultures.

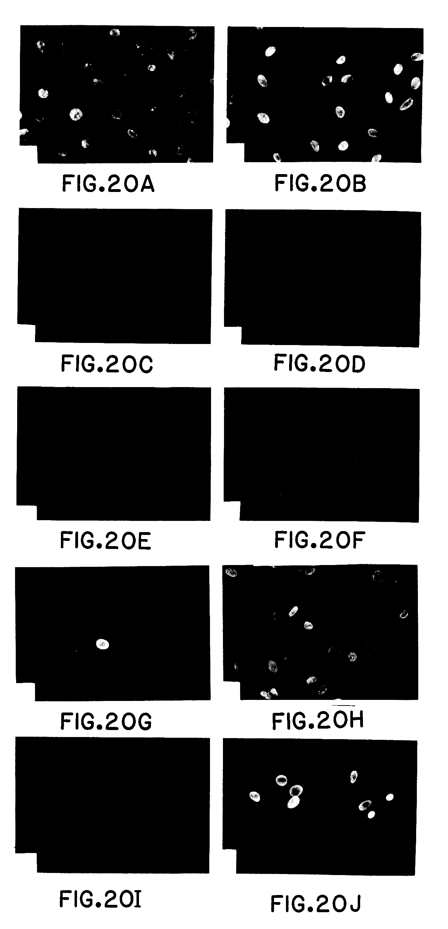

FIGURE 20. Induction of DNA synthesis by BDNF and NT-3. Quiescent (A, C, E, G, I) NIH3T3 and (B, D, F, H, J) Z52-17 cells were incubated in the presence of 100 $\mu$M BrdUrd in the presence of (A, B) 20% calf serum; (C, D) 5 $\mu$g/ml of insulin; (E, F) 50 ng/ml of NGF; (G, H) 1:10 dilution of supernatants derived from COS cells expressing BDNF; or (I, J) 1:10 dilution of supernatants derived from COS cells expressing NT-3. Cells were fixed after 24 hours and analyzed for immunofluorescence using a mouse anti-BrdUrd monoclonal antibody and donkey anti-mouse IgG conjugated with Texas-Red (400 x magnification).

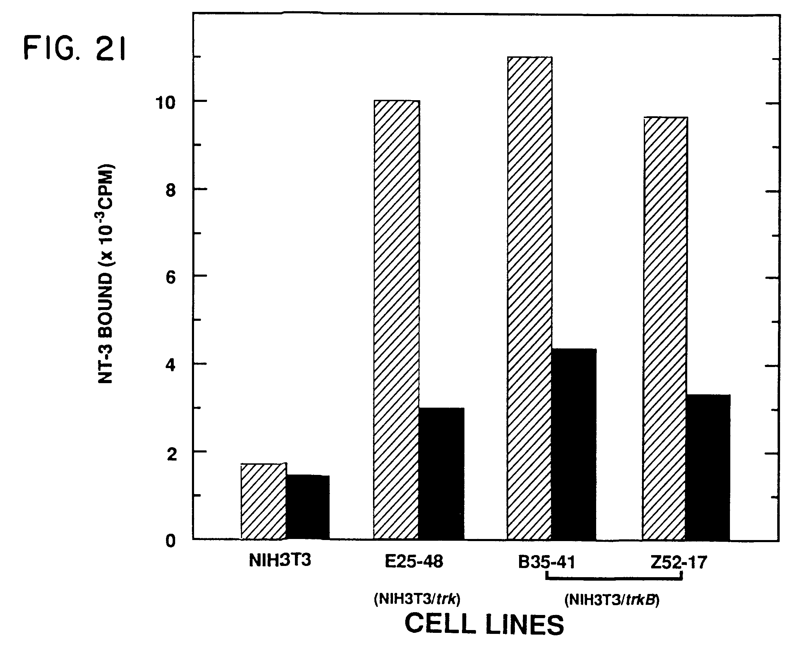

FIGURE 21. Binding of NT-3 to cells expressing *trk* and *trk*B tyrosine protein kinases. Cells, including parental NIH3T3 cells and NIH3T3 cells expressing the human *trk* (E25-48) and mouse *trk*B (B35-41, Z52-17) proto-oncogenes were incubated with 1 nM [$^{125}$I]-labeled NT-3 at 4°C for 2 hours in the absence (hatched bars) or presence (filled bars) of 75 nM of purified unlabeled NT-3. Unbound NT-3 was removed at the end of the incubation, and the radioactivity associated with the cells was measured as described herein below in Experimental Procedures (Example 3). Results are expressed as the mean of duplicate samples.

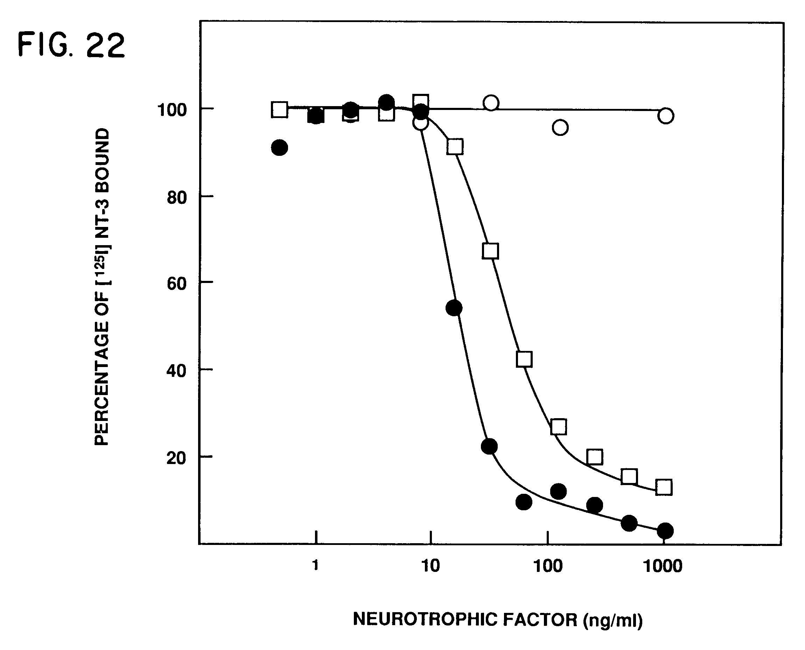

FIGURE 22. BDNF competes with [$^{125}$I]-labeled NT-3 for binding to gp145$^{trkB}$ receptors. B35-41 cells were pre-incubated with unlabeled NGF (open circles), purified NT-3 (open squares), or purified BDNF (filled circles) at 4°C for 1 hour. [$^{125}$I]-labeled NT-3 was added to a final concentration of 5 ng/ml and the cells incubated for an additional 2 hours. The percentage of [$^{125}$I]-labeled NT-3 bound to the cells was determined as described herein below in Experimental Procedures (Example 3). Non-specific binding to control NIH3T3 cells (<10%) was subtracted from the results presented. The data shown here represents the average of two separate experiments with duplicate samples.

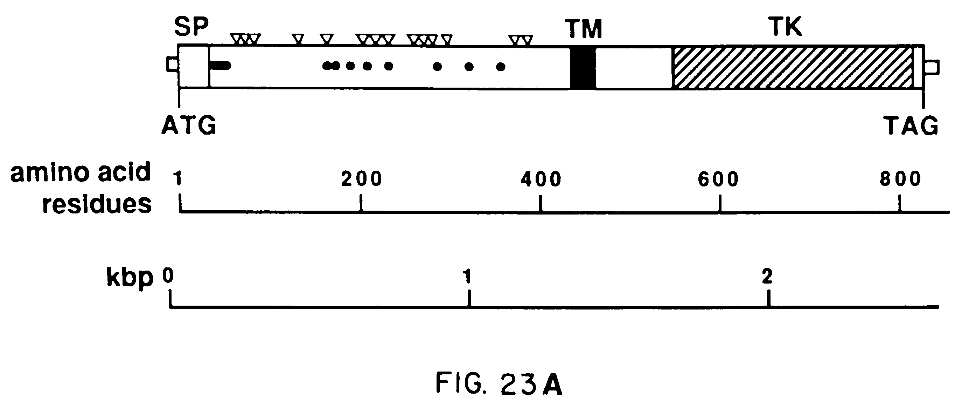

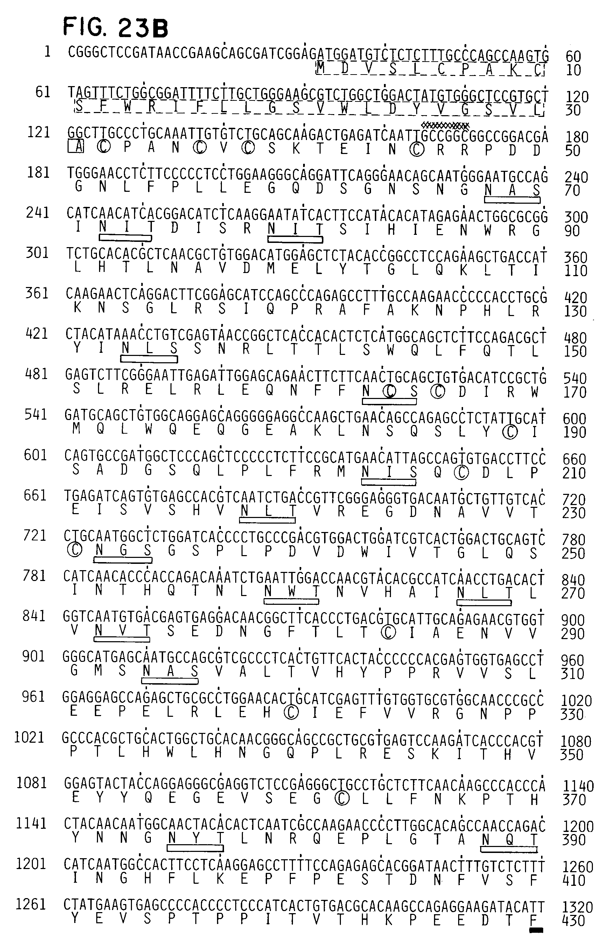

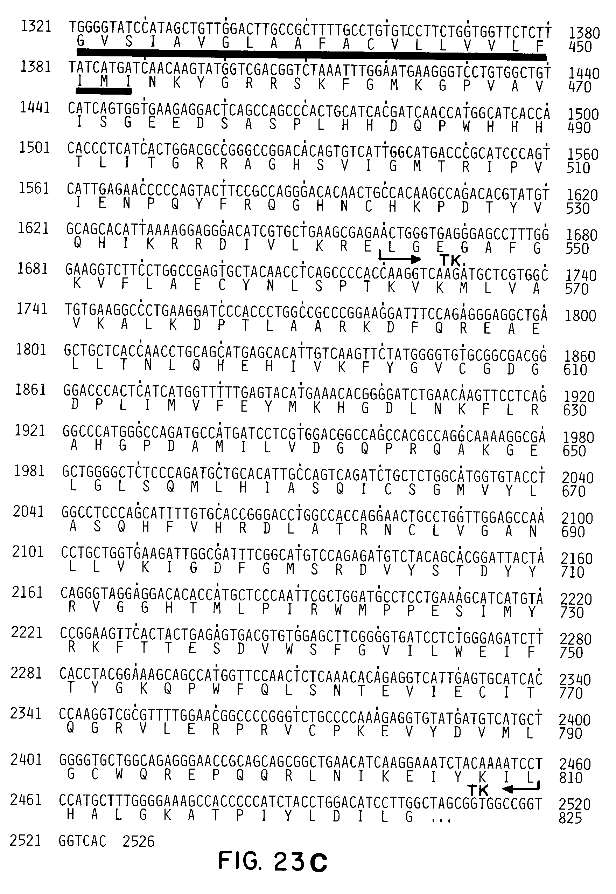

FIGURE 23. Nucleotide sequence analysis of the 2526 bp long insert of pFL19, a cDNA clone of the porcine *trk*C gene. (A) Schematic representation of pFL19. The thick bar represents coding sequences flanked by the initiating (ATG) and terminating (TAG) codons. The putative signal peptide (SP, dotted box), transmembrane (TM, black box) and tyrosine kinase (TK, hatched box) domains are indicated. Other symbols represent cysteine residues (closed dots) and consensus N-glycosylation sites (inverted triangles) present in the extracellular domain. Thin open bars represent 5' and 3' non-coding sequences. (B) Nucleotide and deduced amino acid sequence of the 2526 bp insert of pFL19. The putative signal peptide (amino acids 1-31) is highlighted by a dotted box. The unique Nae I site used to fuse the pFL7 and pFL15 cDNA clones into pFL19 (see Example 4) is overlined. The consensus N-glycosylation sites are underlined by open bars. The cysteine residues in the extracellular domain are circled. The putative transmembrane domain (amino acid residues 430-453) is underlined by a solid bar. The tyrosine kinase catalytic domain (amino acids 544-810) is flanked by horizontal arrows. The in-frame terminator codon TAG (positions 2507 to 2509) is indicated by asterisks.

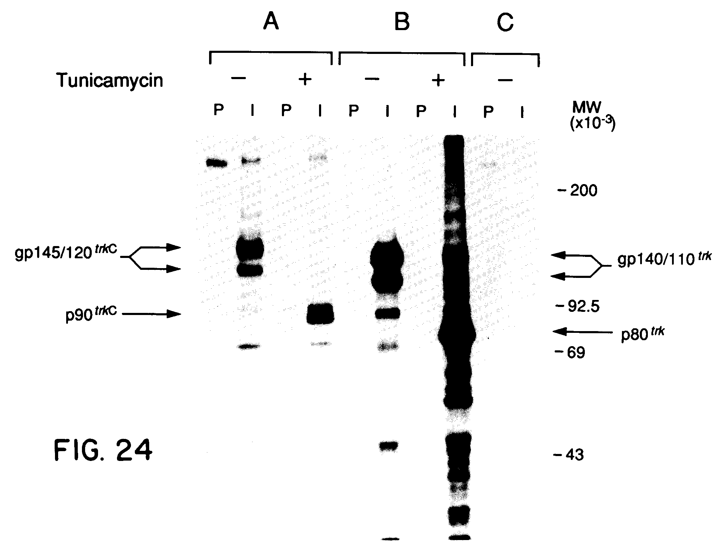

FIGURE 24. Identification of the *trk*C products. [$^{35}$S] methionine-labeled cell extracts of (A) NIH3T3 cells transfected with the expression plasmid pFL20, (B) NIH3T3 cells expressing the *trk* proto-oncogene products and (C) parental NIH3T3 cells were grown either in absence (-) or presence (+) of 10 μg/ml of tunicamycin and submitted to immunoprecipitation analysis with preimmune (P) or immune (I) serum raised against a peptide corresponding to the 14 carboxy-terminal amino acids of the *trk* proto-oncogene product (Fig. 2). The migration of the glycosylated gp145/120$^{trkC}$ and gp140/110$^{trkC}$ molecules and their corresponding polypeptidic backbones p90$^{trkC}$ and p80$^{trk}$ are indicated by arrows. Co-electrophoresed molecular weight markers include myosin (200,000), phosphorylase B (92,500), bovine serum albumin (69,000) and ovalbumin (43,000).

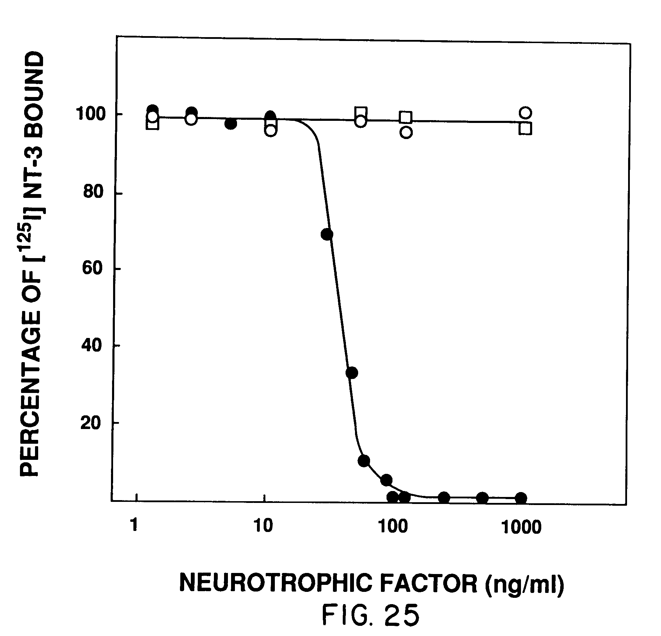

FIGURE 25. Binding of [$^{125}$I]-labeled NT-3 to NIH3T3 cells expressing gp145$^{trkC}$. Parental NIH3T3 cells and NIH3T3 cells expressing gp145$^{trkC}$ (R4-31) were preincubated for 1 hour at 4°C with various amounts of unlabeled NT-3 (filled circles), NGF (open squares) or BDNF (open circles) followed by an additional 2 hour incubation at 4°C with 4 ng/ml of [$^{125}$I]-labeled NT-3. Non-specific binding to parental NIH3T3 cells was subtracted from the data presented.

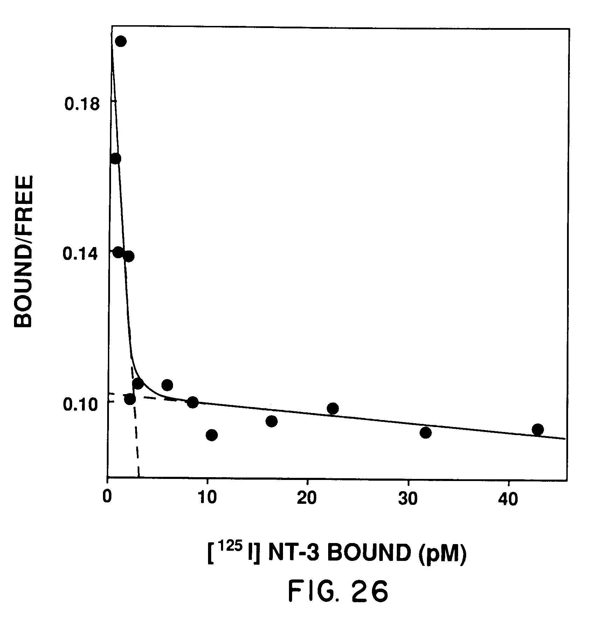

FIGURE 26. Scatchard plot analysis of the binding of [$^{125}$I]-labeled NT-3 to NIH3T3 cells expressing gp145$^{trkC}$. NIH3T3 and R4-31 cells were incubated for 2 hours at 4°C with various concentrations (0.004 nM to 8 nM) of [$^{125}$I]-labeled NT-3. The incubation was performed for 2 hours at 40°C. Binding of [$^{125}$I]-labeled NT-3 to NIH3T3 cells was considered as non-specific binding and subtracted in each case. The percentage of non-specific binding varied from 5% to 15% of the amount of radioactivity bound to R4-31 cells.

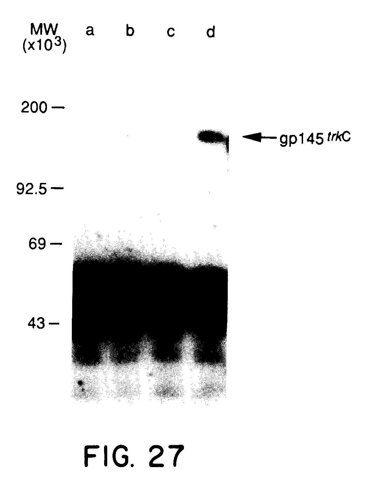

FIGURE 27. NT-3 induces the phosphorylation of tyrosine residues in gp145$^{trkC}$. R4-31 cells were incubated for 10 minutes at 37°C either (a) in the absence of neurotrophic factors or (b-d) in the presence of 50 ng/ml of either (b) NGF; (c) baculovirus-derived BDNF; or (d) baculovirus-derived NT-3. Cells were lysed and immunoprecipitated with rabbit polyclonal antibodies raised against the 14 carboxy-terminal residues of *trk* proto-oncogene [Martin-Zanca, D. et al., Mol. Cell. Biol. 9, 24-33 (1989)]. Immunoprecipitates were analyzed on a 8% SDS-PAGE, transferred to a nitrocellulose membrane and blotted with antiphosphotyrosine monoclonal antibody 4G10 (UBO) as described in Example 1 herein below. Filters were exposed to X-OMAT film for 7 days at -70°C with the help of intensifying screens. The migration of gp145$^{trkC}$ is indicated by an arrow. Molecular weight markers were those described in the legend of Figure 3.

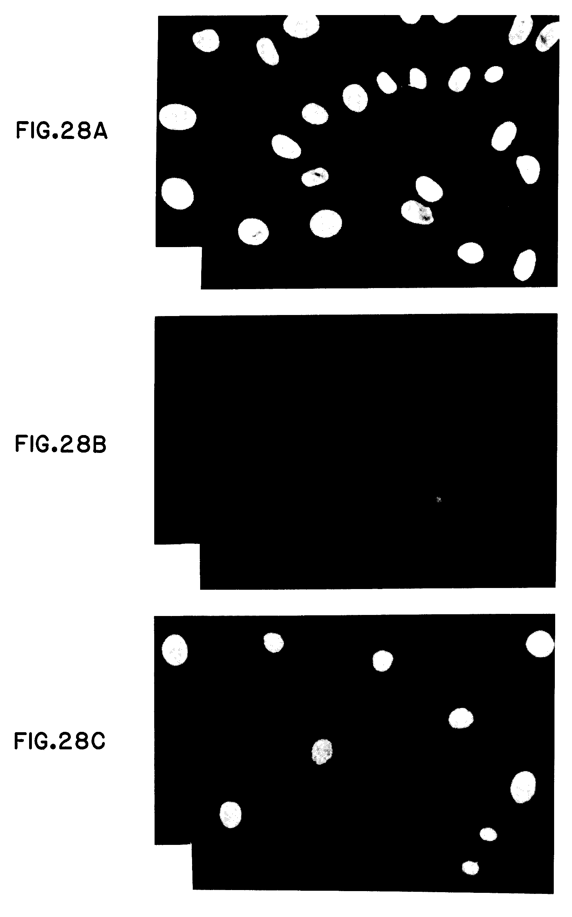

FIGURE 28. NT-3 induces DNA synthesis in quiescent NIH3T3 cells expressing gp145$^{trkC}$. Quiescent R4-31 cells were stimulated with DMEM containing (A) 20% calf serum; (B) 5 μg/ml of insulin; or (C) 50 ng/ml of partially purified NT-3 derived from COS cell supernatants. DNA synthesis was determined by immunofluorescence analysis of incorporated BrdUrd (see Example 4). (400 x magnification).

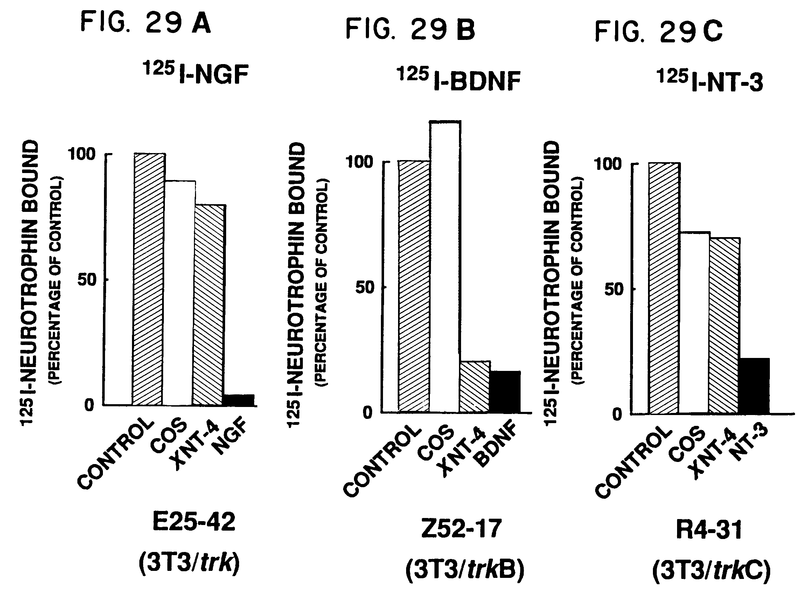

Figure 29. Xenopus NT-4 competes with [$^{125}$I]-BDNF for binding to the gp145$^{trkB}$ receptor. (A) gp140$^{trk}$-expressing E25-42 cells, (B) gp145$^{trkB}$-expressing Z52-17 cells, or (C) gp145$^{trkC}$-expressing R4-31 cells were preincubated for 1 hour at 4ºC with binding media (control); a 1:2 dilution of supernatant from COS cells transfected with vector CDM7B DNA (COS); a 1:2 dilution of supernatant from COS cells transfected with the XNT-4 encoding pFRK84 plasmid (XNT-4); and 15 nM of purified (A) NGF, (B) BDNF, or (C) NT-3. [$^{125}$I]-labeled (A) NGF; (B) BDNF or (C) NT-3 were added to a final concentration of 0.1 nM and the cells incubated for an additional 90 minutes at 4ºC. Results are presented as percentage of the amount of [$^{125}$I]-

labeled neurotrophin bound in the presence of binding media. Nonspecific binding to control NIH3T3 cells was substracted from the results presented.

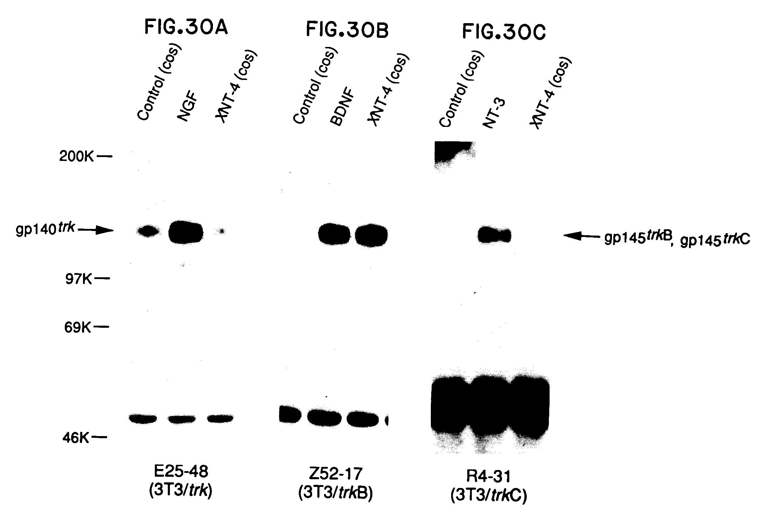

Figure 30. COS cell-expressed Xenopus NT-4 induces tyrosine phosphorylation of gp145$^{trkB}$ receptors. (A) gp140$^{trk}$-expressing E25-48 cells; (B) gp145$^{trkB}$-expressing Z52-17 cells and (C) gp145$^{trkC}$-expressing R4-31 cells were incubated for 10 minutes at 37°C with either a 1:10 dilution of supernatant from COS cells transfected with vector CDM7B DNA (control COS); 100 ng/ml of their respective ligands (A) NGF; (B) BDNF and (C) NT-3, or a 1:10 dilution of supernatant from COS cells transfected with the XNT-4 expression plasmid pFRK84. Cells were lysed and incubated with rabbit polyclonal antibodies raised against a synthetic peptide corresponding to the 14 carboxy-terminal residues of gp140$^{trk}$ [Martin-Zanca, et al., Mol. Cell. Biol. 9, 24-33 (1989)]. The resulting immunoprecipitates were analyzed by 7.5% SDS-polyacrylamide gel electrophoresis, transferred to nitrocellulose filters and blotted with the anti-phosphotyrosine monoclonal antibody 4G10 (UBI) as described in Experimental Procedures herein below. Filters were exposed to Kodak X-OMAT film for 2 to 4 days at -70°C with the help of intensifying screens. The migration of the gp140$^{trkB}$ and gp145$^{trkC}$ receptors is indicated by arrows. Co-electrophoresed molecular weight markers include myosin (200,000), phosphorylase B (97,000), albumin (69,000), and ovalbumin (46,000).

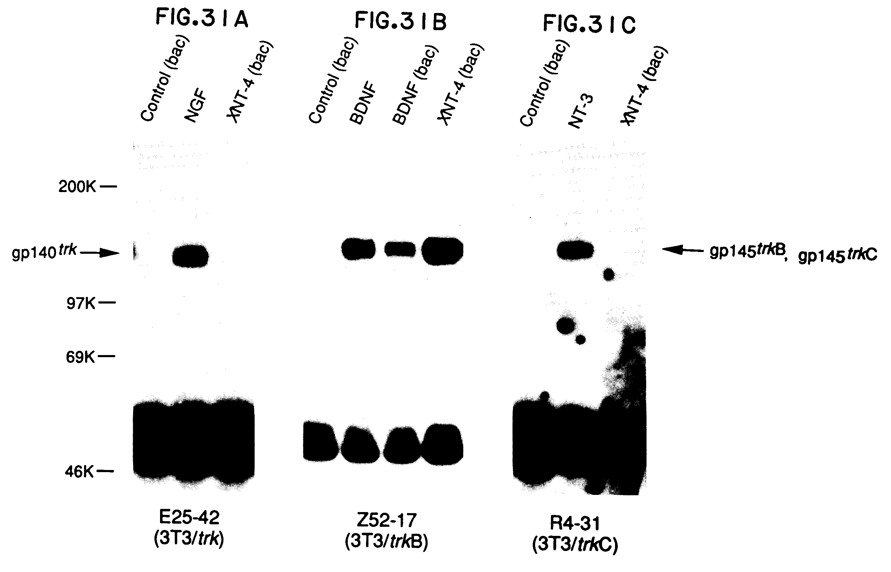

Figure 31. Baculovirus-expressed Xenopus NT-4 induces tyrosine phosphorylation of gp145$^{trkB}$. (A) gp140$^{trk}$-expressing E25-42 cells; (B) gp145$^{trkB}$-expressing Z52-17 cells and (C) g p145$^{trkC}$-expressing R4-31 cells were incubated for 10 minutes at 37°C with either a 1:10 dilution of supernatant from Sf9 cells infected with wild-type baculovirus AcNPV (Control bac.); 100 ng/ml of their respective ligands (A) NGF; (B) BDNF and (C) NT-3 or a 1:10 dilution of supernatant from Sf9 cells infected with the NT-4 recombinant baculovirus FRK83-10. Z52-17 cells were also incubated with a 1:10 dilution of supernatant from Sf9 cells infected with the BDNF recombinant baculovirus AcS27-6 (BDNF bac) [Cordon-Cardo, C. et al., Cell 66, 173-183 (1991)]. Cells were processed as described in the legend to Figure 30.

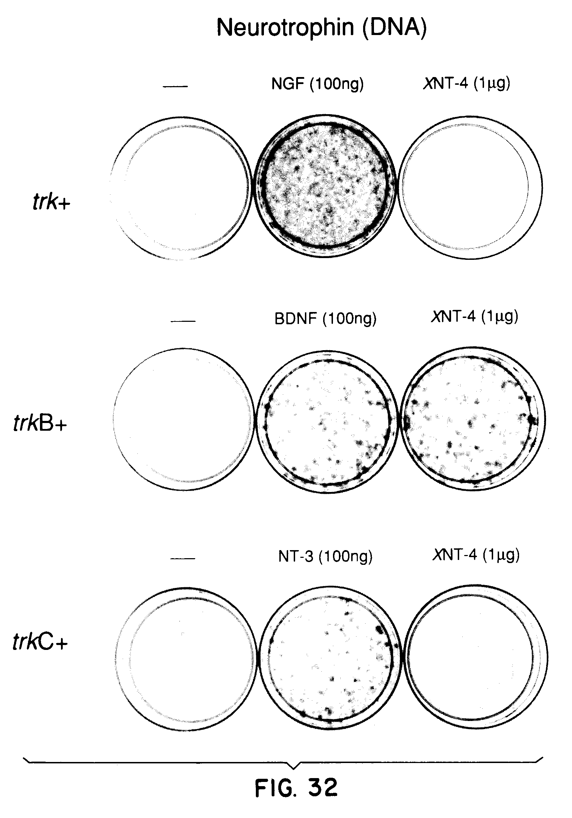

Figure 32. Transformation of NIH3T3 cells by co-transfection of expression plasmids encoding Xenopus NT-4 and the *trk*, *trk*B, and *trk*C receptors. NIH3T3 cells were transfected with 1 $\mu$g of expression plasmids pDM69 (gp140$^{trk}$), pFRK44 (gp145$^{trkB}$), or pFL20 (gp145$^{trkC}$), either in the absence or presence of 1 $\mu$g of *Xenopus* NT-4 expression plasmid, pFRK82. As positive controls, DNAs encoding each of the three *trk* receptors were co-transfected with 100 ng of expression plasmids DNA coding for their corresponding cognate ligands as indicated. Plates were stained with Giemsa 14 days after transfection.

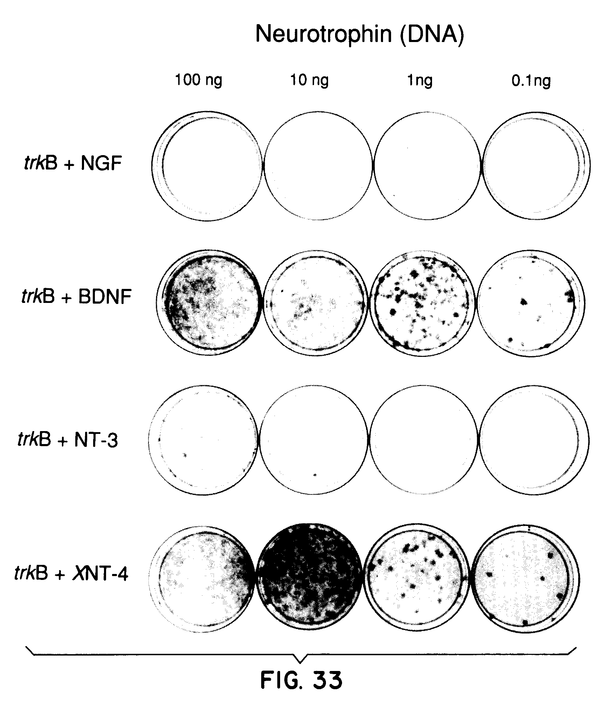

Figure 33. Transformation of NIH3T3 cells by co-transfection of expression plasmids encoding gp145$^{trkB}$ and the neurotrophins NGF, BDNF, NT-3, and Xenopus NT-4. NIH3T3 cells were co-transfected with 1 $\mu$g of the *trk*B receptor expression plasmid pFRK44 along with varying amounts (100 ng to 0.1 ng) of neurotrophin expression plasmids pLTRSNGF (NGF), pLL42 (BDNF), pLL43 (NT-3) and pFRK82 (XNT-4). Plates were stained with Giemsa 14 days after transfection.

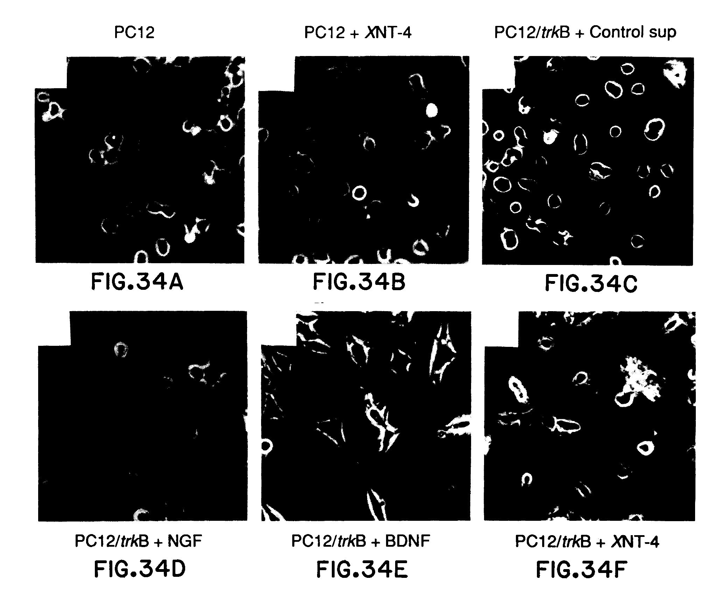

Figure 34. Xenopus NT-4 induces neurite outgrowth in PC12 cells expressing gp145$^{trkB}$ receptors. (A,B) PC12 cells and (C-F) PC12 cells expressing gp145$^{trkB}$ receptors (PC12/*trk*B cells) were treated with (A) media alone; (B,F) a 1:5 dilution of supernatant from COS cells transfected with the XNT-4 expression plasmid pFRK84; (C) a 1:5 dilution supernatant from COS cells transfected with CDM7B vector DNA; (D) 50 ng/ml of NGF and (E) a 1:5 dilution of supernatant from COS cells transfected with the BDNF expression plasmid pVN1 [ See, Example 3 herein below and Klein, R. et al., Cell 66, 395-403 (1991)].

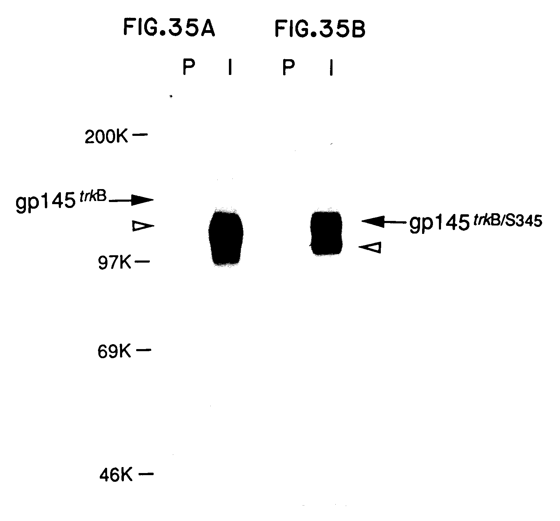

Figure 35. Expression of the mutant gp145$^{trkB/S345}$ receptor. [$^{35}$S] methionine-labeled cell extracts of NIH3T3 cells transfected with (A) the wild-type *trk*B cDNA clone pFRK44 or (B) the mutant *trk*B/S345 cDNA clone pFL3 were incubated either with (P) preimmune sera or (I) rabbit antiserum raised against the bacterially-expressed tyrosine protein kinase domain of gp145$^{trkB}$. The resulting immunoprecipitates were analyzed by 7.5% SDS-polyacrylamide gel electrophoresis. Gels were fixed, subjected to fluorography, dried, and exposed to Kodak X-OMAT film at -70°C with the help of intensifier screens for 48 hours. The migration of the mature gene products is indicated by arrows. The migration of precursor proteins is indicated by open arrow heads. Co-electrophoresed molecular weight markers are the same as in Figure 30.

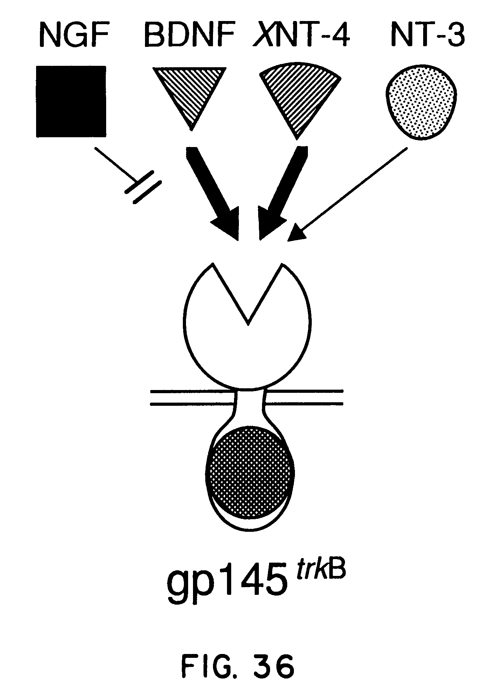

Figure 36. Schematic representation of the interaction of the known members of the NGF family of neurotrophins with the gp145$^{trkB}$ tyrosineprotein kinase receptor.

The present invention is based, in part, on a number of discoveries by Applicants regarding the *trk* family of receptors and their tyrosine protein kinase products, that is, the *trk*, *trk*B and *trk*C proto-oncogenes and their products, tyrosine protein kinases. More particularly, the present invention is based, in part, on the discovery that the protein products of the *trk* family of proto-oncogenes are functional high affinity receptors for various neurotrophic factors, and that the *trk* family tyrosine kinases mediate the biological activities of these neurotrophic factors. Thus, the present invention is based, in part, on the discovery that the product of the *trk* proto-oncogene is a functional high affinity receptor for NGF and neurotropin-3 (NT-3), and that the *trk* tyrosine kinase mediates the biological activity of NGF and NT-3. The present invention is also based, in part, on the discovery that the product of the *trk*B proto-oncogene is a functional, high affinity receptor for brain derived neurotrophic factor (BDNF), NT-3, and neurotrophin-4 (NT-4), and that the *trk*B tyrosine kinase mediates various biological activities of BDNF, NT-3 and NT-4. The present invention is further based, in part, on the discovery that the product of the *trk*C proto-oncogene is a functional, high affinity receptor for NT-3 and that the *trk*C tyrosine kinase mediates various biological activities of NT-3. These discoveries provide the basis for various methods for the detection of agonists and/or antagonists of neurotrophic factors such as NGF, NT-3, NT-4 and BDNF. Thus, the present invention concerns a method for detecting an agonist or an antagonist of a neurotrophic factor comprising:

(a) contacting a *trk* family receptor with the neurotrophic factor and a test substance which may contain the agonist or antagonist; and

(b) determining whether the agonist or antagonist competes with the neurotrophic factor in binding to the *trk* family receptor.

As used in the present application, the phrase "agonist of a neurotrophic factor" refers to a substance which can stimulate and/or replace the action of the neurotrophic factor. As used in the present application, the phrase "antagonist of a neurotrophic factor" refers to a substance which can inhibit or block the action of the neurotrophic factor. As used in the present application, the term "neurotrophic factor" means a substance that promotes survival of cells of neuronal origin, and includes nerve growth factor, neurotrophin-3, neurotrophin-4, neurotrophin-5 (NT-5) [ See, Berkemeyer, L.R. et al., Neuron 7, 857-866 (1991)], brain derived neurotrophic factor and the like. As used in the present application, the term "*trk* family receptor" means a protein such as gp140$^{trk}$ encoded by a *trk* family proto-oncogene which is able to bind a neurotrophic factor, and is meant to include *trk*, *trk*B and *trk*C receptors. The term is also meant to include modified forms of the receptors, for example, those containing amino acid additions, deletions, substitutions, insertions and inversions, as long as the modified form is still able to bind a neurotrophic factor.

This method measures the competition between the neurotrophic factor and the agonist or antagonist in binding to the *trk* family receptor. Various methods can be used to determine whether the agonist or antagonist competes with the neurotropic factor in binding to the *trk* family receptor. For example, the neurotrophic factor can be labeled with a detectable marker so that binding to the *trk* family receptor can be measured. Detectable markers which can be used include, for example, radiolabels, fluorogenic compounds and colorimetric compounds, which can be incorporated into the neurotrophic factor using standard metabolic labeling techniques or chemical conjugation methods. Preferably, a radiolabeled neurotrophic factor (e.g., [$^{125}$I]-labeled $\beta$NGF) is used.

The present invention further concerns a method for detecting an agonist or an antagonist of a neurotrophic factor comprising:

(a) contacting a *trk* family receptor with a test substance thought to contain the agonist or antagonist; and

(b) determining whether the agonist or antagonist binds to the *trk* family receptor.

This method measures the binding of the agonist or antagonist to the *trk* family receptor. For example, the agonist or the antagonist can be labeled with a detectable marker so that binding to the *trk* family receptor can be measured. Detectable markers which can be used include, for example, radiolabels, fluorogenic compounds and colorimetric compounds, which can be incorporated into the agonist or antagonist using standard metabolic labeling techniques or chemical conjugation methods.

Other methods contemplated by the present invention include those in which a biological activity of the *trk* family receptor is measured. Thus, the present invention also concerns a method for detecting an agonist of a neurotrophic factor comprising:

(a) contacting a *trk* receptor with a test substance which may contain the agonist; and

(b) determining whether the agonist which may be contained in the test substance activates one or more biological activities of the *trk* family receptor.

The present invention further concerns a method for detecting an antagonist of a neurotrophic factor comprising:

(a) contacting a *trk* family receptor with a neurotrophic factor and a test substance which may contain the antagonist; and

(b) determining whether the test substance affects the activation by the neurotropic factor of one or more of the biological activities of the *trk* family receptor.

Various biological activities of the *trk* family receptor may be determined. For example, in the case of gp140$^{trk}$, gp145$^{trkB}$ and gp145$^{trkC}$, the tyrosine kinase activity of the receptor or the phosphorylation of tyrosine residues in the receptor can be determined. Other biological responses which can be determined include the transient expression of c-Fos proteins, the induction of DNA synthesis, and the stimulation of cells to enter the S phase.

8

One method to determine the tyrosine kinase activity of gp140$^{trk}$, gp145$^{trkB}$ and gp145$^{trkC}$ is described in Example 1 under the heading "Kinase assay". One method for determining the phosphorylation of tyrosine residues is described in Example 1 under the heading "Immunoblotting". One method for determining the induction of DNA synthesis is described in Example 2 herein below. One method for determining the transient expression of c-Fos proteins is described in Example 2 herein below. One method for determining the stimulation of cells to enter the S phase is described in Example 2 below. However, other methods for carrying out these assays will be apparent to those of ordinary skill in the art in view of the teachings of the present application.

Furthermore, as noted herein below, neurotropic factor antagonists may be potential anti-cancer drugs. Thus, other biological responses which can be determined include the growth of cells in semi-solid media and the ability of cells to become morphologically transformed. One method for determining the growth of cells in semi-solid media and one method for determining the ability of cells to become morphologically transformed are described in Example 2 herein below.

In practicing the methods of the present invention, any *trk* family receptor can be used as long as it is capable of carrying out the function to be measured (e.g., neurotrophic factor binding, tyrosine kinase activity, phosphorylation of tyrosine residues etc.). It is preferred that the *trk* family receptor be the glycosylated protein product of the human *trk* proto-oncogene (hereinafter referred to as gp140$^{trk}$), the *trk*B proto-oncogene (hereinafter referred to as gp145$^{trkB}$) or the *trk*C proto-oncogene (hereinafter referred to as gp145$^{trkC}$), or be derived from gp140$^{trk}$, gp145$^{trkB}$ or gp145$^{trkC}$. For example, in the case of gp140$^{trk}$, if a binding assay is to be performed, only those portions of gp140$^{trk}$ responsible for binding of the neurotrophic factor need be used (i.e., a gp140$^{trk}$ fragment may be employed). If an assay based on one or more of the biological activities of gp140$^{trk}$ is to be performed, those portions of gp140$^{trk}$ responsible for the binding of the neurotrophic factor and the desired biological activity (e.g., tyrosine kinase activity) must be used. However, it is preferred that the full-length gp140$^{trk}$ protein be used.

The methods of the present invention can employ various *trk* family receptor preparations. For example, the *trk* family receptor can be isolated and purified to some degree and used in this form. Preferably, cells expressing the *trk* family receptor (either endogenously or after transformation with a DNA sequence encoding the *trk* family receptor) are employed.

Various cells which endogeneously express the *trk* family receptor may be used in the methods of the present invention. For example, PC12 cells (ATCC CRL 1721) may be used.

It is preferred that recombinant cells transformed with a DNA sequence encoding all or part of the *trk* family receptor and which express the *trk* family receptor be used in the methods of the present invention. For example, cells transformed with a DNA sequence encoding gp140$^{trk}$ and which express gp140$^{trk}$ can be used.

The DNA sequence encoding gp140$^{trk}$ is known [See,Martin-Zanca, D. et al., Mol. Cell. Biol. 9, 24-33 (1989), which is incorporated herein by reference]. The DNA sequence of gp145$^{trkB}$ is known [See Klein, R. et al., EMBO Journal 8, 3701-3709 (1989), which is incorporated herein by reference]. The DNA sequence of gp145$^{trkC}$ is disclosed herein below. A patent application directed to *trk*C is being filed on even date herewith listing Mariano Barbacid and Fabienne Lamballe as co-inventors and entitled "*TRK*C Protein," and is incorporated herein by reference. These DNA sequences can be obtained using various methods well-known to those of ordinary skill in the art. At least three alternative principal methods may be employed:

    (1) the isolation of a double-stranded DNA sequence from genomic DNA or complementary DNA (cDNA) which contains the sequence;

    (2) the chemical synthesis of the DNA sequence; and

    (3) the synthesis of the DNA sequence by polymerase chain reaction (PCR).

In the first approach, a human genomic or cDNA library can be screened in order to identify a DNA sequence coding for all or part of a *trk* family receptor such as gp140$^{trk}$, gp145$^{trkB}$ of gp145$^{trkC}$.

In the second approach, the DNA sequence of coding for all or part of a *trk* family receptor such as gp140$^{trk}$, gp145$^{trkB}$ or gp145$^{trkC}$ can be chemically synthesized. For example, the DNA sequence coding for gp140$^{trk}$, gp145$^{trkB}$ or gp145$^{trkC}$ can be synthesized as a series of 100 base oligonucleotides that can then be sequentially ligated (via appropriate terminal restriction sites) so as to form the correct linear sequence of nucleotides.

In the third approach, the DNA sequences coding for all or part of a *trk* family receptor such as gp140$^{trk}$, gp145$^{trkB}$ or gp145$^{trkC}$ can be synthesized using PCR. Briefly, pairs of synthetic DNA oligonucleotides at least 15 bases in length (PCR primers) that hybridize to opposite strands of the target DNA sequence are used to enzymatically amplify the intervening region of DNA on the target sequence. Repeated cycles of heat denaturation of the template, annealing of the primers and extension of the 3'-termini of the annealed primers with a DNA polymerase results in amplification of the segment defined by the 5' ends of the PCR primers. See, U.S. Patent Nos. 4,683,195 and 4,683,202.

The DNA sequences coding for all or part of gp140$^{trk}$, gp145$^{trkB}$ or gp145$^{trkC}$ can also be modified (i.e., mutated) to prepare various mutations. Such mutant DNA sequence may be prepared, for example, by modifying the gp140$^{trk}$, gp145$^{trkB}$ or gp145$^{trkC}$ DNA sequence so that the mutation results in the deletion, substitution, insertion, inversion or addition of one or more amino acids in the encoded polypeptide using various methods known in the art. For example, the methods of site-directed mutagenesis described in Taylor, J. W. et al., Nucl. Acids Res. 13, 8749-8764 (1985) and Kunkel, J. A., Proc. Natl. Acad. Sci. USA 82, 482-492 (1985) may be employed. In addition, kits for site-directed mutagenesis may be purchased from commercial vendors. For example, a kit for performing site-directed mutagenesis may be purchased from Amersham Corp. (Arlington Heights, IL). The resulting modified polypeptides can also be used in practicing the methods of the present invention.

As used in the present application, the term "modified", when referring to a nucleotide or polypeptide sequence, means a nucleotide or polypeptide sequence which differs from the wild-type sequence found in nature.

Once a DNA sequence encoding a *trk* family receptor such as gp140$^{trk}$, gp145$^{trkB}$ or gp145$^{trkC}$ has been obtained, suitable expression vectors containing the desired coding and control sequences may be constructed using standard recombinant DNA techniques known in the art, many of which are described in Maniatis, T. et al., Molecular Cloning: A Laboratory Manual, Cold Spring Harbor Laboratory, Cold Spring Harbor, NY (1982). For example, a number of suitable expression vectors are described herein below.

These expression vectors may be then introduced into host cells (e.g., NIH 3T3 cells, *Spodoptera frugiperda* (Sf9) cells) by various methods known in the art. For example, transfection of host cells with expression vectors can be carried out by the calcium phosphate precipitation method. However, other methods for introducing expression vectors into host cells, for example, electroporation, biolistic fusion, liposomal fusion, nuclear injection and viral or phage infection can also be employed. Host cells may be tested for successful transformation using various approaches. For example, DNA-DNA hybridization, the presence or absence of marker gene functions, assessing the level of transcription as measured by the production of gp140$^{trk}$, gp145$^{trkB}$ or gp145$^{trkC}$ mRNA transcripts in the host cell, and detection of the gene product immunologically may be used.

Virtually any material thought to contain an agonist or antagonist of a neurotrophic factor, may be employed as a test substance in the methods of the present invention. For example, extracts of plants, bacteria or other biological organisms, peptides and chemically synthesized compounds may be tested using the methods of the present invention.

Substances (e.g., agonists) identified using the methods of the present invention should be useful to avoid neuronal death, and in the treatment of degenerative neural diseases (e.g., Alzheimer's disease, Parkinson's disease etc.). Substances (e.g., antagonists) identified using the methods of the present invention may also be potential anti-cancer drugs.

The particular parameters employed in the methods of the present invention can vary widely depending on various factors such as the concentration of agonist or antagonist in the test substance, the nature of the test substance, the type of assay and the like. Optimal conditions can be readily established by one of ordinary skill in the art. For example, the amount of *trk* family receptor should range from about 1 nM to about 100 nM. Typical assay conditions include a temperature range of about 4°C to about 37°C, a pH valve range of about 6.8 to about 7.6, and an ionic strength of about 10 mM to about 250 mM. Times will vary widely depending upon the nature of the assay, and generally range from about 5 minutes to about 2 hours. A wide variety of buffers, for example Tris, may be employed, and other reagents such as salts to enhance ionic strength, stabilizers and biocides may also be included.

The following examples are further illustrative of the present invention. These examples are not intended to limit the scope of the present invention, and provide further understanding of the invention.

EXAMPLE 1

I. EXPERIMENTAL PROCEDURES

A. Cells, growth factors, antisera

NIH3T3 [Jainchill, J.L. et al., J. Virol. 4, 549-553 (1969)], E25-427 [Martin-Zanca, D. et al., Mol. Cell. Biol. 9, 24-33 (1989)], E25-48, B8-923 [Klein, R. et al., EMBO J. 8, 3701-3709 (1989)] and B35-41 cells were grown in Dulbecco's modified Eagle's medium (DMEM) containing 10% calf serum. E25-48 and B35-41 cell lines were obtained by transfecting NIH3T3 cells with the mammalian *trk* and *trk*B expression plasmids

EP 0 504 914 A2

pDM38 [Martin-Zanca, D. et al., Mol. Cell. Biol. 9, 24-33 (1989)] and pFRK44 [Klein, R. et al., EMBO J. 8, 3701-3709 (1989)], respectively. PC12 cells were grown on collagen-coated (Vitrogen) dishes in DMEM containing 10% horse serum and 5% fetal calf serum. Human 2.5S NGF ($\beta$NGF) was purchased from Upstate Biotechnology, Inc., Lake Placid, N.Y. Murine [125I]-labeled $\beta$NGF was obtained from Amersham (1500 Ci/mmol).

Rabbit polyclonal anti-phosphotyrosine antibodies were provided by G.L. Schieven and J.A. Ledbetter. Such antibodies are also available from various commercial sources such as Oncogene Science, Mineola, N.Y. Rabbit polyclonal antibodies elicited against peptides corresponding to the carboxy terminus of the *trk* proto-oncogene product [Martin-Zanca, D. et al., Mol. Cell. Biol. 9, 24-33 (1989)] and the *trk*B tyrosine protein kinase domain [Klein, R. et al. EMBO J. 8, 3701-3709 (1989)] have been previously described. A broadly reactive antiserum elicited against the rat gp80$^{LNGFR}$ receptor was a gift of G. Weskamp and L.F. Reichardt of the University of California, San Francisco (See, Neuron, April, 1991, In Press).

### B. NGF binding

The number of NGF binding sites and their dissociation constants were determined by Scatchard analysis of [125I]-labeled $\beta$NGF binding at 4°C, to various cell lines (either in monolayer or in suspension) as previously described [Jing, S. et al., J. Cell Biol. 110, 283-294 (1990)]. For binding studies on monolayer cultures, cells were plated at a density of 7.5 x 10$^4$ cells/cm$^2$ in 24-well Costar tissue culture plates 24 hours before the binding assay. cells in duplicate wells were washed once with 1 ml of ice-cold DMEM containing 1 mg/ml of BSA (DMEM-BSA) and incubated with 150 $\mu$l of ice-cold DMEM-BSA containing various concentrations (0.02 nM to 50 nM) of [125I]-labeled $\beta$NGF at 4°C for 2 hours. Cells were washed four times with 0.5 ml of ice-cold DMEM-BSA, removed with 0.5 ml of 1 N NaOH, and the radioactivity counted in a G5500 gamma counter. For binding studies on cells in suspension, cells were harvested, washed with DMEM-BSA, and the rest of the experiment was performed as described above using 2-3 x 10$^5$ cells in each binding sample. Nonspecific binding at each concentration of [125I]-labeled $\beta$NGF was determined by the addition of a 100-fold excess of unlabeled $\beta$NGF.

### C. Chemical cross-linking and immunoprecipitation

Cells were harvested in phosphate-buffered saline, pH 7.1, containing 1 mM EDTA, pelleted, and resuspended in ice-cold binding buffer (137 mM NaCl, 4.7 mM KCl, 2.5 mM CaCl$_2$, 1.2mM KH$_2$PO$_4$[pH7.4], 1.2 mM MgSO$_4$, 1 mg/ml glucose, 1 mg/ml BSA, 10 mM HEPES [pH 7.0] and 1 mM phenylmethysulfonyl fluoride) [Meakin, S.O. and Shooter, E.M., Neuron 6, 153-163 (1991)] at a concentration of 1-2 x 10$^6$ cells/ml. [125I]-labeled $\beta$NGF was added to a final concentration of 1 nM, and the cells were incubated at 4°C for 2 hours. The chemical cross-linker dithiobis (succinimidylpropionate) (DSP) (Pierce) was dissolved in dimethyl sulfoxide and added to a final concentration of 150 $\mu$M (with a final concentration of 0.5% dimethyl sulfoxide). The reaction was incubated at room temperature for 30 minutes, and was quenched by washing the cells three times with 5 ml of Tris-buffered saline. The cells were then either lysed directly in sample buffer (80 mM Tris HCl pH 6.8, 10% [v/v] glycerol, 1% [w/v] SDS, 0.025% bromophenol blue) or processed for further immunoprecipitation as described previously [Martin-Zanca, D. et al., Mol. Cell. Biol. 9, 24-33 (1989)].

### D. Immunoblotting

To assay for NGF-induced tyrosine phosphorylation, cells were grown on collagen-coated dishes as indicated above. Cells were rinsed once with prewarmed serum-free DMEM and incubated for 4 minutes at 37°C with 100 ng/ml of $\beta$NGF in serum-free DMEM. After induction, NGF was aspirated and the cells rapidly lysed with ice-cold PK lysis buffer (10mM sodium phosphate pH 7.0, 100mM NaCl, 1% Triton X-100, 5 mM EDTA, 100 $\mu$M sodium vanadate, 2mM PMSF and 0.2 trypsin inhibitor units of aprotinin). After immunoprecipitation with antisera raised against the *trk* proto-oncogene product, the proteins were separated by 7.5% SDS-PAGE and blotted onto nitrocellulose as previously described [Harlow, E. and Lane, D., Antibodies:A Laboratory Manual, Cold Spring Harbor Laboratory, Cold Spring Harbor, New York (1988)]. The filters were blocked with 5% BSA (Sigma) and incubated with anti-phosphotyrosine antibodies for 2 hours at room temperature. Proteins phosphorylated on tyrosine residues were visualized by autoradiography after incubation with [125I]-labeled Protein A (5.6 $\mu$Ci/ $\mu$g; 5 $\mu$Ci per 10 ml of Tris-buffered saline).

### E. Baculovirus expression of *trk* and *trk*B gene products

*Spodoptera frugiperda* (Sf9) cells were obtained from American Type Culture Collection, Rockville, MD. The Sf9 cells were grown in Ex-Cell 400 medium (JRH Biosciences) in monolayer culture or in shaker flasks. *Autograpba californica* Multiple Nuclear Polyhedrosis Virus Strain E2 (AcNPV) was obtained from Max D. Summers (Texas A&M University) [Summers, M.D. and Smith, G.E., in Bulletin No. 1555, College Station, Texas: Texas Agricultural Experiment Station and Texas A&M University, pp. 10-39 (1987)]. Recombinant baculoviruses that are capable of expressing *trk* (pAcS2) or *trkB* (pAcS1) proteins (see below) were isolated by plaque purification following cotransfection of AcNPC DNA and the corresponding baculovirus transfer vector plasmid into Sf9 cells using described procedures [Summers, M.D. and Smith, G.E., in Bulletin No. 1555, College station, Texas: Texas Agricultural Experiment Station and Texas A&M University, pp.10-39 (1987)]. Expression of the recombinant proteins was confirmed by immunoblot analysis using specific antibodies. Infections of Sf9 cells with recombinant baculoviruses for the expression of the *trk* and *trk*B proteins were done at a multiplicity of infection of 5-10. Time course studies were performed in order to determine the time of optimal expression of ligand-stimulatable protein.

The baculovirus transfer vector plasmids were constructed using standard methods [Maniatis et al., Molecular Cloning: A Laboratory Manual, Cold Spring Harbor Laboratory, Cold Spring Harbor, New York (1982)] in pVL1392 and pVL1393 [Luckow, V.A. and Summers, M.D., Virology 167, 56-71 (1988)]. To generate pAcS1, the plasmid pFRK43 containing the full-length mouse *trk*B cDNA [Klein, R. et al., EMBO J. 8, 3701-3709 (1989)] was first modified by deleting the complete 3'- untranslated region, thereby creating a new EcoRI restriction site immediately 3' of the stop codon. A partially digested 2.9 kbp NcoI-EcoRI cDNA fragment encompassing the entire mouse *trk*B coding sequences was subcloned into the BamHI and EcoRI cloning sites of pVL1393 by using the necessary adaptors. pAcS2 was generated by ligating the 2.57 kbp BstUI-EcoRI DNA fragment of pLM6 [Martin-Zanca, D. et al., Mol. cell. Biol. 9, 24-33 (1989)] into BamHI and EcoRI-digested pVL1393 using the appropriate adaptors.

F. Kinase assay

Sf9 cells were collected 36 hours after infection with recombinant baculoviruses expressing *trk* and *trk*B proteins. After washing once with protein-free medium, cells were suspended in lysis buffer containing 10 mM Tris-HCl pH 7.4 and 1 mM $MgCl_2$ at a concentration of $4 \times 10^6$ cells/ml. After 30 minutes of incubation at 4°C, cells were dounced 70 strokes with a cell douncer. Cell nuclei and other debris were removed by centrifugation at 500 x g for 5 minutes. The supernatant containing the membranes was centrigued at 140,000 x g for 30 minutes at 4°C. The pellets containing the membrane fractions were collected and resuspended in buffer containing 20mM Hepes-KOH pH 7.2, 10% glycerol, 150 mM NaCl and 0.05% Triton X-100. These membrane preparations (600 ng protein per assay) were preincubated with 1 $\mu$g/ml of $\beta$NGF for 10 minutes at 4°C. Following the incubation, 100 $\mu$l of PK lysis buffer was added and the *trk* and *trk*B proteins immunoprecipitated with specific polyclonal antisera. After 2 hours incubation at 4°C, the resulting immunocomplexes were precipitated with protein A Sepharose beads and washed twice with 1 ml of PK lysis buffer. Beads containing the *trk* and *trk*B proteins were resuspended in the presence or absence of $\beta$NGF in 50 $\mu$l of incubation buffer containing 20 mM Hepes-KOH pH 7.2, 150 mM NaCl, 0.05% Triton X-100, 10 mM $MgCl_2$, 1 mM DTT and 50 $\mu$Ci of $[\gamma^{32}P]$-labeled ATP (6000 Ci/mmol) for 5 minutes at 30°C [Konopka, J.B. and Witte, O.N., Mol. Cell. Biol. 5, 3116-3123 (1985)]. The reactions were terminated by the addition of 10 $\mu$l of 0.2 M EDTA and the beads washed three times with RIPA buffer. Phosphorylated proteins were analyzed by 8% SDS-polycrylamide gel electrophoresis and visualized by autoradiography.

II. RESULTS

A. NGF binding to mouse fibroblasts expressing the *trk* proto-oncogene

Heterologous mouse NIH3T3 cells expressing the human *trk* (E25-427 and E25-48 cell lines) and the mouse *trk*B (B8-923 and B35-41) proto-oncogenes were incubated with nanomolar amounts of [$^{125}$I]-labeled $\beta$NGF at 4°C as indicated herein above. As shown in Figure 1, those cell lines expressing the human *trk* proto-oncogene product, gp140$^{trk}$, bound NGF efficiently. The amount of $\beta$NGF bound to these cells correlated well with their relative levels of expression of gp140$^{trk}$ (Fig.2). More importantly, the binding of [$^{125}$I]-labeled $\beta$NGF could be efficiently competed with a 100-fold excess of unlabeled NGF (Fig. 1). In contrast, no significant binding of $\beta$NGF to either the parental NIH3T3 cells or to B8-923 and B35-41 cells, two NIH3T3-derived cell lines expressing comparable levels of the highly related mouse *trk*B tyrosine protein kinase, gp145$^{trkB}$, was observed (Fig. 1).

As a control, the binding of NGF to PC12 cells, a rat pheochromocytoma cell line known to possess high affinity NGF receptors, was examined. βNGF efficiently bound to PC12 cells at levels comparable to those previously reported in the literature. As shown in Figure 2, PC12 cells also express the *trk* proto-oncogene, albeit at considerably lower levels than E25-427 or E25-48 cells (Fig. 2). It should be noted that the product of the *trk* proto-oncogene in these rat neural cells exhibits a slower electrophoretic migration than its human counterpart when expressed in NIH3T3 cells, probably due to more extensive glycosylation.

B. Cross-linking of NGF to gp140*trk*

The above results raised the possibility that the product of the *trk* proto-oncogene might be a receptor for NGF. In order to examine this possibility, [$^{125}$I]-labeled NGF was chemically cross-linked to NIH3T3-derived E25-427 and E25-48 cells. When the radiolabeled cell lysates were analyzed by SDS-polyacrylamide gel electrophoresis, most of the cross-linked radioactivity migrated as a diffuse band with an apparent molecular weight of 140,000 to 160,000. No specific labeling could be observed when control NIH3T3 cells or NIH3T3 cells expressing the mouse *trk*B tyrosine protein kinase were used (Fig. 3). When similar experiments were performed with the NGF-responsive PC12 cells, at least two distinct bands become radiolabeled. One of them exhibited an apparent molecular weight of 90,000 to 100,000 and is likely to correspond to the well characterized low affinity NGF receptor encoded by the LNGFR gene [Chao, M.V. et al., Science 232, 518-521 (1986); Radeke, M.J. et al., Nature 325, 593-597 (1988)]. The second band exhibited a similar electrophoretic migration as the protein species detected in E25-427 and E25-48 cells and may correspond to the previously described high affinity NGF receptor [Massague, J. et al., J. Biol. Chem. 256, 9419-9424 (1981); Hosang, M. and Shooter, E.M., J. Biol. Chem. 260, 655-662 (1985)].

In order to establish whether any of these βNGF cross-linked molecules were the product of the *trk* proto-oncogene, [$^{125}$I]-labeled E25-427 and E25-48 cell extracts were incubated with an antiserum elicited against the carboxy-terminal domain of gp140*trk* [Martin-Zanca, D. et al., Mol. Cell. Biol. 9, 24-33 (1989)]. As shown in Figure 3, this antiserum specifically immunoprecipitated a protein with an apparent molecular weight of 155,000 which is likely to correspond to cross-linked NGF-gp140*trk* complexes. More importantly, this antiserum also recognized the same [$^{125}$I]-labeled protein species from PC12 cells (Fig. 3). Each of these immunoprecipitations could be specifically blocked by the addition of 5 μg of the immunizing peptide (Fig. 2). The results indicate that the product of the *trk* proto-oncogene is one of the NGF receptors present in PC12 cells.

C. High affinity NGF receptors

In order to determine whether the product of the *trk* proto-oncogene could serve as a high affinity receptor for NGF, Scatchard plot analysis (Scatchard, 1949) of the binding of [$^{125}$I]-labeled NGF to E25-427, E25-48 and PC12 cells (Fig. 4) was conducted. As summarized in Table 1, each of these cell lines exhibited high affinity NGF receptors ($K_d = 0.09$ nM to 0.3 nM). The number of these high affinity receptors was similar in each of these cells (17,000 in PC12 cells versus 28,600 in E25-427 cells and 20,500 in E25-48 cells). These numbers did not correlate with the levels of *trk* proto-oncogene expression present in each of these cell lines (Fig. 2).

Consistent with previous studies, PC12 cells also exhibited low affinity NGF receptors ($K_d = 33$ nM and 109,000 binding sites/cell) which are likely to correspond to the well characterized LNGFR gene product, gp80$^{LNGFR}$ [Chao et al., Science 232, 518-521 (1986); Radeke, M. J. et al., Nature 325, 593-597 (1987)]. This NGF receptor appears to be preferentially expressed in cells of neuroectodermal origin, although it has also been identified in cells of different developmental origins [Thomson, T.M. et al, Exp. Cell. Res. 174, 533-539 (1988)]. As illustrated in Table 1, E25-427 and E25-48 cells also contain significant levels of low affinity NGF receptors (1.26 x $10^6$ and 0.2 X $10^6$ binding sites per cell, respectively). However, the absence of detectable βNGF binding sites in NIH3T3 cells strongly suggests that these fibro-blastic cells do not express gp80$^{LNGFR}$ receptors. Indeed, immunoprecipitation of metabolically labeled NIH3T3 cells with polyclonal antibodies elicited against the rat gp80$^{LNGFR}$ did not reveal detectable levels of this receptor in this fibroblastic cell line (data not shown). Therefore, the low affinity binding sites present in E25-427 and E25-48 cells may represent modified forms of the *trk* proto-oncogene product. Alternatively, expression of the *trk* proto-oncogene may induce the synthesis of low affinity gp80$^{LNGFR}$ receptors in these fibroblastic cells.

To address these possibilities, PC12 cells along with NIH3T3, E25-427 and E25-48 cells were cross-linked to [$^{125}$I]-labeled NGF and immunoprecipitated with polyclonal antibodies raised against rat gp80$^{LNGFR}$. This antisera has broad interspecies reactivity since it also recognizes the chicken LNGFR gene product (G. Weskamp, personal communication). As shown in Figure 5, this antiserum recognized the cross-linked gp80[LNGFR] molecules in extracts obtained from PC12 cells, but in the corresponding extracts derived from either parental NIH3T3 cells or the *trk* proto-oncogene expressing E25-427 and E25-48 cell lines. Since E25-427 and E25-48 cells contain significantly higher numbers of low affinity receptors than PC12 cells, these results stronly suggest that the low affinity NGF binding sites present in these NIH3T3 derived cells do not correspond to the previously characterized low affinity NGF receptor.

### D. NGF binding to the human *trk* proto-oncogene product expressed in insect cells

The above results illustrate that heterologous expression of the human *trk* proto-oncogene in NIH3T3 cells elicits the appearance of high affinity NGF receptors. Unfortunately, the presence of high numbers of low affinity binding sites in these cells precluded us from demonstrating that such high affinity receptors are encoded by the *trk* proto-oncogene. In an attempt to resolve this matter, the human *trk* proto-oncogene was expressed in Sf9 insect cells using a baculovirus expression system. [$^{125}$I]-labeled NGF binding studies indicated that neither Sf9 cells or Sf9 cells infected with the wild type AcNPV virus contained detectable NGF binding sites (data not shown). In contrast, Sf9 insect cells infected with a recombinant virus strain (pAcS2) carrying the human *trk* proto-oncogene exhibited significant levels of NGF binding. Scatchard plot analysis revealed that these infected insect cells expressed a considerable number (31,000 sites per cell) of high affinity (0.42 nM) NGF receptors (Fig.6). As a control, Sf9 cells infected with a similar recombinant virus (pAcS1) expressing the highly related mouse *trk*B tyrosine protein kinase did not exhibit detectable NGF binding sites (data not shown). These results strongly support the concept that the *trk* proto-oncogene encodes high affinity NGF receptor.

### E. NGF-induced tyrosine phosphorylation of gp140[trk]

To determine whether the NGF receptors encoded by the human *trk* proto-oncogene participate in signal transduction, the ability of NGF to induce phosphorylation of gp140[trk] molecules on tyrosine residues was examined. Unfortunately, incubation of either E25-427 or E25-48 cells with NGF did not result in a significant increase in tyrosine phosphorylation of gp140[trk] proteins due to their high level of steady state phosphorylation in these cells (data not shown). Therefore, PC12 cells were utilized. As shown in Figure 7, the *trk* proto-oncogene product present in untreated PC12 cells does not contain detectable phosphotyrosine residues. However, when these cells were exposed to BNGF for a short period of time (4 minutes), a rapid phosphorylation in tyrosine residues of the PC12 endogenous *trk* proto-oncogene product was observed.

### F. NGF stimulates the kinase activity of gp140[trk]

The rapid phosphorylation of gp140[trk] as a response to $\beta$NGF binding suggests that this neurotrophic factor may activate the tyrosine kinase activity of the *trk* proto-oncogene product. To examine this possibility, membranes were isolated from Sf9 insect cells expressing the human *trk* proto-oncogene product. In these cells the human *trk* proto-oncogene encodes a glycoprotein with an apparent molecular mass of 106,000 daltons (Fig.8). The smaller size of the human *trk* proto-oncogene product expressed in insect cells is likely to be due to a different glycosylation pattern. These membrane preparations were subsequently incubated with antiserum raised against the *trk* proto-oncogene product. The resulting immunoprecipitates were assayed for protein kinase activity in the presence or absence of $\beta$NGF. As shown in Figure 8, addition of nanomolar amounts of $\beta$NGF to baculovirus-expressed *trk* proto-oncogene proteins led to a significant increase (six to ten-fold) in their kinase activity. As a control, addition of $\beta$NGF to membrane preparations obtained from Sf9 cells expressing comparable levels of the related mouse *trk*$\beta$ protein had no effect on its tyrosine kinase activity (Fig.8). Each of these kinase reactions contained similar amounts of the *trk* and *trk*B proto-oncogene products as determined by immunoblotting analysis (data not shown). These results suggest that the interaction of $\beta$NGF with the high affinity receptors encoded by the *trk* proto-oncogene may play an important role in the signal transduction processes elicited by this neurotrophic factor.

### III. DISCUSSION

NGF plays a major role in the development and survival of certain neurons, in particular those of the dorsal root and sympathetic ganglia [Levi-Montalcini, R., EMBO J. 6, 1145-1154 (1987)]. An additional role for this neurotrophic factor in the maintenance of certain CNS cholinergic neurons has also been proposed [Dreyfus, C.F., Trends Pharmacol. Sci. 10, 145-149 (1989)]. The interaction of NGF with its target cells appears to be mediated through two classes of receptors which can be distinguished on the basis of their relative affinities [Sutter, A. et al., J. Biol. Chem. 254, 5972-5982 (1979)]. The high affinity receptors ($K^d \cong$ $10^{-10}$M) mediate all the known biological actions of NGF. The physiological role of the more abundant low affinity receptors ($K^d \cong 10^{-8}$M) remains to be defined. Based on the differential expression of these two classes of receptors in various cell types, it has been possible to determine that the high affinity receptors have molecular weights of about 140,000, whereas the low affinity receptors have a molecular mass of around 80,000 daltons [Massague, J. et al., J. Biol. Chem. 256, 9419-9424 (1981); Hosang, M. and Shooter, E.M., J. Biol. Chem. 260, 655-662 (1985)].

Gene transfer strategies have led to the isolation of a gene encoding low affinity NGF receptors [Chao, M.V. et al., Science 232, 518-521 (1986); Radeke, M.J. et al., Nature 325, 593-597 (1987)]. The human LNGFR gene encodes a polypeptide of 427 amino acids residues (424 in the rat LNGFR product) of which 28 correspond to the signal peptide [Johnson, D. et al., Cell 47, 545-554 (1986); Radeke, M.J. et al., Nature 325, 593-596 (1987)]. The mature receptor appears as an 80,000 dalton glycoprotein (gp80$^{LNGFR}$) when analyzed by SDS-polyacrylamide gel electrophoresis. This glycoprotein contains an extracellular domain rich in cysteine residues and acidic amino acids, a single transmembrane region and a relatively short cytoplasmic domain which does not contain distinctive motifs [Johnson, D. et al., Cell 47, 545-554 (1986); Radeke, M.J. et al., Nature 325, 593-597 (1987); Large, T.H. et al., Neuron 2, 1123-1134 (1989)]. The structural nature of the high affinity NGF receptors is by far less well understood. It has been proposed that these receptors might be a multi-protein complex in which the NGF binding site is provided at least in part, by the product of the LNGFR gene. However, antibodies raised against various epitopes of the gp80$^{LNGFR}$ low affinity receptor failed to recognize high affinity receptors, suggesting that these two classes of receptors may be encoded by different loci [Meakin, S.O., and Shooter, E.M., Neuron 6, 153-163 (1991)]. Moreover, high affinity, but not low affinity receptors can be immunoprecipitated by antiphosphotyrosine antibodies [Meakin, S.O. and Shooter,E.M., Neuron 6, 153-163 (1991)]. These observations have raised the possibility that the NGF high affinity receptor might be either closely associated with or have intrinsic tyrosine protein kinase activity.

The results presented in this study provide strong support to the notion that at lease some of the high affinity receptors for NGF correspond to gp140$^{trk}$, the tyrosine protein kinase encoded by the *trk* proto-oncogene [Martin-Zanca, D. et al., Mol. Cell. Biol. 9, 24-33 (1989)]. Several independent lines of evidence support this hypothesis: (i) βNGF efficiently binds to non-neural NIH3T3 mouse fibroblasts and Sf9 insect cells only when they express the *trk* proto-oncogene product; (ii) βNGF can be chemically cross-linked to the product of the *trk* proto-oncogene independently of whether it is ectopically expressed in NIH3T3 cells or is present in PC12 cells; (iii) cells expressing the *trk* proto-oncogene depict significant numbers (20,000 to 30,000) of high affinity NGF receptors regardless of their species of origin or their developmental lineage; (iv) the product of the *trk* proto-oncogene becomes rapidly phosphorylated on tyrosine residues upon incubation of PC12 cells with βNGF; and (v) the tyrosine kinase activity of the *trk* proto-oncogene product can be stimulated by βNGF.

The structural relationship between the two classes of NGF receptors have been the subject of intense debate. Transfection of a cDNA clone of the human LNGFR gene into a PC12 cell line variant that lacks NGF receptors resulted in the appearance of low and high affinity NGF binding sites [Hempstead, B.L. et al., Science 243, 373-375 (1989)]. Similar observations have been reported with neuroblastoma and medulloblastoma cell lines [Matsushima, H. and Bogenmann, E., Mol. Cell. Biol. 10, 5015-5020 (1990); Pleasure, S.J. et al., Proc. Natl. Acad. Sci. USA 87, 8496-8500 (1990)]. These results suggest that expression of high affinity binding sites is dependent upon the presence of the low affinity receptors encoded by the LNGFR gene. The findings presented herein cannot be easily reconciled with this interpretation. Expression of high affinity NGF receptors in NIH3T3 cells does not require synthesis of the gp80$^{LNGFR}$ protein. Moreover, induction of high affinity receptors in Sf9 insect cells upon expression of the *trk* proto-oncogene makes it unlikely that gp140$^{trk}$ may require other proteins to generate high affinity NGF binding sites. It is possible however, that neural cells may have more than one class of high affinity NGF receptors, only one of which requires the presence of gp80$^{LNGFR}$ low affinity receptors. Availability of antibodies against gp140$^{trk}$ capable of blocking NGF binding may help to resolve this possibility. Alternatively, expression of the LNGFR gene might induce synthesis of the *trk* proto-oncogene product. Further studies will be required to establish the role of the low affinity LNGFR receptors in NGF function.

Cells known to contain high affinity NGF receptors also exhibit low affinity binding sites. Interestingly, binding of βNGF to non-neural cells ectopically expressing high affinity gp140$^{trk}$ receptors has revealed the presence of low affinity NGF receptors distinct from gp80$^{LNGFR}$. This new class of low affinity receptors might be modified forms of gp140$^{trk}$. The high concentration of gp140$^{trk}$ molecules in these cells may lead to receptor oligomerization and/or ligand independent autophosphorylation which may decrease their affinity for NGF. Alternatively, NIH3T3 and Sf9 cells may only be able to properly present a finite number of high affinity receptors. Both of these hypotheses are consistent with the observation that the total number of receptors present in these non-neural cells correlates well with the estimated number of gp140$^{trk}$ molecules. Further studies are needed to determine whether low affinity binding sites, regardless of the biochemical nature, are a requirement for the existence of high affinity NGF receptors.

High affinity receptors are known to mediate the biological responses to NGF. One of the earliest responses to this factor involves an increase in tyrosine phosphorylation [Maher,P.A., Proc. Natl. Acad. Sci. USA 85, 6788-6791 (1988)]. The results presented herein demonstrating rapid phosphorylation of tyrosine residues in gp140$^{trk}$ upon NGF treatment of PC12 cells, support the concept that gp140$^{trk}$ molecules not only serve as high affinity receptors, but also mediate signal transduction. The stimulation of the tyrosine kinase activity of the *trk* protein by NGF in Sf9 membrane preparation adds further support to this concept. Exposure of PC12 cells to NGF also leads to the rapid induction of the *fos* proto-oncogene [Curran, T., and Morgan, J.I., Science 229, 1265-1268 (1985); Greenberg, M.E. et al., J. Biol. Chem. 260, 14101-14110 (1985); Kruijer, W. et al., Proc. Natl. Acad. Sci. USA 82, 7330-7334 (1985)]. It would be important to determine whether this response is also mediated by the product of the *trk* proto-oncogene (for instance by using anti gp140$^{trk}$ antibodies that block NGF binding) and whether a similar response can be elicited in non-neural cells ectopically expressing *trk* receptors. Such studies will provide relevant information regarding the signal transduction pathways involved in the development and survival of neurons. The inter-action of NGF with gp140$^{trk}$ receptors exhibits a high degree of specificity since NGF does not bind to the highly related gp145$^{trkB}$ tyrosine protein kinase. This is rather remarkable since the extracellular domains of these kinases are 57% homologous (38% identical amino acids) in spite of their different species of origin (human *trk* vs. mouse *trkB*). Similar observations have been made regarding the specificity of neurotrophic factor binding. It has been reported that competition of NGF binding to its high affinity receptors by the related neurotrophic factor $\beta$DNF requires a 1000-fold excess of protein [Rodriguez-Tebar, A. et al., Neuron 4, 487-492 (1990)]. These levels of specificity might be a reflection of the highly specialized function of these neurotrophic factors. Finally, the structural and functional similarities of the three known members of the NGF family of neurotrophic factors raises the possibility that $\beta$DNF and/or NT3 may use other members of the *trk* family of tyrosine protein kinases [Barbacid, M. et al., Biochem. Biophys. Acta Rev. on Cancer, in press (1991)] as their high affinity receptors.

Example 2

I. EXPERIMENTAL PROCEDURES

A. Cells and neurotrophic factors

Cells, including NIH3T3 [Jainchill, J.L. et al., J. Virol. 4, 549-553 (1969)], E25-48, B35-41 (See Example 1 herein above) and B38-94 cell lines were grown in Dulbecco's modified Eagle's medium (DMEM) containing 10% calf serum. B38-94 cells were obtained by transfecting NIH3T3 cells with pRK25, a mammalian expression plasmid containing the *trk*5 oncogene [Coulier, F. et al., Mol. Cell. Biol. 10, 4202-4210 (1990)]. PC12 cells [Greene, L.A. and Tischler, A.S., Proc. Natl. Acad. Sci. 73, 2424-2428, (1976)] were grown on collagen-coated (Vitrogen) dishes in DMEM containing 10% horse serum and 5% fetal calf serum. NIH3T3, E25-48 and B38-94 cells were grown in semisolid media as described in Ozanne, B. et al., J. Cell Physiol. 105, 163-180 (1980). Gene transfer assays were performed by the calcium-phosphate precipitation technique as described in Graham, F.L. and van der Eb, A.J., Virology 52, 457-467 (1973).

Murine 2.5S NGF was purchased from Upstate Biotechnology, Inc. [$^{125}$I]-labeled NGF was obtained from Amersham (1,500 Ci/mmol). Human BDNF and NT-3 were produced using a baculovirus expression system [Summers, M.D. and Smith, G.E., In, Bulletin No. 1555, College Station, Texas: Texas Agricultural Experiment Station and Texas A&M Univ., pp. 10-39 (1987)]. Human BDNF and NT-3 cDNA cassettes containing the coding region for the pre-pro-hormones were generated by PCR amplification and subcloned into pBluescript KS(-) (Stratagene) [Jones, K.R. and Reichardt, L.F., Proc. Natl. Acad. Sci. USA 87, 8060-8064 (1990)]. The 850 bp BDNF and 960 bp NT-3 inserts were released by EcoRI/BamHI digestion and subcloned into EcoRI/BamHI linearized pVL1392 vector to generate pAcS27 (BDNF) and pAcS28 (NT-3). Properly processed, biologically active factors were harvested from 25 ml suspension cultures of Sf9 insect cells 48 hours after infection with either AcS27-6 or AcS28-3 recombinant baculoviruses. Cells were centrifuged and the cell culture supernatants dialyzed against 50 volumes of PBS at 4°C for 24 hours. Transient expression of BDNF and NT-3 using the COS cell expression system was performed as described in Example 3 herein below.

## B. Thymidine incorporation

Cells were seeded into collagen-coated 6-well plates (Vitrogen) at a density of $5 \times 10^5$ cells/well. After 24 hours, cultures were made quiescent by washing twice with serum-free DMEM followed by a 2-day incubation in DMEM containing 0.5% calf serum. After adding the corresponding growth factors, cells were incubated for 18 hours prior to the addition of 1 $\mu$Ci of [$^3$H]-thymidine (85 Ci/mmol; Amersham). Cells were incubated for an additional 4 hours, washed once with cold phosphate-buffered saline (PBS), trypsinized, and the amount of [$^3$H] thymidine incorporated into DNA determined by filtration through glass filters (Schleicher & Schuell) followed by extensive washing with deionized water. Filter-bound [$^3$H]-thymidine was measured by liquid scintillation counting.

## C. Immunofluorescence assays

Cells were grown on collagen-coated glass coverslips (Bellco) in 6-well plates at a density of $5 \times 10^5$ cells/well. Cells were made quiescent as described above and incubated with the required factors for 16 hours prior to the addition of 100 $\mu$M 5-bromodeoxyuridine (BrdUrd) (Sigma). After an additional 8 hour incubation, cells were fixed with cold methanol (4°C) for 10 minutes, rehydrated in PBS, and incubated for 30 minutes in 1.5 M HCl in order to denature the DNA. After washing the coverslips three times in PBS, cells were incubated with a 1:50 dilution of a mouse monoclonal anti-BrdUrd antibody (Becton-Dickinson) for 30 minutes at room temperature, washed with PBS and incubated with a 1:50 dilution of a donkey polyclonal anti-mouse immunoglobulin antiserum conjugated with Texas-Red (Amersham). Finally, cells were washed in PBS/Hoechst dye for 10 minutes to visualize and to count the nuclei. Coverslips were mounted using Fluoromount G (Southern Biotechnic) and slides examined under a Zeiss Microscope equipped with Ploems epifluorescence optics. A minimum of five random high power fields (x 400) were analyzed and the results expressed as number of cells showing positive nuclear fluorescence over the total number of cells counted. NGF induced c-Fos expression was detected by utilizing a similar procedure. Cells were instead incubated with a 1:500 dilution of rabbit polyclonal anti-c-Fos specific antibodies [Kovary, K. and Bravo, R., Mol. Cell. Biol. 11, 2451-2459 (1991)] followed by incubation with swine polyclonal anti-rabbit IgG conjugated with rhodamine (Becton Dickinson).

## D. Immunocytochemistry

Cells were fixed with methanol and incubated with a 1:1000 dilution of a rabbit polyclonal antiserum raised against a peptide corresponding to the carboxy-terminus of the human gp140$^{trk}$ receptor [Martin-Zanca, D. et al., Mol. Cell. Biol. 9, 24-33 (1989)] followed by a second incubation with a 1:1000 dilution of goat anti rabbit IgG biotinylated antibodies and a 1:25 dilution of avidin-biotin peroxidase complexes (Vector Laboratories). The sites of immunoprecipitate formation were identified using light microscopy following treatment with the chromogen 3,3'-diaminobenzidine tetrahydrochloride (DAB) (Sigma).

## E. [$^{125}$I]-NGF binding assays

Cells were harvested and washed once with ice-cold DMEM containing 0.1% BSA (DMEM-BSA). cells (2 x 10$^5$ cells per sample) were incubated with 80 $\mu$l of ice-cold DMEM-BSA containing various concentrations ranging from 4 ng/ml to 500 ng/ml of unlabeled NGF, BDNF or NT-3 at 4°C for 1 hour. 20 $\mu$l of ice-cold DMEM-BSA containing 5 ng/ml or 25 ng/ml of [$^{125}$I]-labeled NGF were added to each sample to reach a final concentration of 1 ng/ml or 5 ng/ml of [$^{125}$I]-labeled NGF. Cells were further incubated at 4°C for 2 hours, washed four times with ice-cold DMEM-BSA and lysed in 100 $\mu$l of 1N NaOH. The radioactivity associated with the cells was determined in a G5500 gamma counter [Jing, S. et al., J. Cell Biol. 110, 283-294 (1990)]. Non-specific binding was determined by measuring the radioactivity associated with control NIH3T3 cells under the same experimental conditions. Non-specific binding (<10% of the amount of [$^{125}$I]-labeled NGF bound to E25-48 cells in absence of unlabeled ligand) was subtracted from the results presented.

## F. Immunoprecipitation assays and Western blots

Metabolic labeling of E25-48 and PC12 cells with [$^{35}$S] methionine and immunoprecipitation analysis with anti-c-Fos specific antibodies was performed as described in Kovary, K. and Bravo, R., Mol. Cell. Biol. 11, 2451-2459 (1991). Detection of tyrosine phosphorylation of gp140$^{trk}$ receptors by Western blot analysis was essentially done as described in Example 1 herein above with the following modifications: mouse antiphosphotyrosine monoclonal antibodies 4G10 (Upstate Biotechnology, Inc.) were used as primary antibodies. Nitrocellulose filters were subsequently incubated with a secondary rabbit anti mouse IgG (Dako) before probing with [$^{125}$I]-labeled protein A.

II. RESULTS

A. Mitogenic activity of NGF in NIH3T3 cells expressing gp140$^{trk}$

To ascertain whether gp140$^{trk}$ receptors mediate NGF-induced signal transduction, it was first examined whether this neurotrophic factor had mitogenic activity on E25-48 cells, an NIG3T3-derived cell line expressing gp140$^{trk}$ receptors (see Example 1 herein above).

The selection of NIH3T3 cells was based on their lack of endogenous NGF receptors (See Example 1 herein above) and their capacity to respond to the *trk* tyrosine kinase, since these cells can be efficiently transformed by *trk* oncogenes. As shown in Figure 9A, addition of NGF to quiescent NIH3T3 cells did not elicit any mitogenic response. However, when NGF was added to E25-48 cells, an NIH3T3-derived cell line known to express gp140$^{trk}$, a significant increase in their ability to incorporate [$^{3}$H]thymidine into their DNA was observed (Fig. 9A). The amount of [$^{3}$H] thymidine incorporated by these cells in response to NGF was comparable to that observed in the presence of platelet-derived growth factor (PDGF), a well known mitogen for NIH3T3 cells and only slightly lower than that induced by 20% calf serum (Fig. 9A). In contrast, addition of equal amounts of NGF to another NIH3T3-derived cell line (B35-41 cells) which expressed the highly related gp145$^{trkB}$ tyrosine protein kinase [Klein, R. et al., EMBO J. 8, 3701-3709 (1989); Middlemas, D.S. et al., Mol. Cell. Biol. 11, 143-153 (1991)] did not induce significant [$^{3}$H] thymidine incorporation (Fig. 9A). Moreover, the mitogenic effect of NGF on gp140$^{trk}$ expressing E25-48 cells was completely abolished by the addition of a monoclonal antibody elicited against NGF (Fig. 9B).

B. Induction of DNA synthesis.

In order to determine the percentage of E25-48 cells responsive to NGF, DNA synthesis was examined by immunofluorescence analysis of cells incubated in the presence of 5-bromodeoxyuridine (BrdUrd). As shown in Figure 10, addition of 20% calf serum to quiescent NIH3T3 and E25-48 cells induced most (≧70%) of these cells to enter the S phase. As a control, insulin, a growth factor that does not have mitogenic properties in NIH3T3 cells only elicited DNA synthesis in 1% of NIH3T3 cells and in 6% of E25-48 cells (Fig. 10, Table 2). A similar percentage of NIH3T3 cells transfected with control pSV2*neo* plasmid DNA were capable of entering the S phase in the absence of added mitogen. Addition of saturating amounts of NGF to NIH3T3 cells failed to elicit any detectable mitogenic response (Fig. 10). However, when this neurotrophic factor was added to E25-48 cells, more than 40% of them initiated DNA synthesis (Fig. 10, Table 2).

E25-48 cells were derived from a single neomycin-resistant colony and have maintained their *neo*$^{R}$ phenotype and consistent levels of gp140$^{trk}$ expression during storage and subsequent culture. Yet, only a fraction of these cells respond to the mitogenic effect of NGF. Therefore, it was decided to examine whether gp140$^{trk}$ was homogeneously expressed in this cell line. For this purpose, E25-48 cells were submitted to immunocytochemical analysis using polyclonal rabbit antibodies elicited against the carboxy-terminus of the *trk* proto-oncogene product. As shown in Figure 11, only about half of the E25-48 cells expressed significant levels of gp140$^{trk}$. More importantly, those cells expressing gp140$^{trk}$ were those that responded to NGF (Fig. 11 C,D). These results indicate that expression of the *trk* proto-oncogene in heterologous NIH3T3 cells confers them with mitogenic responsiveness to NGF.

C. NGF induces cells to grow in semisolid media

Although DNA synthesis is often utilized as a parameter to determine mitogenic activity, the above experiments do not resolve whether NGF has a significant effect on the proliferative properties of cells expressing gp140$^{trk}$. For this purpose, it was examined whether NGF could induce E25-48 cells to grow in semisolid media. As illustrated in Table 3, E25-48 cells in the presence of 5% calf serum exhibited a very limited growth under these conditions. However, addition of NGF resulted in a 7 to 10-fold induction in the number of E25-48 agar colonies. More importantly, the percentage of E25-48 cells capable of growing in semisolid media as a result of the mitogenic properties of NGF was comparable to that of B38-94 cells, a tumorigenic NIH3T3-derived cell line transformed by a *trk* oncogene.

D. NGF and cell transformation.

The effect of NGF on the growth properties of E25-48 cells in semi-solid media raised the possibility that this neurotrophic factor may also confer transforming properties to the product of the *trk* proto-oncogene. To test this hypothesis, NIH3T3 cells were transfected with pDM69, a mammalian expression vector containing the human *trk* proto-oncogene, either in the presence or absence of 30 ng/ml of NGF. As shown in Table 4, expression of the *trk* proto-oncogene in the presence of NGF elicited the transformation of the recipient NIH3T3 cells with a specific activity of $2\times10^4$ ffu per $\mu$g of pDM69 DNA. This transforming activity is similar to that of most known oncogenes and just 5- to 10-fold lower than that of *trk* oncogenes isolated from human tumors (Table 4). In agreement with previous studies, no significant transforming activity could be observed in the absence of NGF [Martin-Zanca, D. et al., Mol. Cell. Biol. 9, 24-33 (1989)].

Most *trk* oncogenes lose their extracellular ligand binding domain during their oncogenic activation. However, it has been previously reported that certain mutations in the extra-cellular domain of the *trk* proto-oncogene can also confer transforming properties to its gene product [Oskam, R. et al., Proc. Natl. Acad. Sci. 85, 2964-2968 (1988); Coulier, F. et al., Mol. Cell. Biol. 10, 4202-4210 (1990)]. One of these oncogenes, designated *trk*S345, was generated by replacing a cysteine residue (cys$^{345}$) by serine [Coulier et al., Mol. Cell. Biol. 10, 4202-4210, (1990)]. This mutation confers to the *trk*S345 gene modest transforming activity ($10^2$ to $10^3$ ffu/$\mu$g) (Table 4). However, in the presence of NGF, *trk*S345 exhibited a transforming activity ($2 \times 10^5$ ffu/$\mu$g) slightly higher than that of the original human *trk* oncogene isolate [Martin-Zanca, D. et al., Nature 319, 743-748 (1986)] (Table 4). These results illustrate an intimate connection between the mitogenic properties of NGF and the ability of the tyrosine protein kinase of the *trk* proto-oncogene product to induce cell transformation.

E. gp140$^{trk}$ is also a receptor for neurotrophin-3

Two NGF-related neurotrophic factors, brain-derived neurotrophic factor (BDNF) and neurotrophin -3 (NT-3), have been recently isolated and characterized at the molecular level [Barde, Y.A. et al., EMBO J. 1, 549-553 (1982); Leibrock, J. et al., Nature 341, 149-152 (1989); Hohn, A. et al., Nature 344, 339-341 (1990); Jones, K.R. and Reichardt, L.F., Proc. Natl. Acad. Sci. USA 87, 8060-8064 (1990); Maisonpierre, D.C. et al., Science 247, 1446-1451 (1990); Rosenthal, A. et al., Neuron 4, 767-773, (1990)]. To determine whether any of these neurotrophic factors may also interact with the product of the *trk* proto-oncogene, partially purified BDNF and NT-3 proteins were obtained from supernatants derived from Sf9 cells infected with recombinant baculoviruses carrying BDNF and NT-3 cDNA clones, respectively. These baculovirus-expressed proteins were tested for their ability to compete with [$^{125}$I]-labeled NGF for binding to the gp140$^{trk}$ receptors expressed in E25-48 cells. As shown in Figure 12, NT-3, but not BDNF, was able to compete with NGF for binding to the *trk* proto-oncogene product, gp140$^{trk}$. The observed competition was not complete even at saturating amounts of the partially purified NT-3 protein, suggesting that this neurotrophic factor recognizes a domain in the gp140$^{trk}$ receptor that overlaps with, but is not identical to that responsible for NGF binding.

The interaction of NT-3 with gp140$^{trk}$ may have physiological relevance since partially purified NT-3 elicited mitogenic activity in E25-48 cells. As illustrated in Figure 13, addition of NT-3 to gp140$^{trk}$-expressing E25-48 cells resulted in the induction of DNA synthesis in a significant percentage of cells. No effect was observed when this factor was added to control NIH3T3 cells (Table 5). Quantitative analysis revealed that NT-3 has a more limited mitogenic effect on E25-48 cells than NGF (Table 5). Whereas most ($\geq$90%) of the E25-48 cells expressing detectable levels of gp140$^{trk}$ entered the S phase upon NGF stimulation, only about half of these cells responded to NT-3 (Table 5).

F. gp140$^{trk}$ mediates induction of c-Fos by neurotrophic factors

It has been previously shown that addition of NGF to PC12 cells results in the rapid phosphorylation of gp140$^{trk}$ receptors [See Example 1 herein above and Kaplan, D.R. et al., Nature 350, 158-160 (1991)] followed by the induction of *c-Fos* gene expression [Curran, T. and Morgan, J.I., Science 229, 1265-1268 (1985); Greenberg, M.E. et al., J. Biol. Chem. 260, 14101-14110 (1985); Kruijer, W. et al., Proc. Natl. Acad. Sci. USA 82, 7330-7334 (1985)]. In order to determine whether the mitogenic effect of NGF and NT-3 on NIH3T3 cells followed similar signal transduction pathways, the levels of tyrosine phosphorylation of gp140$^{trk}$ in E25-48 cells treated with either purified NGF or with supernatants of Sf9 cells infected with NT-3 recombinant baculoviruses were examined. As shown in Figure 14, both NGF and NT-3 elicited a rapid increase in the phosphorylation levels of gp140$^{trk}$ receptors present in these cells. However, no significant effect was observed with Sf9 supernatants containing BDNF. Similar results were obtained with different preparations of NT-3 and BDNF derived from supernatants of COS cells transiently transfected with CMV/SV40-derived NT-3 and BDNF expression vectors (Fig. 14 D).

Addition of NGF and NT-3 to E25-48 cells induced the rapid expression of c-Fos proteins as determined by immuofluorescence analysis (Fig. 15). No such effect was observed when E25-48 cells were replaced by parental NIH3T3 cells. As summarized in Table 6, NGF was somewhat more effective in inducing c-Fos expression than NT-3, thus supporting the concept that NGF is more efficient than NT-3 in mediating signal transduction through gp140$^{trk}$ receptors. Finally, kinetic studies of the induction of c-Fos expression in E25-48 cells as a response to NGF were conducted. As shown in Figure 14, c-Fos proteins were rapidly synthesized as a response to NGF, reaching maximal expression at 30 to 60 minutes after addition of the neurotrophic factor. The induced c-Fos proteins disappeared 3 hours after NGF stimulation. Parallel studies with the NGF-responsive PC12 cells revealed similar kinetics (Fig. 16). These results suggest that the mitogenic activity of neurotrophic factors in heterologous cells expressing functional gp140$^{trk}$ receptors is transmitted by signal transduction pathways related to those used to exert their neurotrophic properties.

## III. DISCUSSION

The present studies demonstrate that NGF has strong mitogenic activity in heterologous NIH3T3-derived E25-48 cells which express the *trk* tyrosine protein kinase gp140$^{trk}$. Addition of NGF to quiescent E25-48 cells resulted in the induction of DNA synthesis in essentially each of the cells that expressed gp140$^{trk}$ receptors. Such a response did not require the presence of additional factors, except insulin. Perhaps, the most dramatic illustration of the mitogenic properties of NGF resides in its ability to induce E25-48 cells to grow in semi-solid agar and to induce the morphologic transformation of NIH3T3 cells transfected with the *trk* proto-oncogene. These observations indicate that NGF has a mitogenic activity comparable to that of growth factors with oncogenic potential such as v-*sis*/PDGF, EFG/TGFa and some of the members of the FGF family [Deuel, T. F., Annu. Rev. Cell. Biol. 3, 443-492 (1987); Ross, R., Annu. Rev. Med. 38, 71-79 (1987); Burgess, W.H. and Maciag, J., Annu. Rev. Biochem. 58, 575-606 (1989)]. NGF also contributed to the morphologic transformation of NIH3T3 cells induced by the *trk*S345 oncogene, a mutated *trk* receptor in which one of the cysteine residues conserved among the three members of the *trk* gene family (cys$^{345}$), was replaced by serine [Coulier, F. et al., Mol. Cell. Biol. 10, 4202-4210 (1990)]. Removal of NGF from morphologically transformed cultures of NIH3T3 cells expressing either the normal *trk* proto-oncogene or its mutated allele *trk*S345 causes the rapid reversion of their transformed phenotype, thus adding support to the concept that cellular transformation is a direct response to the mitogenic stimulus of NGF on gp140$^{trk}$ receptors.

It has been recently proposed that the biological effects of NGF are mediated by high affinity NGF receptors that encompass the low affinity gp80$^{LNGFR}$ NGF binding protein and the gp140$^{trk}$ tyrosine kinase [Hempstead, B.L. et al., Nature 350, 678-683 (1991)]. However, it has been shown that E25-48 cells do not express detectable levels of gp80$^{LNGFR}$ (See Example 1 herein above). Therefore, the observed mitogenic effects of NGF on these cells must be solely mediated by gp140$^{trk}$ receptors. Similar observations have been recently reported by Nebreda, A.R. et al., Science 252, 558-561 (1991) who were able to induce the maturation of *Xenopus* oocytes by microinjecting transcripts encoding the human *trk* proto-oncogene product. Moreover, Weskamp and Reichardt [Neuron 6, 649-663 (1991)] have demonstrated that blocking NGF binding to gp80$^{LNGFR}$ does not prevent NGF from inducing neuronal survival or neurite outgrowth. These findings, taken together, suggest that gp140$^{trk}$, regardless of whether it binds NGF with high (picomolar) (See Example 1 herein above.) or low (nanomolar) [Hempstead, B.L. et al., Nature 350, 678-683 (1991); Kaplan, D.R. et al., Science 252, 554-558 (1991)] affinities, are the functional receptors responsible for mediating the biological activities of NGF.

The product of the *trk* proto-oncogene may also serve as a receptor for neurotrophic factors other than NGF. Competition experiments indicated that NT-3, but not BDNF, partially displaced [$^{125}$I]-NGF from binding to gp140$^{trk}$. Since saturating amounts of NT-3 only displaced about 60% of the bound NGF, it is likely that these factors compete for overlapping but not identical binding sites. The interaction of NT-3 with gp140$^{trk}$ receptors elicits a limited functional response. Addition of partially purified NT-3 produced in a baculovirus expression system stimulates thymidine incorporation in E25-48 cells and induces a significant number of these cells to enter the S phase. However, the overall mitogenic effect of NT-3 on E25-48 cells appears to be more limited than that of NGF. NT-3, like NGF, promotes neurite outgrowth and induces the neural differentiation of PC12 cells [Rosenthal, A. et al., Neuron 4, 767-773 (1990)]. Whether the neurotrophic effects of NT-3 are mediated by gp140$^{trk}$ or by another neurotrophic receptor, remains to be determined.

The interaction of NGF and NT-3 with gp140$^{trk}$ receptors in heterologous E25-48 cells appears to activate signal transduction pathways similar to those observed in PC12 cells. Addition of either NGF or NT-3 to quiescent E25-48 cells elicits the rapid phosphorylation of tyrosine residues in gp140$^{trk}$ [See Example 1 herein above and Kaplan, D.R. et al., Nature 350, 158-160 (1991)]. Moreover, both of these neurotrophic factors induced the rapid expression of the c-$Fos$ gene, a hallmark of the biochemical response of PC12 cells to NGF [Curran, T. and Morgan, J.I., Science 229, 1265-1268 (1985); Greenberg, M.E. et al., J. Biol. Chem. 260, 14101-14110 (1985); Kruijer, W. et al., Proc. Natl. Acad. Sci. USA 82, 7330-7334 (1985)]. Immunoprecipitation analysis of c-Fos proteins in NGF-stimulated PC12 and E25-48 cells revealed similar kinetics, comparable levels of expression and an indistinguishable pattern of phosphorylation, as deduced from their heterogeneous electrophoretical mobilities. These observations raise the possibility that NGF and NT-3 may utilize similar signal transduction pathways to elicit their mitogenic responses in dividing cells and their neutrophic effects in non-dividing neuronal cells.

NGF, NT-3 and BDNF are highly related molecules even in their pro-hormone form. As mature neurotrophic factors, they exhibit almost 60% amino acid identity, including their six cysteine residues which presumably play a major role in determining their tertiary structure [Barde, Y.A. et al., EMBO J. 1, 549-553 (1982); Leibrock, J. et al., Nature 341, 149-152 (1989); Hohn, A. et al., Nature 344, 339-341 (1990); Jones, K.R. and Reichardt, L.F., Proc. Natl. Acad. Sci. USA, 87, 8060-8064 (1990); Maisonpierre, P.C. et al., Science 247, 1446-1451 (1990); Rosenthal,A. et al., Neuron 4, 767-773 (1990)]. Based on their primary amino acid sequences, each of these factors is equally related to the other two. Yet, their specificities for the members of the $trk$ family of receptors are significantly different. NGF interacts with gp140$^{trk}$ receptors with high affinity, yet it does not bind to the highly related gp145$^{trkB}$ tyrosine kinase (See Example 1 herein above).

Recent studies have indicated that whereas NT-3 is widely expressed in immature regions of the central nervous system, NGF and BDNF expression is largely restricted to specific structures [Maisonpierre, P.C. et al., Neuron 5, 501-509 (1990); Kaisho, Y. et al., Biochem. Biophys. Res. Commun. 174, 379-385 (1991)]. The widely distributed expression of NT-3 dissipates as the developing structures reach maturity. Therefore, it is tempting to speculate that NT-3 may play an important role in the early stages of development, a time in which its broad reactivity might be advantageous to the embryo. However, additional biological studies along with a more precise definition of the pattern of gp140$^{trk}$ expression in the embryonic and adult nervous system will be required to establish the extent of the involvement of this tyrosine protein kinase in mediating the neurotrophic properties of NGF and NT-3.

Example 3

I. EXPERIMENTAL PROCEDURES

A. Cells and expression plasmids.

Cells, including NIH3T3 [Jainchill, J.L. et al., J. Virol. 4, 549-553 (1969)], B35-41 and E25-48 (See Example 1 above), Z52-17 and Z82-41 cells were grown in Dulbecco's modified Eagle's medium (DMEM) containing 10% calf serum. Z52-17 cells were obtained by transfecting NIH3T3 cells with pFRK44, a pMEX $neo$-derived expression plasmid [Martin-Zanca, D. et al., Mol. Cell. Biol. 9, 24-33 (1989)] containing the mouse $trkB$ cDNA encoding the catalytic gp145$^{trkB}$ protein [Klein, R. et al., EMBO J. 8, 3701-3709 (1989)]. Z82-41 cells were generated by co-transfection of NIH3T3 cells with pFRK44 and pLL42 DNAs. pLL42 is a pMEX-derived expression plasmid [Martin-Zanca, D. et al., Mol. Cell. Biol. 9, 24-33 (1989)] which contains an 850 bp insert encoding the human prepro-form of BDNF [Jones, K.R. and Reichardt, L.F., Proc. Natl. Acad. Sci. USA 87, 8060-8064 (1990)]. pLL43 is a similar pMEX-derived plasmid which contains a 930 bp insert corresponding to the human prepro-form of NT-3 [Jones, K.R. and Reichardt, L.F., Proc. Natl. Acad. Sci. USA 87, 8060-8064 (1990)]. pLTRSNGF is a pBR322 based expression plasmid containing a 960 bp insert encoding the mouse prepro-form of NGF flanked by the Moloney MSV-LTR and the SV40 polyadenylation signal. pVN1 and pVN2 expression plasmids were generated by amplifying the corresponding inserts of pLL42 and pLL43 and subcloning them into pCM7B, a COS cell expression vector that contains the cytomegalovirus enhancer/promoter element and the SV40 polyadenylation signal [Aruffo, A. and Seed, B., Proc. Natl. Acad. Sci. USA 84, 8573-8577 (1987)]. Gene transfer assays in NIH3T3 cells were performed by the calcium-phosphate precipitation technique, as previously described [Graham, F.L. and van der Eb, A.J., Virology 52, 456-467 (1973)].

### B. Neurotrophic factors

Murine 2.5S NGF was purchased from Upstate Biotechnology, Inc. Expression of human BDNF and NT-3 in a baculovirus expression system [Summers, M.D. and Smith, G.E. In Bulletin No. 1555, College Station, Texas: Texas Agricultural Experiment Station and Texas A&M Univ., pp. 10-39 (1987)] is described in Example 2 herein above. Purification of BDNF and NT-3 was basically done as described [Barde, Y.A. et al., EMBO J. 1, 549-553 (1982)]. Cell-free supernatants from Sf9 insect cells infected with recombinant baculoviruses pAcS27 (BDNF) and pAcS28 (NT-3) (See Example 2 herein above) were batch adsorbed onto CM-52 cation exchange (Whatmann) previously equilibrated with 100 mM sodium phosphate buffer pH 6.5, containing 10% glycerol, 25 mM NaCl, 1 mM EDTA, and 1mM PMSF. Bound proteins were eluted with a linear NaCl gradient. Fractions eluting at 0.4-0.5 M NaCl were collected, dialyzed against 10 mM sodium phosphate buffer pH 6.8, lyophilized and reconstituted in 10 mM sodium phosphate buffer pH 6.8, containing 25 mM NaCl. This procedure yielded approximately 90% pure BDNF and NT-3 as judged by coomassie-stained SDS-polyacrylamide gels.

Transient expression of BDNF and NT-3 using a COS cell expression system was essentially performed as described [Aruffo, A. and Seed, B., Proc. Natl. Acad. Sci. USA 84, 8573-8577 (1987)]. $5 \times 10^6$ COS cells were transfected with 10 $\mu$g of pVN1 (BDNF) and pVN2 (NT-3) DNAs and 24 hours later split at a 1:2 dilution. After an additional 24 hour incubation in DMEM media containing 10% calf serum, cells were placed in serum-free DMEM media containing 5 $\mu$g/ml of insulin and transferrin (Sigma). Cell-free supernatants were harvested 4 days later.

### C. Immunoblotting.

Detection of phosphorylated tyrosine residues in gp140$^{trk}$ and gp145$^{trkB}$ receptors was carried out by Western blot analysis as described in Example 1 herein above. Cell extracts were immunoprecipitated with a rabbit polyclonal antiserum raised against a peptide corresponding to the 14 carboxy-terminal amino acid residues of the human *trk* proto-oncogene product [Martin-Zanca, D. et al., Mol. Cell. Biol. 9, 24-33 (1989)] or against the mouse *trk*B tyrosine kinase domain expressed in bacteria [Klein, R. et al., Cell 61, 647-656 (1990)]. Mouse antiphosphotyrosine monoclonal antibodies (UBI) were then used as primary antibodies after transfer of the immunoprecipitated proteins onto nitrocellulose filters. Filters were subsequently incubated with rabbit anti-mouse IgG (Dako) before probing with [$^{125}$I]-labeled protein A.

### D. DNA synthesis.

Induction of DNA synthesis in quiescent cells following stimulation by neurotrophic factors was measured by incorporation of [$^3$H]-labeled thymidine into newly synthesized DNA as described in Example 2 herein above. The percentage of cells entering the S phase was determined by immunofluorescence assays on cells incubated in the presence of 5-bromodeoxyuridine (BrdUrd) as described in Example 2 herein above. Fixed cells were incubated with a 1:50 dilution of a mouse monoclonal anti-BrdUrd antibody (Becton-Dickinson), washed with PBS and subsequently incubated with a 1:50 dilution of a donkey polyclonal anti-mouse immunoglobulin anti-serum conjugated with Texas-Red (Amersham).

### E. Binding assays.

Purified baculovirus-expressed NT-3 was radioiodinated using [$^{125}$I]-labeled Bolton-Hunter reagent (2 mCi per reaction; 4000 Ci/mmol; Dupont) as previously described [Rodriguez-Tebar and Barde, J. Neurosci. 8, 3337-3342 (1988)]. The labeled protein was separated by gel filtration through a G-25 column (medium) pre-equilibrated with 50 mM sodium acetate (pH 4.0) containing 150 mM NaCl and 0.5% (w/v) BSA and was eluted with the same buffer at a flow rate of 0.2-0.25 ml/min. Fractions of approximately 0.5 ml were collected and the radioactivity in each fraction was determined in an Atomlab 100 gamma counter. The fractions containing the protein (3-4 fractions) were pooled. Analysis of the purity of [$^{125}$I]-labeled NT-3 by 20% SDS-polyacrylamide gel electrophoresis did not reveal other significant protein species present in the NT-3 preparation.

Competition binding assays were essentially done as described in Example 2 herein above [See also, Jing, S. et al., J. Cell Biol. 110, 283-294 (1990)] Cells ($2 \times 10^5$ per sample) were preincubated with various concentrations (0.5 ng/ml to 1 $\mu$g/ml) of unlabeled factors at 4°C for 1 hour. The same volume of [$^{125}$I]-labeled NT-3 was added to each sample to reach a final concentration of 5 ng/ml. Cells were further incubated at 4°C for 2 hours, washed four times with ice-cold DMEM containing 0.1% BSA, lysed in 100 $\mu$l

of 1 M NaOH, and the radioactivity counted in a G5500 gamma counter (Beckmann).

## II. RESULTS

### A. Transformation assays identify receptor/ligand relationships

As noted in Example 2 herein above, NGF, and to a lesser extent NT-3, are mitogens for NIH3T3 cells expressing gp140$^{trk}$ receptors. Moreover, NIH3T3 cells become morphologically transformed when they express the *trk* proto-oncogene in the presence of exogenous NGF. Therefore, it was investigated whether co-tranfection of NIH3T3 cells with DNAs encoding the *trk* proto-oncogene and either of its two cognate ligands, NGF and NT-3, may also lead to morphologic transformation. As shown in Table 7, co-transfection of saturating amounts of the *trk* proto-oncogene expression plasmid pDM69 [Martin-Zanca, D. et al., Mol. Cell. Biol. 9, 24-33 (1989)] with various amounts of pLTRSNGF, a mammalian NGF expression plasmid, led to the induction of multiple foci of transformed cells. The number of foci was directly proportional to the amount of pLTRSNGF DNA added to the recipient NIH3T3 cells (Table 7). Similarly, co-transfection of pDM69 with pLL43, a mammalian expression plasmid that directs the synthesis of NT-3, also resulted in morphologic transformation of NIH3T3 cells (Table 7). However, NT-3 was found to be significantly (200-fold) less efficient than NGF in these transformation assays. These observations are in agreement with the results described in Example 2 herein above indicating that NT-3 has a more limited mitogenic effect than NGF on gp140$^{trk}$-expressing NIH3T3 cells. No transformation was observed when pDM69 was co-transfected with the corresponding BDNF expression plasmid, pLL42 (Table 7).

### B. Co-expression of *trk*B with BDNF or NT-3 induces cell transformation

The above results suggest that transformation of NIH3T3 cells can be used as an experimental approach to investigate the ligand/receptor relationships between the *trk* and NGF families of neurotrophic molecules. To examine this possibility, NIH3T3 cells were co-transfected with a series of MSV-LTR driven mammalian expression plasmids (see Experimental Procedures) encoding the gp145$^{trkB}$ tyrosine kinase (pFRK44) and each of the three neurotrophic factors, NGF (pLTRSNGF), BDNF (pLL42) and NT-3 (pLL43) (Table 8). As illustrated in Figure 17, co-transfection of saturating amounts of pFRK44 DNA (2$\mu$g) with serial dilutions of pLL42 DNA resulted in the efficient transformation of NIH3T3 cells. Quantitative analysis of these co-transfection assays (Table 8) indicated that BDNF can transform NIH3T3 cells with an efficiency of 4 x 10$^4$ ffu per $\mu$g of DNA (Table 9), a transforming activity comparable to that of *trk* receptors, since pLL42 DNA failed to induce any detectable morphologic transformation when transfected alone into NIH3T3 cells.

Co-transfection of pFRK44 (*trk*B) and pLL43 (NT-3) DNAs also resulted in morphologic transformation of the recipient NIH3T3 cells (Table 8), albeit with a 500-fold lower transformation efficiency (Table 9). Co-expression of pFRK44 and pLTRSNGF DNAs did not lead to detectable morphologic transformation (Table 9). This result is in agreement with the results reported in Example 1 herein above that [$^{125}$I]-labeled NGF does not bind to NIH3T3 cells expressing the gp145$^{trkB}$ kinase. These findings, taken together, indicate that gp145$^{trkB}$ may be a functional receptor for BDNF and NT-3 neurotrophic factors.

### C. Phosphorylation of gp145$^{trkB}$ receptors by BDNF and NT-3

Recent studies have shown that the interaction of NGF with its functional gp140$^{trk}$ receptors elicits its rapid phosphorylation in tyrosine residues [See results reported in Example 1 herein above and Kaplan, B.R. et al., Nature 350, 158-160 (1991)]. To examine whether BDNF and/or NT-3 could induce a similar response on the gp145$^{trkB}$ kinase, Z52-17 cells, a NIH 3T3-derived cell line expressing gp145$^{trkB}$, were incubated with NGF, BDNF and NT-3. BDNF and NT-3 were derived from Sf9 insect cells infected with recombinant baculoviruses or from COS cells transfected with CMV/SV40-derived BDNF and NT-3 expression vectors (see Experimental Procedures). As shown in Figure 18, BDNF and NT-3 stimulated the rapid phosphorylation of tyrosine residues in gp145$^{trkB}$ receptors regardless of whether they were synthesized in mammalian (Fig. 18B) or insect (Fig. 18C) cells. In contrast, neither NGF or control supernatants from COS or Sf9 cells induced detectable phosphorylating activity.

Next, the extent of phosphorylation of gp145$^{trkB}$ receptors expressed in Z82-41 cells, a transformed cell line generated by co-transfection of *trk*B and BDNF DNAs, was investigated. As shown in Figure 18D, Z82-41 cells expressed constitutively phosphorylated gp145$^{trkB}$ receptors. The extent of phosphorylation of these receptors in the absence of ligand was at least comparable to that of gp145$^{trkB}$ expressed in Z52-17 cells in the presence of saturating amounts of BDNF (50 ng/ml). In addition to gp145$^{trkB}$, Z82-41 transformed cells contain a second molecular species constitutively phosphorylated in tyrosine residues (Fig. 18D). This protein is likely to correspond to partially glycosylated gp145$^{trkB}$ precursors. These precursors do not become phosphorylated as a response to exogenously added ligands, suggesting that they are not located in the cell membrane (Fig. 18). Therefore, their constitutive phosphorylation in Z82-41 cells raises the possibility that BDNF can recognize *trk*B receptors intracellularly before they are completely processed and properly anchored in the plasma membrane. Whether this interaction elicits a flow of signal transduction remains to be determined.

### D. Mitogenic activity of BDNF and NT-3.

As described in Example 2 herein above, gp140$^{trk}$ receptors mediate the mitogenic activity of their cognate NGF and NT-3 ligands on NIH3T3 cells. To examine whether BDNF and NT-3 have a similar activity on cells expressing gp145$^{trkB}$ receptors, quiescent Z52-17 cells were incubated with partially purified BDNF and NT-3 factors derived from Sf9 insect cells infect with the corresponding recombinant baculoviruses. As controls, E25-48 cells, a NIH3T3 cell line that expresses gp140$^{trk}$ receptors (See Example 1 herein above) were utilized. As shown in Figure 19A, NGF and to a lesser extent NT-3, stimulated the incorporation of [$^3$H]-labeled thymidine in these E25-48 cells. Neither BDNF nor control supernatants obtained from Sf9 cells infected with wild type baculoviruses had any significant effect. When the same experiment was performed on Z52-17 cells, both BDNF and NT-3 induced DNA synthesis in a dose-dependent manner with half-maximal stimulation in the 5 to 10 ng/ml range (Fig. 19B). Quiescent Z52-17 cells could not be stimulated either by NGF or by control supernatants (Fig. 19B). Similar results were obtained when these partially purified baculovirus-expressed factors were replaced with crude preparations derived from BDNF and NT-3 transfected COS cells. Finally, the parental NIH3T3 cells did not respond to any of the neurotrophic factor preparations used in these experiments.

To determine the percentage of Z52-17 cells responsive to BDNF and NT-3, DNA synthesis assays were conducted in the presence of 5-bromodeoxyuridine. As illustrated in Figure 20A and B, stimulation of quiescent NIH3T3 and Z52-17 cells with 20% calf serum induced more than 80% of these cells to enter the S phase within 24 hours. Neither insulin nor NGF elicited significant DNA synthesis (Fig. 20C-F). In contrast, supernatants derived from COS cells transfected with BDNF and NT-3 expression plasmids induced 64% and 47% of Z52-17 cells to enter the S phase, respectively (Fig. 20H,J and Table 10). As negative controls, neither BDNF or NT-3 containing COS cell-derived supernatants had significant mitogenic activity on the parental NIH3T3 cells (Fig. 20G,I). Immunocytochemistry studies revealed that about 80% of Z52-17 cells expressed detectable levels of gp145$^{trkB}$ receptors, thus indicating that the percentage of Z52-17 cells responding to BDNF is similar to that responding to 20% calf serum.

The more limited mitogenic effects of NT-3 (Table 10) are in agreement with the results obtained in the transformation assays described above. Partially purified preparations (50 ng/ml) of baculovirus-derived BDNF and NT-3 had similar mitogenic effects, although they appeared to be somewhat less efficient. Whether this difference is due to their processing in Sf9 insect cells or to loss of activity during their purification remains to be determined.

### E. Binding of [$^{125}$I]-labeled NT-3 to gp145$^{trkB}$

The above results strongly suggest that gp145$^{trkB}$ is a receptor for BDNF and NT-3. However, formal proof of a direct interaction between these molecules and the gp145$^{trkB}$ kinase requires binding data. To this end, purified BDNF and NT-3 proteins isolated from baculovirus-infected Sf9 cells were iodinated as indicated in Experimental Procedures. NT-3 was efficiently iodinated using the Bolton-Hunter reagent which primarily labels lysine residues. Other iodination methods that lead to the specific labeling of tyrosine residues yielded less efficient results. NGF became equally well labeled with either method. Unfortunately, parallel efforts to iodinate the BDNF protein have been, so far, unsuccessful. Therefore, the direct binding studies were limited to the NT-3 protein.

As shown in Figure 21, incubation of NIH3T3 cells expressing gp140$^{trk}$ (E25-48 cells) or gp145*trk*B (B35-41 and Z52-17 cells) receptors with 1 nM [$^{135}$I]-labeled NT-3 at 4°C resulted in its efficient binding. Moreover a significant fraction of the observed [$^{125}$I]-labeled NT-3 binding could be readily competed by a 75-fold excess of unlabeled NT-3. In contrast, the parental NIH3T3 cells exhibited very limited binding (less than 20% of that bound to E25-48, B35-41 or Z52-17 cells), none of which could be competed with a similar excess of cold neurotrophic factor (Fig. 21). To overcome the unavailability of [$^{125}$I]-labeled BDNF, competitive bindings assays were utilized to determine whether BDNF may also bind to gp140$^{trk}$ and/or gp145$^{trkB}$ receptors. In agreement with our previous studies, NGF but not BDNF could displace binding of

[$^{125}$I]-labeled NT-3 to gp140$^{trk}$-expressing E25-48 cells, thus adding further support to the concept that BDNF does not interact with gp140$^{trk}$ receptors. In contrast, BDNF readily displaced [$^{125}$I]-labeled NT-3 from binding to gp145$^{trkB}$-expressing B35-41 cells (Fig. 22). Moreover, BDNF was several fold more effective in inhibiting [$^{125}$I]-labeled NT-3 binding than unlabeled NT-3. Similar results were obtained with Z52-17 cells. These observations indicate that BDNF binds to gp145$^{trkB}$ receptors with higher affinity than NT-3. Finally, NGF, a factor which does not bind to cells expressing the gp145$^{trkB}$ kinase (See Example 1 herein above), failed to displace [$^{125}$I]-labeled NT-3 from binding to B35-41 cells (Fig. 22).

III. DISCUSSION

As described in Example 2 herein above, the transfection of NIH3T3 cells with an expression plasmid encoding gp140$^{trk}$ results in the efficient transformation of the recipient cells only in the presence of nanomolar quantities of exogenously added NGF. In the present studies, these observations have been extended by demonstrating that NGF can confer transforming activity to the gp140$^{trk}$ kinase in an autocrine manner when the genes encoding these molecules are co-transfected into the same cell. These observations suggest that transfection assays may provide a valuable tool to unveil ligand-receptor relationships between other *trk*-related tyrosine protein kinases and the members of the NGF family of neurotrophic factors.

Co-expression of gp145$^{trkB}$ and BDNF results in transformation of NIH3T3 cells with an efficiency comparable to that of *trk* oncogenes isolated from human tumors. These results strongly suggest that BDNF is a ligand for the *trk*B kinase. This is supported by several other lines of evidence including (i) the induction of DNA synthesis upon addition of BDNF to quiescent NIH3T3 cells expressing gp145$^{trkB}$ receptors; (ii) the rapid phosphorylation in tyrosine residues of gp145$^{trkB}$ molecules in cells exposed to BDNF; (iii) the constitutive tyrosine phosphorylation of gp145$^{trkB}$ in cells co-expressing BDNF and (iv) the efficient displacement of [$^{125}$I]-labeled NT-3 from binding to cells expressing gp145$^{trkB}$ receptors. These results taken together, firmly establish gp145$^{trkB}$ as a functional receptor for BDNF.

NT-3 is also a ligand for gp145$^{trkB}$ receptors, since [$^{125}$I]-labeled NT-3 binds efficiently to cells expressing the *trk*B kinase. This interaction has functional relevance since it triggers the rapid phosphorylation in tyrosine residues of gp145$^{trkB}$ molecules and induces cells expressing these receptors to enter the S phase. However, the mitogenic activity of NT-3 on cells expressing the *trk*B kinase is more limited than that of BDNF. The differential activity of these two neurotrophic factors on gp145$^{trkB}$ expressing cells could be best quantitated by using transformation assays, where NT-3 exhibited about 400-fold lower activity than BDNF. It could be argued that the observed differences between NT-3 and BDNF may reflect lower expression levels or less efficient processing of NT-3 in NIH3T3 cells. The lack of antibodies against these factors has precluded this possibility from being ruled out. However, we favor the hypothesis that the limited biological activity of NT-3 on gp145$^{trkB}$ (as compared to BDNF) or on gp140$^{trk}$ (as compared to NGF) receptors reflects a less efficient interaction between this neurotrophic factor and these kinase receptors. This hypothesis is supported by the preliminary NT-3 binding studies discusses herein. Currently being investigated is whether NT-3 may mediate stronger biological responses through other *trk*-like receptors such as the product of the *trk*C gene. Regardless of the outcome of these studies, the data discusses herein clearly indicates that NT-3 has a wider binding spectrum than NGF and BDNF (See Examples 1 and 2 herein above). Whether this property confers NT-3 a unique physiological role among the members of the NGF family of neurotrophic factors remains to be determined.

The *trk*B gene is expressed in most structures of the peripheral and central nervous systems of the developing mouse embryo [Klein, R. et al., EMBO J. 8, 3701-3709, (1989); Klein, R. et al., Development 109, 845-850 (1990)]. In the adult, available information regarding *trk*B expression is limited to the dorsal region of the cerebrum and certain areas of the cerebellum [Klein, R. et al., Cell 61, 647-656 (1990); Klein, R. et al., Development 109, 845-850 (1990)]. *In situ* hybridization analysis conducted with probes specific for the various *trk*B transcripts has indicated that gp145$^{trkB}$ receptors are primarily expressed in the cerebral cortex, hippocampus and thalamus. Other structures such as the choroid plexus and the ependymal layer of the ventricles contain transcripts encoding the non-catalytic gp95$^{trkB}$ protein [Klein, R. et al., Cell 61, 647-656 (1990)]. Whether this protein also serves as a BDNF and/or NT-3 receptor is currently under investigation. BDNF transcripts are also widely distributed in the adult brain [Ernfors, P. et al., Neuron 5, 511-526 (1990); Hofer, M. et al., EMBO J. 9, 2459-2464, (1990); Maisonpierre, D.C. et al., Science 247, 1446-1451 (1990); Phillips, H.S. et al., Science 250, 290-294 (1990)]. Highest levels of expression have been observed in the hippocampus (both pyramidal and granular neurons), cortex, amygdala and the thalamic and hypothalamic nuclei. In the cerebellum, BDNF appears to be expressed in the internal granular layer [Hofer, M. et al., EMBO J. 9, 2459-2462 (1990)] whereas *trk*B transcripts are most prominent in the Purkinje cell layer [Klein, R. et al., Development 109, 845-850, (1990)].

The biological properties of BDNF appear to be as diverse as its pattern of expression. For instance, BDNF promotes the survival and differentiation of developing neurons of the dorsal root ganglion [Lindsay, R.M. et al., Dev. Biol. 112, 319-328 (1985); Kalcheim, C. et al., EMBO J. 6, 2871-2873 (1987)]. In addition, BDNF stimulates the survival as well as the differentiation of septal cholinergic neurons which are known to degenerate in Alzheimer's disease [Alderson, R.F. et al., Neuron 5, 297-306 (1990); Knusel, B. et al., Proc. Natl. Acad. Sci. USA 88, 961-965 (1991)]. The identification of the *trk*B tyrosine kinase as a functional receptor for BDNF should make it possible to identify the primary cellular targets of this neurotrophic factor and help to elucidate its role in the normal brain as well as its potential involvement in neurodegenerative diseases such as Alzheimer's and Parkinson's disease.

## Example 4

### I. EXPERIMENTAL PROCEDURES

### A. Isolation of cDNA clones

A λgt 10 cDNA library (1.5 x $10^6$ phages) prepared from adult male porcine brain (Clontech Laboratories, Inc.) was plated on a lawn of *Escherichia coli* C 600 Hfl. Phages were absorbed onto nitrocellulose filters and lysed. Their DNAs were hybridized under relaxed conditions (48 hours at 42°C in 5 x SSC, 40% formamide, 1 x Denhardt's solution and 10% dextran sulfate) with a nick-translated $^{32}$P-labeled probe derived from the 1.2 kb Ball-EcoRI DNA fragment of pDM17 (ATCC 41055). This insert encompasses the entire tyrosine protein kinase catalytic domain of *trk* [Martin-Zanca, D. et al., Mol. Cell. Biol. 9, 24-33 (1989). Filters were washed three times at room temperature in 2 x SSC, 0.1% SDS and once at 42°C in 0.1 x SSC, 0.1% SDS, and exposed 3 days at -70°C with intensifying screens. The filters were then washed for 3 hours at 70°C in 2.5 mM Tris pH 8.0, 0.1 mM EDTA, 0.025% sodium pyrophosphate and 0.001% Denhardt's solution.

The above library was rescreened under stringent conditions with nick-translated $^{32}$P-labeled probes derived from either pFRK46, a full length mouse *trk*B TK+ cDNA lacking the 3' untranslated region [See, Klein, R. et al., EMBO J. 8, 3701-3709 (1989)] or pDM17. Phages showing strong hybridization signals to either of these probes were discarded. Those depicting weak hybridization were picked up and plaque purified as described in Maniatis et al., supra. The inserts were subcloned into pBluescript (Stratagene). The plasmid with the largest cDNA insert was designated pFL7.

A cDNA clone containing the 5' *trk*C sequence was isolated by rescreening the library with a nick-translated $^{32}$P-labeled probe (300 bp) generated by PCR, corresponding to the 5' end of pFL7, a partial cDNA clone containing part of the ligand binding region and the complete transmembrane and tyrosine kinase catalytic domains. This probe corresponds to sequences encoding the carboxy terminus of the extracellular domain and the entire transmembrane region of the porcine *trk*C product (nucleotides 1086-1600 in Figure 1). In this case, the hybridization was performed under stringent conditions (48 hours at 42°C in 5 x SSC, 50% formamide, 1 x Denhardt's solution and 10% dextran sulfate). The positive clone was plaque purified as described previously (Maniatis et al., supra). Its 2.2 kb EcoRI insert was subcloned into pBluescript (Stratagene) to generate pFL15.

Mouse *trk*C cDNA clones were isolated from an adult mouse brain cDNA library [Citri, Y. et al., Nature 326, 42-47 (1987)]. 2 x $10^6$ phages were plated on a lawn of *Escherichia coli* LE 392, absorbed onto nitrocellulose filters and hybridized under relaxed conditions (as described above) with a 315 bp Sal I $^{32}$P-labeled DNA fragment of pFL7. Positive phages were plaque purified as described (Maniatis et al., supra). A 2.4 kb EcoRI insert, the longest insert, was subcloned into pBluescript to generate pFL16. The partial nucleotide sequence [SEQ. ID. NO.: 3] and the partial deduced amino acid sequence [SEQ. ID. NO.: 4] of the pFL16 insert is shown herein below. Plasmid pFL16 was deposited with the American Type Culture Collection, Rockville, Maryland on July 3, 1991 under the Budapest Treaty and assigned ATCC accession no. 75045.

### B. Nucleotide sequencing

The 5' end of the cDNA sequence of pFL15 was assembled to the cDNA sequence of pFL7 using a unique Nae I site to generate pFL19. Sequencing was performed using the dideoxy chain termination method with double-stranded plasmid DNA, synthetic oligonucleotides and a modified T7 DNA polymerase (Sequenase; US Biochemicals).

## C. Expression plasmids and gene transfer assays

The 2,538 bP cDNA insert of pFL19 was subcloned into the mammalian expression vector pMEX-neo [Martin-Zanca, D. et al., Mol. Cell. Biol. 9, 24-33 (1989)]. The resulting plasmid, pFL20, was linearized by Aat II digestion. Human BDNF and NT-3 cDNAs containing the coding region for the pre-prohormones were subcloned into pBluescript KS (-) [Jones, K.R. and Reichardt, K.F., Proc. Natl. Acad. Sci. USA 87, 8060-8064 (1990)]. After digestion with EcoRI, the 850 bp BDNF and 960 bp NT-3 inserts were subcloned into the expression vector pMEX to generate pLL42 and pLL43, respectively. These plasmids were linearized by Pvu I digestion.

Mouse NIH3T3 cells were transfected with the linearized plasmids according to the calcium phosphate precipitation technique to generate G418-resistant R4-31 cells. [Graham, F.L. and van der Eb, A.J., Virology 52, 456-467 (1973)].

## D. Immunoprecipitation and western blot analysis

Cells were metabolically labeled with $^{35}$S-labeled methionine (50 $\mu$Ci/ml, 1,200 Ci/mmol, Amersham) for 3 hours in the absence or presence of tunicamycin (10 $\mu$g/ml). The immunoprecipitation analysis was performed as previously described [Martin-Zanca, D. et al., Mol. Cell. Biol. 9, 24-33 (1989)] using a polyclonal antibody (43-4) raised in the rabbit against a synthetic peptide corresponding to the 14 carboxy-terminal residues of the deduced *trk* proto-oncogene sequence.

Analysis of tyrosine phosphorylation by Western blot was performed as described in Example 2 herein above.

## E. Binding assays

Binding assays were performed on cells in suspension as previously described in Example 1 herein above. Briefly, R4-31 cells were preincubated with various concentrations (0.5 ng/ml to 1 $\mu$g/ml) of unlabeled NT-3, NGF or BDNF factors for 1 hour at 4°C with 4 ng/ml of [$^{125}$I]-labeled NT-3. After being washed in ice-cold DMEM containing 0.1% BSA (DMEM/BSA), cells were lysed in 100 $\mu$l of 1 N NaOH and the radioactivity determined in a G5500 gamma counter (Beckman). The dissociation constants and number of NT-3 binding sites on R4-31 cells were determined by Scatchard plot analysis. NIH3T3 and R4-31 cells were incubated with various concentrations (0.004 nM to 8 nM) of [$^{125}$I]-labeled NT-3 at 4°C for 2 hours, washed four times in cold DMEM/BSA and resuspended in 100 $\mu$l of 1 N NaOH. The radioactivity was counted in a G5500 gamma counter. Binding of [$^{125}$I]-labeled NT-3 to NIH3T3 cells was considered as non-specific binding and subtracted from the amount of [$^{125}$I]-labeled NT-3 bound to R4-31 cells. For each concentration, non-specific binding was never higher than 15% of the amount of radioactivity bound to R4-31 cells. Binding data was analysed with the Cricket Graph program. Isolation, purification and radioiodination of the NT-3 and BDNF proteins utilized in this study has been previously described. [See, Examples 2 and 3 herein above]. Mouse 2.5 S NGF was purchased from Upstate Biotechnology, Inc.

## F. DNA synthesis analysis

Cells were grown on collagen-coated 6 well plates (Vitrogen) at a density of 5 x 10$^5$ cells/well. After 24 hours, the cells were made quiescent as described in Example 2 herein above. 5-bromodeoxyuridine (BrdUrd) (Sigma) incorporation of cells incubated in the presence or absence of neurotrophic factors were measured according to the protocol described in Example 2 herein above. Cells were fixed with cold methanol, incubated with a mouse monoclonal anti BrdUrd antibody (Becton-Dickinson) at a 1:50 dilution, washed with PBS and incubated with a 1:50 dilution of a donkey polyclonal anti-mouse immunoglobulin antiserum conjugated with Texas-Red. Finally, the cells were incubated with Hoechst dye to count the nuclei.

## II. RESULTS

## A. Molecular cloning of *trk*C cDNA clones

An adult porcine brain cDNA library was screened with a probe corresponding to the catalytic domain of the human *trk* proto-oncogene under relaxed hybridization conditions. Over 100 recombinant phages were found to be positive. Filters containing these phages were rehybridized under stringent hybridization conditions with probes specific for either *trk* or *trk*B sequences (see Experimental Procedures above) in order to identify undesirable phage carrying *trk* or *trk*B cDNA inserts. Six positive clones that hybridized only weakly to these probes were isolated, and their EcoRI inserts subcloned in pBluescript vectors and submitted to further characterization. These six clones contained overlapping inserts ranging in size from 1.9 kbp to 2.3 kbp. Restriction enzyme analysis followed by partial nucleotide sequence analysis of these clones revealed that they were highly related to but distinct from the *trk* and *trk*B proto-oncogenes. Hybridization of genomic DNAs of porcine, mouse and human origin with a 320 bp Scal-Apal DNA fragment derived from these *trk*-related cDNA clones identified a series of DNA fragments that did not hybridize to probes derived from the corresponding regions of human *trk* and mouse *trk*B cDNA clones (data not shown). These results indicate that the above cDNA clones were not derived from the porcine *trk* or *trk*B locus. Therefore, they must correspond to transcripts encoded by a new *trk*-related gene, designated as *trk*C.

B. Nucleotide and deduced amino acid sequence of *trk*C

None of the above clones contained sequences coding for the amino terminus of the putative *trk*C gene product. A small probe derived from the 5' end of the longest cDNA clone (pFL7) was used to rescreen the porcine cDNA library. Only one recombinant phage carrying a 2.2 kbp EcoRI insert was identified. This insert, which extended furthest to the 5' end, was assembled with the pFL7 insert using a common Nae I site (Figure 23A) to generate a single cDNA clone, pFL19. Plasmid pFL19 was deposited with the American Type culture Collection, Rockville, Maryland on July 3, 1991 under the Budapest Treaty and assigned ATCC accession no. 75046. The nucleotide sequence of pFL19 is shown in Figure 23B [SEQ. ID. NO.: 1]. Nucleotides 1-31 are likely to represent 5' non-coding sequences. Nucleotides 32 to 2506 correspond to a long (2475 bp) open reading frame capable of coding for an 825 amino acid long polypeptide. The predicted ATG initiator codon conforms well with the canonical sequences of mammalian initiator codons. Moreover, the presence of an in-frame terminator (TAA, nucleotides 11-13) just upstream of this ATG, supports the concept that this codon represents the translational initiator of the *trk*C gene product. The last 20 nucleotides contain the terminator codon TAG and 17 3' untranslated residues. The small size of this region along with the absence of a polyadeny-lation signal suggests that pFL19 lacks a signifi-cant fraction of the 3' untranslated region of *trk*C transcripts.

The deduced amino acid sequence of the porcine *trk*C protein encoded by pFL19 is also depicted in Figure 23B [SEQ. ID. NO.: 2]. This 825 amino acid long polypeptide (93,129 daltons) exhibits the characteristic features of cell surface tyrosine protein kinase including a signal peptide (positions 1 to 31), a long extracellular region encompassing 14 consensus N-glycosylation sites (Asn-X-Ser/Thr) (positions 32 to 429), a single transmembrane domain (positions 430 to 453) and a cytoplasmic region (positions 454 to 825) which includes the kinase catalytic domain (positions 544 to 810). The consensus sequence for the ATP binding motif is located at positions 545 to 572 [See, Hanks, S.K. et al., Science 241, 42-52 (1988)]. The *trk*C product, like the other two members of the *trk* gene family, has a very short carboxy terminal region of 15 amino acid residues which include a conserved free tyrosine residue at the carboxy-terminus.

The overall homology of the *trk*C protein to the products of the human *trk* and mouse *trk*B proto-oncogene products is 67% and 68%, respectively. Their external domains exhibit 54% (*trk*C and *trk*) and 53% (*trk*C and *trk*B) similarities. Alignment of the deduced amino acid sequences of the three members of the mammalian *trk* gene family shows that the twelve external cysteine residues of the *trk*C product are present in the corresponding region of the *trk*B proteins and ten of them are shared with the *trk* gene product. Moreover, this alignment reveals a highly conserved region (residues 368-378 of the *trk*C sequence) which depicts an 82% identity among these three kinases. Interestingly, this sequences is part of the 51 amino acid long deletion responsible for the malignant activation of the *trk*5 oncogene [Oskam, R. et al., Proc. Natl. Acad. Sci. USA 85, 8913-8917 (1988); Coulier, F. et al., Mol. Cell. Biol. 10, 4202-4210 (1990)-]. Therefore, this region may play an important role in regulating the catalytic activity of the internal kinase domain. Finally, the 267 amino acid long catalytic domain is 76% identical (87% homologous) to that of human *trk* and 83% identical (88% homologous) to that of the mouse *trk*B kinase. Much lower homologies were obtained when the sequence of the *trk*C protein was compared with other members of the cell surface tyrosine protein kinase family. The catalytic kinase region of the *trk*C product exhibits the characteristic features of the *trk* and *trk*B tyrosine kinases [Klein, R. et al., EMBO J. 8, 3701-3709 (1989); Martin-Zanca, D. et al., Mol. Cell. Biol. 9, 24-33 (1989)]. They include (i) a threonine residue (position 682) instead of the alanine present in all the other tyrosine protein kinases (with the exception of the putative tyrosine kinase JTK10 expressed in K562 human leukemia cells) [Partanen, J. et al., Proc. Natl. Acad. Sci. USA 87, 8913-8917 (1990)]; (ii) the putative autophosphorylation site, $Tyr^{709}$, is followed by a second tyrosine residue, a feature also present in the insulin receptor subfamily; (iii) a simple amino acid gap (between residues of 576 and 577); (iv) a tryptophane in position 757; and (v) the absence of a helix-breaking proline in position 801 [Hanks, S.K. et al., Science 241, 42-52 (1988)]. Finally, the *trk*C protein shares the *trk* and *trk*B kinases their characteristic short carboxy-terminal tail. This 15 amino acid long region contains eight residues identical (12 homologous) to those of the *trk* and *trk*B kinases, including a tyrosine (Tyr$^{820}$) located five residues from the carboxy-terminus.

## C. Identification of the *trk*C product gp140$^{trkC}$

The pFL19 insert was next subcloned in the mammalian expression vector pMExneo [Martin-Zanca, D. et al., Mol. Cell. Biol. 9, 24-33 (1989)], and the resulting plasmid, pFL20, used to transfect NIH3T3 cells. Several G418 resistant colonies were isolated and submitted to immunoprecipitation analysis using rabbit polyclonal antibodies elicited against a peptide corresponding to the 14 carboxy-terminal residues of the *trk* protein [Martin-Zanca, D. et al., Mol. Cell. Biol. 9, 24-33 (9189)]. As shown in Figure 24, this antiserum recognized a major protein species with an apparent molecular weight of 145,000 likely to correspond to the mature *trk*C product (designated gp145$^{trkC}$). This molecular species is likely to be a glycoprotein since in the presence of tunicamycin it exhibits a much faster electrophoretical mobility corresponding to a protein with a molecular weight of 90,000. This value corresponds well with the predicted size (93 kDa minus the signal peptide) for the polypeptide backbone of the *trk*C product. In addition to the gp145$^{trkC}$ protein, a smaller glycoprotein species of about 120,000 daltons was identified, which likely represents a partially glycosylated precursor. As expected, gp145$^{trkC}$ exhibited an *in vitro* autokinase activity specific for tyrosine residues.

## D. NIH3T3 cells expressing gp145$^{trkC}$ bind NT-3

It was next examined whether NGF, BDNF or NT-3 interact with the trkC product, gp145$^{trkC}$. A NIH3T3-derived cell line known to express gp145$^{trkC}$ (R4-31 cells) was incubated with [$^{125}$I]-labeled NT-3 (see Experimental Procedures). Only [$^{125}$I]-labeled NT-3 exhibited significant binding to R4-31 cells. As shown in Figure 25, binding of [$^{125}$I]-labeled NT-3 to R4-31 cells could be efficiently competed by unlabeled NT-3 but not by NGF or BDNF.

Scatchard plot analysis of the binding of [$^{125}$I]-labeled NT-3 to R4-31 cells revealed the presence of high affinity (26 pM) and low affinity (4 nM) binding sites (Fig. 26). The total number of [$^{25}$I]-labeled NT-3 binding sites (150,000 sites per cell) corresponds well with the estimated number of gp145$^{trkC}$ molecules present in R4-31 cells. Therefore, it is likely that both high and low affinity binding sites corresponds to gp145$^{trkC}$ receptors. The percentage of high affinity receptors in these cells (2%) is similar to that previously found in NIH3T3 cells expressing *trk* receptors (See Example 1 herein above. R4-31 cells do not express detectable levels of the previously described low affinity NGF receptor, gp80$^{LNGFR}$. These results indicate that high affinity binding of NT-3 to gp145$^{trkC}$ does not require the presence of gp80$^{LNGFR}$ receptors.

## E. gp145$^{trkC}$ mediates the mitogenic properties of NT-3

It was next examined whether the interaction of NT-3 with gp145$^{trkC}$ receptors elicits functional responses. As shown in Figure 27, incubation of NIH3T3-derived R4-31 cells with saturating amounts (50 ng/ml) of purified NT-3 induced the rapid phosphorylation of tyrosine residues in gp145$^{trkC}$. In contrast, no effect was observed when NT-3 was replaced by the same concentration of either NGF or BDNF (Fig. 27). Similar results were obtained when the baculovirus-derived NT-3 protein was replaced by partially purified supernatants of COS cells transiently transfected with the NT-3 coding plasmid, pVN2 (see Example 3 herein above).

Previous studies have indicated that NT-3 can induce DNA synthesis in resting NIH3T3 cells expressing gp140$^{trkB}$ or gp145$^{trkB}$ receptors (See Examples 2 and 3 herein above). Therefore, it was examined whether NT-3 also had mitogenic properties in cells expressing gp145$^{trkC}$. For this purpose, quiescent R4-31 cells were incubated with 50 ng/ml of partially purified NT-3 produced by COS cells. The extend of DNA synthesis was determined by immunofluorescence analysis of incorporated 5-bromodeoxyuridine (BrdUrd). The results of these experiments are illustrated in Figure 28 and summarized in Table 11. NT-3 was able to induce DNA synthesis in over 65% of the treated R4-31 cells. A much more limited stimulation was observed when NT-3 was replaced by the same concentration of either BDNF produced in COS cells (11.3%) or purified NGF (5.5%) (Table 11). The same preparation of NT-3 had no significant effect on the parental NIH3T3 cells (Table 11). These results suggest that the mitogenic properties of NT-3 on R4-31 cells are mediated by gp145$^{trkC}$ receptors.

F. Co-expression of gp145$^{trkC}$ and NT-3 induces transformation of NIH3T3 cells

As described in Examples 2 and 3 herein above, co-transfection of expression plasmids encoding gp140$^{trk}$ and NGF or gp145 $^{trkB}$ and BDNF elicits the efficient transformation of the recipient NIH3T3 cells. A more limited transforming activity was observed when the *trk* and *trk*B receptors were co-expressed with a plasmid (pLL43) encoding NT-3. In this study, similar transformation assays were performed in which 2 $\mu$g of the gp145$^{trkC}$ expression plasmid pFL20 was co-transfected with various amounts (2 $\mu$g to 2 ng) of plasmids encoding NT-3 (pLL43), NGF (pLTRSNGF) and BDNF (pLL42). As indicated in Table 12, only the NT-3 coding pLL43 DNA resulted in detectable transformation of NIH3T3 cells. These results add further support to the concept that gp145$^{trkC}$ is a receptor for NT-3 but not for NGF or BDNF. Next, a similar transformation assay was performed in which saturating amounts (1 $\mu$g) of NT-3 coding pLL43 DNA were co-transfected with various amounts (1 $\mu$g to 0.1 ng) of plasmids expressing each of the three members of the *trk* family of tyrosine protein kinases. As shown in Table 13, co-expression of NT-3 with each of these receptors resulted in morphologic transformation of the recipient NIH3T3 cells. However, the levels of transformation induced by gp145$^{trkC}$ and NT-3 were about two orders of magnitude higher than those induced by co-expression of either NT-3 and gp140$^{trk}$ or NT-3 and gp145$^{trkB}$ (Table 13). These results illustrate that, at least in NIH3T3 cells, NT-3 elicits a more powerful biological response through *trk*C receptors than through the *trk* or *trk*B kinases. These findings suggest that gp145$^{trkC}$ is a primary receptor for NT-3.

EXAMPLE 5

I. EXPERIMENTAL PROCEDURES

A. Cells and Neurotrophins

COS cells [Gluzman, Y., Cell 23, 175-182 (1981)], NIH3T3 [Jainchill, J.L., et al., J. Virol. 4, 549-553 (1969)] and NIH3T3-derived cell lines including E25-42, E25-48 [ See, Example 3 herein above and Klein, R. et al., Cell 66, 395-403 (1991)], and R4-31 [ See, Example 4 herein above and Lambelle, F. et al., Cell 66, 967-979 (1991)] were grown in Dulbecco's modified Eagle's medium (DMEM) containing 10% calf serum. E25-42 cells were obtained by transfecting NIH3T3 cells with the mammalian *trk* expression plasmid pDM38 [Martin-Zanca, D. et al., Mol. Cell. Biol. 9, 24-33 (1989)]. Rat pheochromocytomas PC12 cells [Greene, L.A. and Tischler, A.S., Proc. Natl. Acad. Sci. USA 73, 2424-2428 (1976)] were grown on collagen-coated (Vitrogen) dishes in DMEM containing 10% horse serum and 5% fetal calf serum. Murine 2.5S NGF was purchased from Upstate Biotechnology, Inc. Human BDNF and NT-3 were purified from supernatants of Sf9 insect cells infected with recombinant baculoviruses pAcS27 (BDNF) and pAcS28 (NT-3), as recently described [ See, Example 2 herein above and Cordon-Cardo, C. et al., Cell 66, 173-183 (1991)].

B. Expression plasmids

Gene transfer assays in NIH3T3 cells were performed by the calcium phosphate preceipitation technique [Graham, F.L. and van der Eb, A.J., Virology 52, 456-467 (1973)]. PC12 cells were transfected with pFRK44 or pFL20 DNAs with the help of lipofection (GIBCO) as described in Muller, S.R. et al., DNA Cell Biol. 9, 221-229 (1990). Expression plasmids include those encoding gp140$^{trk}$ (pDM69) [Martin-Zanca, D. et al., Mol. Cell. Biol. 9, 24-33 (1989)], gp145$^{trkB}$ (pFRK44) [Klein, R. et al., EMBO J. 8, 3701-3709 (1989)-], gp145$^{trkC}$ (pFL20) [ See, Example 4 herein above and Lamballe, F. et al., Cell 66, 967-979 (1991)], NGF (pLTRSNGF) (Clegg and Reichardt, unpublished), BDNF (pLL42) [ See, Example 3 herein above and Klein, R. et al., Cell 66, 395-403 (1991)] and NT-3 (pLL43) [ See, Example 3 herein above and Klein, R. et al., Cell 66, 395-403 (1991)]. pFL3 is a pMEX-derived expression plasmid encoding a mutated gp145$^{trkB}$ receptor in which the cysteine residue located at position 345 has been replaced by serine. In order to generate pFL3, pFRK1 DNA [Klein, R. et al., EMBO J. 8, 3701-3709 (1989)] was digested with the restriction enzymes NcoI and NdeI releasing a 32 bp DNA fragment (nucleotides 1538-1569 of pFRK43) including the TGC codon (nucleotides 1544-1546) encoding the cysteine residue at position 345 of gp145$^{trkB}$. A synthetic NcoI/NdeI DNA fragment which contained an A/T substitution at position 1544 generating an AGC codon encoding a serine instead of a cysteine residue was inserted into pFRK1. In addition, this NcoI/NdeI DNA fragment contained a silent G/T substitution at the third base of the CTG codon (nucleotides 1553-1555) encoding a leucine residue at position 348, thereby destroying a diagnostic PvuII site. From a subclone containing the mutated DNA fragment, a 1024 bp XhoI/ApaI DNA fragment (nucleotides 1386-2409 of pFRK43) was released and ligated into XhoI/ApaI-digested pFRK46 [Lamballe, F. et al., Cell 66, 967-979 (1991)] generating the expression of plasmid pFL3 encoding gp145$^{trkB}$/S345.

C. Cloning and Expression of *Xenopus* NT-4

The complete open reading frame of the *Xenopus laevis* NT-4 gene was obtained from genomic DNA by PCR-aided amplication using as 5' amplimer the oligonucleotide 5'-GTGGT CTGCAG TCGACTAAGTAAT GATCCTCCGC-3' [SEQ. ID NO.: 5] which contains a PstI site (underlined) eleven nucleotides upstream of the initiator ATG codon (in italics) and as the 3' amplimier the oligonucleotide 5'-CAGGTCTAGAGA GAATTC TCTTCTCTC TATTTTGCTAAC-3' [SEQ. ID NO.: 6] which contains an EcoRI site (underlined) 28 nucleotides downstream of the stop TAG codon. The amplified DNA was cloned into the pCR1000 vector using the TA cloning system (Invitrogen). The resulting plasmid, PFRK81, was submitted to nucleotide sequence analysis by the dideoxy chain termination method (Sanger, F. et al., Proc. Natl. Acad. Sci. USA 74, 5463-5467, (1977) using modified T7 DNA polymerase (Sequenase, USB). A translationally silent nucleotide difference (A$^{651}$→C$^{651}$) was observed when compared with the published Xenopus NT-4 sequence (Hallbook, F. et al., Neuron 6, 845-858, (1991). The 760 bp PstI/EcoRI insert of pFRK81 encoding the full-length NT-4 protein was subcloned into PstI/EcoRI-digested pMEX mammalian expression vector [Martin-Zanca, D. et al., Mol. Cell. Biol. 9, 24-33 (1989)] to generate pFRK82. The 760 bp PstI/EcoRI-fragment of pFRK81 was also subcloned into the pVL1392 baculovirus transfer vector [Luckow, V.A. and Summers, M.D., Virology 167, 56-71 (1988)]. The resulting plasmid, pFRK83, was co-transfected with wild-type *Autographa californica* polyhedrosis virus strain E2 (AcNPV) DNA into *Spodoptera frugiperda* (Sf9) insect cells as described [Summers, M.D. and Smith, G.E., Bulletin No. 1555, College Station, Texas: Texas Agrigcultural Experimental Station and Texas A&M University, pp. 10-39 (1987)]. Recombinant baculoviruses capable of expressing XNT-4 protein were isolated by plaque purification.
XNT-4 protein-containing supernatants were harvested after 4 days, clarified and dialyzed at 4°C for 20 hours against 50 volumes of phosphate buffered saline. To express XNT-4 in mammalian COS cells, a 770 bp HindIII-XbaI fragment of pFRK81 was subcloned into CDM7B, a COS cell expression vector that contains the cytomegalovirus enhancer/promoter element and the SV40 polyadenylation signal [Seed, B., Nature 329, 840-842 (1987)]. The resulting plasmid, pFRK84, was transfected into COS cells by the DEAE-dextran/chloroquin method as described [Seed, B. and Aruffo, A., Proc. Natl. Acad. Sci. USA 84, 3365-3369 (1987)]. After 48 hours, cells were placed in DMEM containing 5% calf serum. Supernatants were harvested 5 days later.

D. Binding assay

Purified baculovirus-expressed BDNF and NT-3 were radioiodinated using [$^{125}$I]-labeled Bolton-Hunter reagent (2 mCi per reaction; 4000 Ci/mmol; ICN) as previously described [Rodriguez-Tebar, A. and Barde, Y.A., J. Neurosci. 8, 3337-3342, (1988); See also, Example 1 herein above and Klein, R. et al., Cell 65, 189-197 (1991)]. [$^{125}$I]-labeled $\beta$NGF was obtained from Amersham (1500 Ci/mmol). Competition binding assays were done essentially as described [Jing, S. et al., J. Cell Biol. 110, 283-294 (1990); See also, Example 1 herein above and Klein, R. et al., Cell 65, 189-197 (1991)]. Cells (3 x 10$^5$ per sample) were preincubated at 4°C for 1 hour in binding media (DMEM containing 10 mM Hepes buffer, pH 7.0 and 2.5% calf serum) containing various concentrations of unlabeled purified factors or the indicated dilutions of COS cell supernatants. [$^{125}$I]-labeled neurotrophins were added to each sample to reach a final concentration of 0.1 nM. Cells were further incubated at 4°C for 90 minutes, washed 4 times with 100 $\mu$l of ice-cold DMEM containing 10 mM Hepes pH 7.0 and 0.1% BSA, and lysed in 100 $\mu$l of 1 N NaOH. Cell bound radioactivity was counted in a G5500 Gamma counter (Beckman).

E. Immunoblotting

Detection of phosphorylated tyrosine residues was carried out by Western blot analysis as previously described [ See, Example 1 herein above and Klein, R. et al., Cell 65, 189-197 (1991)]. Cell extracts were immunoprecipitated with rabbit polyclonal antisera raised either against a peptide corresponding to the 14 carboxy-terminal amino acid residues of human gp140$^{trk}$ [Martin-Zanca, D. et al., Mol. Cell. Biol. 9 24-33 (1989)] or against the mouse gp145$^{trkB}$ tyrosine kinase domain expressed in bacteria [Klein, R. et al., Cell 61, 647-656 (1990)]. The resulting immunoprecipitates were fractionated by 7.5% SDS-gel electrophoresis, transferred onto nitrocellulose filters and incubated with the mouse anti-phosphotyrosine monoclonal antibody 4G10 (UBI). Filters were subsequently incubated for 45 minutes with rabbit anti mouse IgG (Dako) before probing with [125 I]-labeled protein A (Dupont).

## II. RESULTS

### A. Xenopus Neurotrophin-4 competes for the binding of BDNF to gp145$^{trkB}$ receptors

In order to determine whether XNT-4 interacts with any of the three known members of the *trk* receptor family, a NT-4 DNA clone was first isolated by PCR-aided amplification of *Xenopus laevis* DNA. The amplified DNA was subcloned into the pCR1000 vector and submitted to nucleotide sequence analysis to verify the fidelity of the amplification process. A selected plasmid, designated pFRK81, was found to possess a XNT-4 DNA insert identical to that reported by [Hallbook, F. et al., Neuron 6, 845-858 (1991)] except for a translationally silent difference at nucleotide 671. This XNT-4 DNA was subsequently used to express the XNT-4 protein in mammalian COS cells. To this end, the XNT-4 DNA insert of pFRK81 was subcloned into the COS expression vector CDM7B [Seed, B., Nature 329, 840-842 (1987)] to generate the XNT-4 expression plasmid pFRK84.

Supernatants derived from COS cells transiently transfected with pFRK84 DNA were tested for their ability to compete with the binding of [125 I]-labeled neurotrophins to their respective receptors. As illustrated in Figure 1, XNT-4-containing COS cell supernatants efficiently displaced [125 I]-BDNF from binding to gp145$^{trkB}$-expressing NIH3T3 cells (Z52-17 cells). In contrast, XNT-4 had no signficiant effect in reducing the binding of [125 I]-NT-3 to NIH3T3 cells expressing gp145$^{trkC}$ (R4-31 cells) or [125 I]-NGF to NIH3T3 cells expressing its cognate gp140$^{trk}$ receptor (E25-42 cells). Control supernatants obtained from COS cells transfected with the CDM7B vector failed to show significant competition in any of the assays tested (Figure 29). These results indicate that NT-4 may specifically interact with gp145$^{trkB}$ receptors.

### B. Xenopus Neurotrophin-4 induces tyrosine phosphorylation of gp145$^{trkB}$ receptors

We next examined whether the interaction of XNT-4 with gp145$^{trkB}$ results in a functional response. Recent studies have shown that the NGF family of neurotrophins can induce the rapid autophosphorylation of their cognate *trk* receptors on tyrosine residues [ See, Example 2 herein above and Cordon-Cardo, C. et al., Cell 66, 173-183 (1991); Kaplan, D.R. et al., Nature 350, 158-160 (1991); Kaplan, D.R. et al., Science 252, 554-558 (1991); Example 1 herein above and Klein, R. et al., Cell 65, 189-197 (1991); Example 3 herein above and Klein, R. et al., Cell 66 395-403 (1991); Example 4 herein above and Lamballe, F. et al., Cell 66, 967-979 (1991); Soppet, D. et al., Cell 65, 895-903 (1991)]. Addition of COS cell supernatants containing the XNT-4 protein to Z52-17 cells expressing gp145$^{trkB}$ induced the rapid phosphorylation of these receptors on tyrosine residues. In contrast, when the same preparation of XNT-4 was added to gp140$^{trk}$-expressing E25-48 cells or to gp145$^{trkC}$-containing R4-31 cells, no XNT-4 induced tyrosine phosphorylation was observed. In control reactions, each of the three *trk* receptors gp140$^{trk}$,gp145$^{trkB}$ and 145$^{trkC}$ became phosphorylated on tyrosine residues when incubated in the presence of their respective cognate ligands NGF, BDNF and NT-3. COS cell supernatants obtained from cells transfected with the CDM7B vector fiailed to induce tyrosine phosphorylation in each of the cell lines used (Figure 30).

In order to provide further support to the concept that XNT-4 is directly responsible for the activation of gp145$^{trkB}$ receptors, XNT-4 protein was prepared in a non-mammalian expression system. To this end, the XNT-4 DNA was subcloned into the pVL1392 baculovirus transfer vector to generate a XNT-4 recombinant baculovirus strain, designated FRK 83-10. Supernatants derived from Sf9 insect cells infected with FRK83-10 were partially purified and added to the corresponding NIH-3T3-derived cell lines expressing each of the three members of the *trk* family of receptors. As shown in Figure 31, only the gp145$^{trkB}$ receptors present in Z52-17 cells became phosphorlyated on tyrosine residues as a response to baculovirus-produced XNT-4. None of the receptors became phosphorylated on tyrosine residues when these NIH3T3-derived cell lines were incubated with control supernatants obtained from Sf9 cells infected with wild type baculovirus. These results indicate that XNT-4 specifically activates gp145$^{trkB}$ receptors.

### C. Co-expression of XNT-4 and gp145$^{trkB}$ causes transformation of NIH3T3 cells

We have previously shown that constitutive activation of the *trk* receptors in NIH3T3 cells results in their morphologic transformation [ See, Example 2 herein above and Cordon-Cardo, C. et al., Cell 66, 173-183 (1991)]. These observations led us to develop simple co-transfection assays which were very instrumental in ascertaining the ligant/receptor interactions among the NGF neurotrophin-family and the known members of the *trk* family of tyrosine protein kinases [ See, Example 2 herein above and Cordon-Cardo, C. et al., Cell 66, 173-183 (1991); Example 3 herein above and Klein, R. et al., Cell 66, 395-403 (1991); Example 4 herein above and Lamballe, F. et al., Cell 66, 967-979 (1991)]. As illustrated in Figure 32, co-transfection of NIH3T3 cells with Moloney sarcoma virus (MSV) LTR-driven mammalian expression plasmids encoding gp140$^{trk}$ (pDM69) and NGF (pLTRSNGF), gp145$^{trkB}$ (pFRK44) and BDNF (pLL42) or gp145$^{trkC}$ and NT-3 (pLL43) results in the rapid (8 to 10 days) and efficient (up to 10$^5$ foci of transformed cells per microgram of DNA) transformation of these cells.

To determine whether XNT-4 may elicit a similar response through any of these receptors, we generated an MSV LTR-driven expression plasmid, pFRK82, by introducing the XNT-4 DNA insert of pFRK81 into the pMEX-*neo* expression vector [Martin-Zanca, D. et al., Mol. Cell. Biol. 9, 24-33 (1989)]. As shown in Figure 32, co-transfection of NIH3T3 cells with saturating amounts of pFRK82 DNA along with expression plasmids encoding gp140$^{trk}$ or gp145$^{trkC}$ receptors did not result in any detectable response. However, when pFRK82 was co-transfected with gp145$^{trkB}$-coding pFRK44 DNA, the appearance of multiple foci of transformed cells was observed (Figure 32).

NIH3T3 transformation assays also provide a quantitative measurement of the biological response elicited when the various neurotrophins interact with their cognate *trk* receptors. For instance, co-transfection of NIH3T3 cells with plasmids encoding gp145$^{trkB}$ (pFRK44) and BDNF (pLL42) induces at least 100 times more foci of transformed cells than when pFRK44 is co-transfected with pLL43, a plasmid encoding NT-3 [ See, Example 3 herein above and Klein, R. et al., Cell 66, 395-403 (1991)]. Therefore, these NIH3T3 transformation assays were utilized to quantitate the extent of the biological response that XNT-4 elicits through the gp145$^{trkB}$ receptor. NIH3T3 cells were co-transfected with microgram amounts of pFRK44 DNA along with various amounts of MSV LTR-driven expression plasmids encoding XNT-4 (pFRK82), BDNF (pLL42), NT-3 (pLL43) and NGF (pLTRSNGF). In agreement with previous observations, co-expression of gp145$^{trkB}$ and NGF did not induce morphologic transformation of the transfected NIH3T3 cells (Figure 33). As indicated above, NT-3 only activated the gp145$^{trkB}$ receptors at high concentrations of plasmid DNA. In contrast, XNT-4 DNA induced a response comparable to that mediated by BDNF (Figure 33). Quantitative analysis of these NIH3T3 transformation assays indicate that XNT-4 possesses a specific transforming activity in NIH3T3 cells expressing gp145$^{trkB}$ receptors of about 3 x 10$^4$ ffu/$\mu$g of DNA. This transforming activity is only five-fold lower than that observed for BDNF and about two orders of magnitude higher than that determined for XNT-3 (Figure 33). Considering that the XNT-4 DNA used in these assays is of *Xenopus* origin, these results suggest that mammalian NT-4 and BDNF may elicit similarly powerful neurotrophic responses through gp145$^{trkB}$ receptors.

D. XNT-4 induces neuronal differentiation of PC12 cells expressing gp145$^{trkB}$ receptors

Exposure of rat pheochromocytoma PC12 cells to NGF induces their differentiation into sympathetic-like neurons, a process characterized by cessation of proliferation and formation of long neuritic outgrowths. In contrast, addition of either BDNF, NT-3 or XNT-4 to these cells does not result in any detectable response, presumably due to the absence of the signal transducing gp145$^{trkB}$ and gp145$^{trkC}$ receptors [Hallbook, F. et al., Neuron 6, 845-858 (1991)]. However, transfection of PC12 cells with DNA encoding gp145$^{trkB}$ renders these cells responsive to BDNF, and to a lesser extent to NT-3 [Squinto, S.P. et al., Cell 65, 885-893 (1991)-]. In order to determine whether XNT-4 could induce neuronal differentiation of PC12 cells expressing gp145$^{trkB}$ receptors, PC12 cells were transfected with the *trk*B plasmid pFRK44. Transfected PC12 cells expressing gp145$^{trkB}$ receptors, DC12/*trk*B cells, were selected by their resistance to G418 followed by their ability to differentiate in response to BDNF. These BDNF-responsive PC12/*trk*B cells were next exposed to various concentrations of XNT-4 produced by transient expression of COS cells. As shown in Figure 34F, a 1:5 dilution of the XNT-4 containing COS supernatant induced the formation of neuritic outgrowths with an efficiency comparable to that observed with similar supernatants containing BDNF (Figure 34E). These neuritic processes were also similar, albeit less abundant, to those induced by NGF through the endogenous gp140$^{trk}$ receptors present in these PC12/*trk*B cells (Figure 34D). As expected, XNT-4 did not elicit any detectable response in wild type PC12 cells which do not express gp145$^{trkB}$ receptors. Finally, control supernatants derived from COS cells transfected with the expression vector CDM7B had no effect on either wild type PC12 or PC12/*trk*B cells (Figure 34C). These results demonstrate that the gp145$^{trkB}$ tyrosine protein kinase can mediate the neurotrophic properties of XNT-4.

E. A point mutation in the extracellular domain of gp145$^{trkB}$ abolishes its activation by NT-4 but not by BDNF

We have previously shown that mutations in the second Ig-like domain of gp140$^{trk}$ [Schneider R. and Schweiger, M. Oncogene 6, 1807-1811 (1991)] induce its ligand-independent activation [Coulier, F. et al., Mol. Cell. Biol. 10, 4202-4210, (1990)], suggesting that this domain may play an important role in mediating the neurotrophic activity of NGF. Based on these observations, a series of similar mutants in the related trkB receptor were generated (unpublished observations). One such mutant, pFL3, carries a single amino acid substitution (Cys$^{345}$--Ser$^{345}$) that replaces one of the ten cysteines conserved among the three known members of the trk gene family [Barbacid, M. et al., BBA Reviews on Cancer 1072, 115-127 (1991)]. Transfection of pFL3 DNA into NIH3T3 cells resulted in the expression of a 145 kDa glycoprotein (along with a 120 kDa precursor proteins at levels comparable to those observed with wild type trkB receptor expression vector pFRK44 (Figure 35). As shown in Table 14, expression of this mutant gp145$^{trkB/S345}$ receptor in NIH3T3 cell, in the absence of neurotrophins, did not induce any detectable levels of morphologic transformation. However, when pFL3 was co-transfected with BDNF-coding pLL42 DNA, the appearance of numerous foci of transformed cells was observed. Quantitative analysis of these transfection assays indicated that the mutant gp145$^{trkB/Ser345}$ protein is about 10 fold less efficient than the wild type gp145$^{trkB}$ receptor in mediateing the biological response of BDNF in these cells (Table 14). These results indicate that the Cys$^{345}$ residue might be involved in, but is not required for the activation of gp145$^{trkB}$ by BDNF. In contrast, co-transfection of pFL3 with the XNT-4-coding pFRK82 expression plasmid failed to elicit any detectable morphologic transformation of NIH3T3 cells (Table 14). Similar results were obtained when co-transfecting pFL3 DNA with the NT-3-expression plasmid pLL43. These observations indicate that the Cys$^{345}$ residue may be required to mediate signal transduction by XNT-4 and NT-3 and suggest that gp145$^{trkB}$ receptors may utilize different domains to interact with their various cognate ligands.

## III. DISCUSSION

Neurotrophin-4, a novel member of the NGF family of neurotrophins, has recently been identified by PCR-aided amplification of Xenopus laevis and viper DNA [Hallbook, F. et al., Neuron 6, 845-858 (1991)]. In the present studies, we report that XNT-4 can exert its neurotrophic properties through gp145$^{trkB}$, a tyrosine protein kinase receptor encoded by the mouse trkB locus. Addition of Xenopus NT-4 (XNT-4) protein, produced in either mammalian COS cells or in baculovirus-infected Sf9 insect cells, induces the rapid phosphorylation on tyrosine residues of gp145$^{trkB}$. However, XNT-4 has no effect on the tyrosine phosphorylation of the related gp140$^{trk}$ and gp145$^{trkC}$ receptors. Moreover, XNT-4 is able to compete with [$^{125}$I]-BDNF for binding to gp145$^{trkB}$, but cannot displace NGF or NT-3 from binding to their primary signal transducing receptors gp140$^{trk}$ and gp145$^{trkC}$, respectively. These results indicate that XNT-4 specifically interacts with and activates gp145$^{trkB}$ receptors. Whether XNT-4 also binds to the non-catalytic trkB receptor, gp95$^{trkB}$ [ See, Example 1 herein above and Klein, R. et al., Cell 61, 647-656 (1991)], remains to be determined.

The activation of gp145$^{trkB}$ receptors by Xenopus NT-4 triggers a flow of signal transduction that results in defined biological responses. Co-expression of plasmids encoding XNT-4 and gp145$^{trkB}$ in NIH3T3 cells leads to their rapid and efficient transformation, presumably by a mechanism involving autocrine activation of the gp145$^{trkB}$ receptors. Quantitative analysis of the transforming effect of XNT-4 on NIH3T3-expressing gp145$^{trkB}$ receptors indicates that this neurotrophin elicits a biological response comparable to that of BDNF and significantly stronger than NT-3 (Figure 36). However, co-expression of XNT-4 with either gp140$^{trk}$ or gp145$^{trkC}$ in these cells does not have any detectable effect. In agreement with previous observations [ See, Example 2 herein above and Cordon-Cardo, C. et al., Cell 66, 173-183 (1991); Example 1 herein above and Klein, R. et al., Cell 65, 189-197 (1991); Example 3 herein above and Klein, R. et al., Cell 66, 395-403 (1991); Glass, D.J. et al., Cell 66, 405-413 (1991)], the transforming activity of NT-4 on gp145$^{trkB}$-expressing NIH3T3 cells does not require the presence of the low affinity NGF receptor, p75$^{LNGFR}$ [Johnson, D. et al., Cell 47, 545-554 (1986); Radeke, M.J. et al., Nature 325, 593-597 (1987)], a protein previously shown to bind XNT-4 [Hallbook, F. et al., Neuron 6, 845-858 (1991)]. The Xenopus NT-4 protein failed to induce neuronal differentiation of PC12 cells, indicating that the endogenous p75$^{LNGFR}$ receptors present in these cells do not mediate the neurotrophic properties of this protein. However, addition of XNT-4 to PC12 cells ectopically expressing gp145$^{trkB}$ receptors led to a block in proliferation, allowed their survival in the absence of serum and induced the formation of neuritic outgrowths. In contrast, addition of XNT-4 to PC12 cells expressing gp145$^{trkC}$ recpetors had no detectable neurotrophic effect (unpublished observations). These findings establish that XNT-4 can mediate at least some of its neurotrophic properties through gp145$^{trkB}$ receptors.

Comparative sequence analysis of Xenopus and viper NT-4 with the other members of the NGF family of neurotrophins has indicated that NT-4 is most closely related to BDNF and more distant to NGF [Hallbook, F. et al., Neuron 6, 845-858 (1991)]. Recent studies have established that the tyrosine protein kinase gp145$^{trkB}$ serves as a primary receptor for BDNF [ See, Example 3 herein above and Klein, R. et al., Cell 66, 395-403 (1991); Soppet, D. et al., Cell 65, 895-903 (1991); Squinto, S.P. et al., Cell 65, 885-893 (1991)]. Moreover, this receptor can mediate some of the biological properties of NT-3 with low efficiency, but it does not recognize NGF [ See, Example 3 herein above and Klein, R. et al., Cell 66, 395-403 (1991); Soppet, D. et al., Cell 65, 895-903 (1991); Squinto, S.P. et al., Cell 65, 885-893 (1991)]. Our findings indicating that gp145$^{trkB}$ also functions as a primary receptor for XNT-4, reveal a close paralellism between the evolutionary relationships of these neurotrophins [Hallbook, F. et al., Neuron 6, 845-858 (1991)] and their relative ability to activate this trkB receptor (Figure 35). Whether mammals contain an as yet unidentified trk-related receptor specific for NT-4, remains to be determined.

We have previously reported that small deletions or miscoding mutations within the second Ig-like domain of the trk proto-oncogene product can activate its transforming properties in a ligand-independent fashion [Coulier, F. et al., Mol. Cell. Biol. 10, 4202-4210 (1990)]. These observations have raised the possibility that this domain might be involved in NGF recognitition. Similar mutations do not activate the related gp145$^{trkB}$ receptor (Lamballe, F. et al., unpublished results). However, replacement of Cys$^{345}$, one of the ten cysteine residues conserved in the three known members of the trk gene family [Barbacid, M. et al., BBA Reviews on Cancer 1072, 115-127 (1991)], selectively affects the ability of gp145$^{trkB}$ to become activated by its three known ligands BDNF, XNT-4 and NT-3. Whereas BDNF efficiently activates the mutant gp145$^{trkBSer345}$ receptor, neither XNT-4 or NT-3 had any effect, at least in NIH3T3 transformation assays. Whether this lack of activity is due to impaired binding or to their ability to activate the tyrosine protein kinase activity of the receptor remains to be determined. In any case, these observations suggest that BDNF and XNT-4 may utilize at least partially distinct domains within the gp145$^{trkB}$ receptor to mediate their respective neurotrophic activities.

Expression studies using Northern blot analysis indicate that NT-4 is expressed at high levels in Xenopus ovaries [Hallbook, F. et al., Neuron 6, 845-858 (1991)]. However, no NT-4-specific transcripts could be detected in other tissues including brain and spinal cord [Hallbook, F. et al., Neuron 6, 845-858 (1991)]. In rodents, trkB is preferentially expressed in the brain [Klein, R. et al., EMBO J. 8, 3701-3709, (1989); Klein, R. et al., Development 109, 845-850 (1990); Middlemas, D.S. et al., Mol. Cell. Biol. 11, 143-153 (1991)]. Moreover, trkB transcripts have been detected in a variety of non-neural tissues, including ovaries [Klein, R. et al., EMBO J. 8, 3701-3709 (1989)]. However, the trkB transcripts found in ovaries encode the non-catalytic receptor gp95$^{trkB}$. The absence of a tyrosine protein kinase domain in gp95$^{trkB}$ makes it unlikely that this receptor will mediate the neurotrophic properties of NT-4. It is possible that ovaries serve as a reservoir of XNT-4 in a fashion reminiscent of the large amounts of NGF found in the submaxillary gland of male mice. If so, gp95$^{trk}$ may play a role in facilitating its transport to other tissues. Moreover, lower levels of XNT-4 mRNA may be expressed in certain specialized structures which would have escaped detection by Northern blot analysis of whole tissues.

Hallbook, F. et al., Neuron 6, 845-858 (1991) have proposed that the NT-4 transcripts found in Xenopus ovaries might be preferentially located in the oocytes and therefore could represent maternal mRNAs. During mouse embryogenesis (stages E6.5 to E17.5), trkB expression can be detected after nine days of gestation, a time that coincides with the onset of neurogenesis [Klein, R. et al., Development 109, 845-850 (1990)]. Therefore, if XNT-4 is indeed expressed during the early stages of embryogenesis, it may play a non-neurotrophic role which may or may not be mediated by gp145$^{trkB}$ receptors. The potential improtance of these observations underscores the need to isolate the mammalian NT-4 gene to determine its precise pattern of expression as well as its range of biological activities.

TABLE 1

| NGF binding to cells expressing the *trk* proto-ongogene | | | | |
|---|---|---|---|---|
| Cell Lines | HIGH AFFINITY RECEPTORS | | LOW AFFINITY RECEPTORS | |
| | Kd (nM) | Sites/Cell $(\times 10^{-3})$ | Kd (nM) | Sites/Cell $(\times 10^{-3})$ |
| PC12 | 0.30 | 17.3 | 33.2 | 109 |
| E25-48 | 0.09 | 20.5 | 2.4 | 204 |
| E25-427 | 0.12 | 28.6 | 7.0 | 1,262 |
| Sf9 (pAcS2) | 0.42 | 31.2 | 13.7 | 61 |
| Summary of the Scatchard plot analysis of the binding of [$^{125}$I]-labeled $\beta$NGF to cell lines expressing the *trk* proto-oncogene (see Figures 4 and 6). The values shown represent the average of two to three experiments. | | | | |

Table 2. Induction of DNA synthesis by NGF.

| ADDITIONS[a] | BROMODEOXYURIDINE INCORPORATION | | | | |
| | NIH3T3 cells | | E25-48 cells | | |
| | Positive/ Total | Percentage Positive | Positive/ Total | Percentage Positive | Percentage positive among gp140$^{trk}$ expressing cells[b] |
|---|---|---|---|---|---|
| None | 4/345 | 1.1% | 22/384 | 5.7% | ND |
| 20% Calf serum | 330/459 | 71.9% | 241/339 | 71.1% | 100% |
| NGF 10 ng/ml | 3/313 | 0.9% | 139/345 | 40.3% | 86% |
| NGF 50 ng/ml | 2/303 | 0.7% | 149/358 | 41.6% | 89% |

a All samples contained 5 µg/ml of insulin.

b 47% of E25-48 cells express detectable levels of gp140$^{trk}$ as determined in the experiment described in Figure 11.

EP 0 504 914 A2

Table 3

| Growth in semi-solid medium of cells expressing the gp140[trk] receptors. | | | | |
|---|---|---|---|---|
| CELL LINE | NUMBER OF CELLS | ADDITIONS[a] | AGAR COLONIES[b] | NGF STIMULATION |
| NIH3T3 | $10^3$ | -- | 1 | |
| | $10^3$ | NGF | 0 | None |
| | $10^4$ | -- | 1 | |
| | $10^4$ | NGF | 2 | None |
| E25-48 | $10^3$ | -- | 12 | |
| | $10^3$ | NGF | 111 | 9-fold |
| | $10^4$ | -- | 79 | |
| | $10^4$ | NGF | 546 | 7-fold |
| B38-94 | $10^3$ | -- | 98 | |
| | $10^3$ | NGF | 90 | None |
| | $10^4$ | -- | 1604 | |
| | $10^4$ | NGF | 1356 | None |

[a]Cultures were fed every four days with 0.5 ml of DMEM containing 5% calf serum in the absence or presence of 30 ng/ml of NGF as indicated.
[b]Colonies containing more than 32 cells were scored as positive after 4 weeks of culture.

Table 4

| NGF confers transforming activity to the *trk* photo-oncogene product | | | | |
|---|---|---|---|---|
| *trk* GENE | PLASMID | AMOUNT OF DNA | TRANSFORMING ACTIVITY[a] (foci/1.5 x $10^5$ cells) | |
| | | | -NGF | +NGF |
| *trk* proto-oncogene | pDM69 | 1 $\mu$g | 0 | TMTC[bc] |
| | | 100 ng | 0 | TMTC |
| | | 10 ng | 0 | 292 |
| | | 1 ng | 0 | 23 |
| *trk*S345 | pRK26 | 100 ng | 62 | TMTC |
| | | 10 ng | 4 | ≧500 |
| | | 1 ng | 0 | 180 |
| | | 100 pg | 0 | 26 |
| *trk* oncogene[c] | pDM16 | 100 ng | TMTC | TMTC |
| | | 1 ng | 120 | 113 |

[a] Foci were scored 12 days after addition of the DNA.
[b] TMTC, too many to count.
[c] *trk* oncogene isolated from a human colon carcinoma biopsy [Martin-Zanca, D. et al., Mol. Cell. Biol. 9, 24-33 (1989)].

Table 5. Mitogenic activity of NT-3.

| ADDITIONS[a] | BROMODEOXYURIDINE INCORPORATION | | | |
| | NIH3T3 cells | | E25-48 cells | |
| | Positive/ Total | Percentage Positive | Positive/ Total | Percentage Positive |
| --- | --- | --- | --- | --- |
| None | 4/179 | 2.2% | 19/305 | 6.2% |
| 20% Calf serum | 270/341 | 79.2% | 274/352 | 77.8% |
| NGF 50 ng/ml | 13/216 | 6.0% | 126/269 | 46.8% |
| BDNF 50 ng/ml | 4/192 | 2.1% | 34/362 | 9.3% |
| NT-3 50 ng/ml | 2/195 | 1.0% | 68/310 | 22.0% |

a All samples contain 5 µg/ml of insulin.

Table 6. Induction of c-*fos* expression by neurotrophic factors in gp140*trk* expressing E25-48 cells.

| ADDITIONS[a] | | Experiment 1 | | Experiment 2 | |
|---|---|---|---|---|---|
| | | Positive/ Total | Percentage Positive | Positive/ Total | Percentage Positive |
| None | | 6/257 | 2.3% | 13/216 | 5.7% |
| 20% Calf serum | | 190/266 | 71.4% | 143/184 | 77.7% |
| NGF | 50 ng/ml | 119/282 | 42.2% | 80/184 | 43.5% |
| BDNF | 50 ng/ml | 12/204 | 5.8% | ND[b] | --- |
| NT-3 | 50 ng/ml | 84/262 | 32.1% | 89/235 | 37.9% |
| NT-3 | 10 ng/ml | ND[b] | --- | 65/191 | 34.1% |

a All samples contain 5 µg/ml of insulin.

b ND, not determined.

EP 0 504 914 A2

Table 7

| Transformation of NIH3T3 cells by co-transfection of expression plasmids encoding the *trk* tyrosine kinase gp140$^{trk}$ and the NGF family of neurotrophic factors. | | | | | |
|---|---|---|---|---|---|
| CO-TRANSFECTED DNAs | | | | | TRANSFORMING ACTIVITY (foci per 1.5x10$^5$ cells) |
| *trk* RECEPTOR | | | NEUROTROPHIC FACTORS | | |
| PLASMID | DNA(ng) | | PLASMID[a] | DNA(ng) | |
| pDM69 | 2000 | | --- | --- | 0 |
| --- | --- | | pLTRSNGF(NGF) | 2000 | 0 |
| pDM69 | 2000 | + | pLTRSNGF(NGF) | 2000 | TMTC[b] |
| pDM69 | 2000 | + | pLTRSNGF(NGF) | 200 | TMTC |
| pDM69 | 2000 | + | pLTRSNGF(NGF) | 20 | 350 |
| pDM69 | 2000 | + | pLTRSNGF(NGF) | 2 | 53 |
| --- | --- | | pLL42(BDNF) | 2000 | 0 |
| pDM69 | 2000 | + | pLL4A2(BDNF) | 2000 | 0 |
| pDM69 | 2000 | + | pLL42(BDNF) | 200 | 0 |
| --- | --- | | pLL43(NT-3) | 2000 | 0 |
| pDM69 | 2000 | + | pLL43(NT-3) | 2000 | 380 |
| pDM69 | 2000 | + | pLL43(NT-3) | 200 | 78 |
| pDM69 | 2000 | + | pLL43(NT-3) | 20 | 6 |
| pDM69 | 2000 | + | pLL43(NT-3) | 2 | 0 |

[a] The neurotrophic factor encoded by each plasmid is indicated in parenthesis.
[b] TMTC, too many to count.

Table 8

| Transformation of NIH3T3 cells by co-transfection of expression plasmids encoding the *trk*B tyrosine kinase gp145$^{trkB}$ and the NGF family of neurotrophic factors. | | | | | |
|---|---|---|---|---|---|
| CO-TRANSFECTED DNAs | | | | | TRANSFORMING ACTIVITY (foci per 1.5x10$^5$ cells) |
| *trkB* RECEPTOR | | | NEUROTROPHIC FACTORS | | |
| PLASMID | DNA(ng) | | PLASMID[a] | DNA(ng) | |
| pFRK44 | 2000 | | --- | --- | 0 |
| --- | --- | | pLTRSNGF(NGF) | 2000 | 0 |
| pFRK44 | 2000 | + | pLTRSNGF(NGF) | 2000 | 0 |
| pFRK44 | 2000 | + | pLTRSNGF(NGF) | 200 | 0 |
| --- | --- | | pLL42(BDNF) | 2000 | 0 |
| pFRK44 | 2000 | + | pLL42(BDNF) | 2000 | TMTC[b] |
| pFRK44 | 2000 | + | pLL42(BDNF) | 200 | TMTC |
| pFRK44 | 2000 | + | pLL42(BDNF) | 20 | >500 |
| pFRK44 | 2000 | + | pLL42(BDNF) | 2 | 80 |
| --- | --- | | pLL43(NT-3) | 2000 | 0 |
| pFRK44 | 2000 | + | pLL43(NT-3) | 2000 | 200 |
| pFRK44 | 2000 | + | pLL43(NT-3) | 200 | 44 |
| pFRK44 | 2000 | + | pLL43(NT-3) | 20 | 6 |
| pFRK44 | 2000 | + | pLL43(NT-3) | 2 | 0 |

a The neurotrophic factor encoded by each plasmid is indicated in parenthesis.
[b] TMTC, too many to count.

TABLE 9

| Transforming activities of neurotrophic factors in the presence of *trk* and *trk*B DNAs. | | | |
|---|---|---|---|
| RECEPTOR KINASE | | NEUROTROPHIC FACTOR | TRANSFORMING ACTIVITY (ffu per $\mu$g of DNA) |
| *trk* | | -- | <10$^0$ |
| *trk* | + | NGF | 2x10$^4$ |
| *trk* | + | BDNF | <10$^0$ |
| *trk* | + | NT-3 | 3x10$^2$ |
| *trk*B | | -- | <10$^0$ |
| *trk*B | + | NGF | <10$^0$ |
| *trk*B | + | BDNF | 4x10$^4$ |
| *trk*B | + | NT-3 | 1x10$^2$ |
| [a] Transforming activites were calculated from data depicted in Tables 7 and 8. Values were extrapolated from the transformation efficiencies of neurotrophic factor DNAs in the linear dose/response range (1 to 250 foci/1.5 x 10$^5$ transfected NIH3T3 cells/10 cm plate). | | | |

Table 10

| Mitogenic activity of BDNF and NT-3 on gp145$^{trkB}$-expressing Z52-17 cells. | | | | |
|---|---|---|---|---|
| ADDITIONS[a] | BROMODEOXYURIDINE INCORPORATION | | | |
| | NIH3T3 cells | | Z52-17 cells | |
| | Positive/Total | Percentage Positive | Positive/Total | Percentage Positive |
| None | 16/421 | 3.8% | 41/374 | 10.9% |
| 20% Calf serum | 525/630 | 83.3% | 532/602 | 88.3% |
| BDNF (COS cells) | 30/254 | 11.8% | 186/289 | 64.3% |
| BDNF (Sf9 cells) | 20/396 | 5.0% | 151/447 | 33.7% |
| NT-3 (COS cells) | 31/349 | 8.8% | 97/206 | 47.0% |
| NT-3 (Sf9 cells) | 19/393 | 4.8% | 186/576 | 32.2% |
| NGF 50 ng/ml | 13/226 | 5.7% | 39/280 | 13.9% |

[a] All samples contain 5 $\mu$g/ml of insulin.

Table 11

| Induction of DNA synthesis by NT-3. | | | | |
|---|---|---|---|---|
| ADDITIONS[a] | BROMODEOXYURIDINE INCORPORATION | | | |
| | NIH3T3 cells | | R4-31 cells | |
| | Positive/Total | Percentage Positive | Positive/Total | Percentage Positive |
| None | 5/467 | 1.1% | 4/475 | 0.8% |
| 20% Calf serum | 298/318 | 93.7% | 274/301 | 91.0% |
| NGF 50 ng/ml | 14/458 | 3.0% | 27/489 | 5.5% |
| BDNF 50 ng/ml | 27/474 | 5.7% | 49/520 | 11.3% |
| NT-3 50 ng/ml | 16/513 | 3.1% | 345/526 | 65.6% |

[a] All samples contain 5 $\mu$g/ml of insulin. NGF was purchased from Upstate Biotechnology Inc. BDNF and NT-3 were purified from baculovirus-infected Sf9 cells as previously described (See Example 2).

Table 12

| Transformation of NIH3T3 cells by co-transfection of expression plasmids encoding gp145$^{trkC}$ receptors and the NGF family of neurotrophic factors. | | | |
|---|---|---|---|
| CO-TRANSFECTION OF pFL20 DNA[a] WITH | | | TRANSFORMING ACTIVITY (foci per 1.5x10$^5$ cells) |
| NEUROTROPHIC FACTOR | PLASMID | DNA(ng) | |
| ----- | ----- | ----- | 0 |
| NGF | pLTRSNGF | 2000 | 0 |
| | pLTRSNGF | 200 | 0 |
| BDNF | pLL42 | 2000 | 0 |
| | pLL42 | 200 | 0 |
| NT-3 | pLL43 | 2000 | TMTC[b] |
| | pLL43 | 200 | TMTC |
| | pLL43 | 20 | ≧300 |
| | pLL43 | 2 | 20 |

[a] Two micrograms of pFL20 DNA were used in each case.
[b] TMTC, too many to count.

Table 13

| Transformation of NIH3T3 cells by co-transfection of expression plasmids encoding NT-3 and the *trk* family of tyrosine kinase receptors. | | | |
|---|---|---|---|
| CO-TRANSFECTION OF pLL43 DNA[a] WITH | | | TRANSFORMING ACTIVITY (foci per 1.5x10$^5$ cells) |
| RECEPTOR | PLASMID | DNA(ng) | |
| ----- | ----- | ----- | 0 |
| gp140$^{trk}$ | pDM69 | 1000 | TMTC[b] |
| | | 100 | 260 |
| | | 10 | 35 |
| | | 1 | 3 |
| gp145$^{trkB}$ | pFRK44 | 1000 | TMTC |
| | | 100 | 139 |
| | | 10 | 15 |
| | | 1 | 2 |
| gp145$^{trkC}$ | pFL20 | 1000 | TMTC |
| | | 100 | TMTC |
| | | 10 | 244 |
| | | 1 | 122 |
| | | 0.1 | 16 |

[a] One microgram of pLL43 DNA was used in each case.
[b] TMTC, too many to count.

Table 14.  A single miscoding mutation in the extracellular domain of the gp145$^{trkB}$ receptor abolishes its activation by XNT-4 and NT-3, but not by BDNF

| NEUROTROPHIC FACTOR DNA[a] | TRANSFORMING ACTIVITY [a] (Foci per $10^5$ cells) | | | |
| --- | --- | --- | --- | --- |
| | pFRK44(gp145$^{trkB}$) | | pFl3(gp145$^{trkBSer345}$) | |
| | Exp 1 | Exp 2 | Exp 1 | Exp 2 |
| None | 0 | 0 | 0 | 0 |
| pLL42 (BDNF) | | | | |
| 100 ng | >500 | >500 | 264 | 175 |
| 10 ng | 218 | 134 | 50 | 32 |
| 1 ng | 34 | 80 | 1 | 3 |
| pLL43(NT-3) | | | | |
| 100 ng | ND[b] | 192 | 0 | 0 |
| 10 ng | ND | 9 | 0 | 0 |
| 1 ng | ND | 0 | 0 | 0 |
| pFRK82(XNT-4) | | | | |
| 100 ng | >500 | >500 | 1 | 0 |
| 10 ng | 54 | 82 | 0 | 0 |
| 1 ng | 0 | 14 | 0 | 0 |

[a]NIH3T3 cells were co-transfected with 1 µg of expression plasmid encoding either pFRK44 or pFL3 and the indicated amounts of neurotrophic factor DNA as described in Experimental Procedures herein above. Foci of transformed cells were scored after 14 days of mutation.
[b] Not Done.

45

SEQUENCE LISTING

(1) INFORMATION FOR SEQ ID NO:1:

    (i) SEQUENCE CHARACTERISTICS:
      (A) LENGTH: 2526 base pairs
      (B) TYPE: nucleic acid
      (C) STRANDEDNESS: double
      (D) TOPOLOGY: linear

    (ii) MOLECULE TYPE: cDNA

    (iii) HYPOTHETICAL: NO

    (ix) FEATURE:
      (A) NAME/KEY: CDS
      (B) LOCATION: 32..2506

(xi) SEQUENCE DESCRIPTION: SEQ ID NO:1:

```
CGGGCTCCGA TAACCGAAGC AGCGATCGGA G ATG GAT GTC TCT CTT TGC CCA          52
                                    Met Asp Val Ser Leu Cys Pro
                                     1               5

GCC AAG TGT AGT TTC TGG CGG ATT TTC TTG CTG GGA AGC GTC TGG CTG        100
Ala Lys Cys Ser Phe Trp Arg Ile Phe Leu Leu Gly Ser Val Trp Leu
         10              15              20

GAC TAT GTG GGC TCC GTG CTG GCT TGC CCT GCA AAT TGT GTC TGC AGC        148
Asp Tyr Val Gly Ser Val Leu Ala Cys Pro Ala Asn Cys Val Cys Ser
         25              30              35

AAG ACT GAG ATC AAT TGC CGG CGG CCG GAC GAT GGG AAC CTC TTC CCC        196
Lys Thr Glu Ile Asn Cys Arg Arg Pro Asp Asp Gly Asn Leu Phe Pro
 40              45              50              55

CTC CTG GAA GGG CAG GAT TCA GGG AAC AGC AAT GGG AAT GCC AGC ATC        244
Leu Leu Glu Gly Gln Asp Ser Gly Asn Ser Asn Gly Asn Ala Ser Ile
                 60              65              70

AAC ATC ACG GAC ATC TCA AGG AAT ATC ACT TCC ATA CAC ATA GAG AAC        292
Asn Ile Thr Asp Ile Ser Arg Asn Ile Thr Ser Ile His Ile Glu Asn
             75              80              85

TGG CGC GGT CTG CAC ACG CTC AAC GCT GTG GAC ATG GAG CTC TAC ACC        340
Trp Arg Gly Leu His Thr Leu Asn Ala Val Asp Met Glu Leu Tyr Thr
         90              95              100

GGC CTC CAG AAG CTG ACC ATC AAG AAC TCA GGA CTT CGG AGC ATC CAG        388
Gly Leu Gln Lys Leu Thr Ile Lys Asn Ser Gly Leu Arg Ser Ile Gln
     105             110             115

CCC AGA GCC TTT GCC AAG AAC CCC CAC CTG CGC TAC ATA AAC CTG TCG        436
Pro Arg Ala Phe Ala Lys Asn Pro His Leu Arg Tyr Ile Asn Leu Ser
120             125             130             135

AGT AAC CGG CTC ACC ACA CTC TCA TGG CAG CTC TTC CAG ACG CTG AGT        484
Ser Asn Arg Leu Thr Thr Leu Ser Trp Gln Leu Phe Gln Thr Leu Ser
             140             145             150

CTT CGG GAA TTG AGA TTG GAG CAG AAC TTC TTC AAC TGC AGC TGT GAC        532
Leu Arg Glu Leu Arg Leu Glu Gln Asn Phe Phe Asn Cys Ser Cys Asp
             155             160             165

ATC CGC TGG ATG CAG CTG TGG CAG GAG CAG GGG GAG GCC AAG CTG AAC        580
Ile Arg Trp Met Gln Leu Trp Gln Glu Gln Gly Glu Ala Lys Leu Asn
         170             175             180

AGC CAG AGC CTC TAT TGC ATC AGT GCC GAT GGC TCC CAG CTC CCC CTC        628
Ser Gln Ser Leu Tyr Cys Ile Ser Ala Asp Gly Ser Gln Leu Pro Leu
     185             190             195
```

```
TTC CGC ATG AAC ATT AGC CAG TGT GAC CTT CCT GAG ATC AGT GTG AGC        676
Phe Arg Met Asn Ile Ser Gln Cys Asp Leu Pro Glu Ile Ser Val Ser
200             205             210             215

CAC GTC AAT CTG ACC GTT CGG GAG GGT GAC AAT GCT GTT GTC ACC TGC        724
His Val Asn Leu Thr Val Arg Glu Gly Asp Asn Ala Val Val Thr Cys
                220             225             230

AAT GGC TCT GGA TCA CCC CTG CCC GAC GTG GAC TGG ATC GTC ACT GGA        772
Asn Gly Ser Gly Ser Pro Leu Pro Asp Val Asp Trp Ile Val Thr Gly
                235             240             245

CTG CAG TCC ATC AAC ACC CAC CAG ACA AAT CTG AAT TGG ACC AAC GTA        820
Leu Gln Ser Ile Asn Thr His Gln Thr Asn Leu Asn Trp Thr Asn Val
                250             255             260

CAC GCC ATC AAC CTG ACA CTG GTC AAT GTG ACG AGT GAG GAC AAC GGC        868
His Ala Ile Asn Leu Thr Leu Val Asn Val Thr Ser Glu Asp Asn Gly
    265             270             275

TTC ACC CTG ACG TGC ATT GCA GAG AAC GTG GTG GGC ATG AGC AAT GCC        916
Phe Thr Leu Thr Cys Ile Ala Glu Asn Val Val Gly Met Ser Asn Ala
280             285             290             295

AGC GTC GCC CTC ACT GTT CAC TAC CCC CCA CGA GTG GTG AGC CTG GAG        964
Ser Val Ala Leu Thr Val His Tyr Pro Pro Arg Val Val Ser Leu Glu
                300             305             310

GAG CCA GAG CTG CGC CTG GAA CAC TGC ATC GAG TTT GTG GTG CGT GGC        1012
Glu Pro Glu Leu Arg Leu Glu His Cys Ile Glu Phe Val Val Arg Gly
                315             320             325

AAC CCG CCG CCC ACG CTG CAC TGG CTG CAC AAC GGG CAG CCG CTG CGT        1060
Asn Pro Pro Pro Thr Leu His Trp Leu His Asn Gly Gln Pro Leu Arg
                330             335             340

GAG TCC AAG ATC ACC CAC GTG GAG TAC TAC CAG GAG GGC GAG GTC TCC        1108
Glu Ser Lys Ile Thr His Val Glu Tyr Tyr Gln Glu Gly Glu Val Ser
                345             350             355

GAG GGC TGC CTG CTC TTC AAC AAG CCC ACC CAC TAC AAC AAT GGC AAC        1156
Glu Gly Cys Leu Leu Phe Asn Lys Pro Thr His Tyr Asn Asn Gly Asn
360             365             370             375

TAC ACA CTC AAT CGC CAA GAA CCC CTT GGC ACA GCC AAC CAG ACC ATC        1204
Tyr Thr Leu Asn Arg Gln Glu Pro Leu Gly Thr Ala Asn Gln Thr Ile
                380             385             390

AAT GGC CAC TTC CTC AAG GAG CCT TTT CCA GAG AGC ACG GAT AAC TTT        1252
Asn Gly His Phe Leu Lys Glu Pro Phe Pro Glu Ser Thr Asp Asn Phe
                395             400             405
```

```
GTC TCT TTC TAT GAA GTG AGC CCC ACC CCT CCC ATC ACT GTG ACG CAC        1300
Val Ser Phe Tyr Glu Val Ser Pro Thr Pro Pro Ile Thr Val Thr His
        410             415             420

AAG CCA GAG GAA GAT ACA TTT GGG GTA TCC ATA GCT GTT GGA CTT GCC        1348
Lys Pro Glu Glu Asp Thr Phe Gly Val Ser Ile Ala Val Gly Leu Ala
        425             430             435

GCT TTT GCC TGT GTC CTT CTG GTG GTT CTC TTT ATC ATG ATC AAC AAG        1396
Ala Phe Ala Cys Val Leu Leu Val Val Leu Phe Ile Met Ile Asn Lys
440             445             450             455

TAT GGT CGA CGG TCT AAA TTT GGA ATG AAG GGT CCT GTG GCT GTC ATC        1444
Tyr Gly Arg Arg Ser Lys Phe Gly Met Lys Gly Pro Val Ala Val Ile
            460             465             470

AGT GGT GAA GAG GAC TCA GCC AGC CCA CTG CAT CAC GAT CAA CCA TGG        1492
Ser Gly Glu Glu Asp Ser Ala Ser Pro Leu His His Asp Gln Pro Trp
        475             480             485

CAT CAC CAC ACC CTC ATC ACT GGA CGC CGG GCC GGA CAC AGT GTC ATT        1540
His His His Thr Leu Ile Thr Gly Arg Arg Ala Gly His Ser Val Ile
        490             495             500

GGC ATG ACC CGC ATC CCA GTC ATT GAG AAC CCC CAG TAC TTC CGC CAG        1588
Gly Met Thr Arg Ile Pro Val Ile Glu Asn Pro Gln Tyr Phe Arg Gln
        505             510             515

GGA CAC AAC TGC CAC AAG CCA GAC ACG TAT GTG CAG CAC ATT AAA AGG        1636
Gly His Asn Cys His Lys Pro Asp Thr Tyr Val Gln His Ile Lys Arg
520             525             530             535

AGG GAC ATC GTG CTG AAG CGA GAA CTG GGT GAG GGA GCC TTT GGG AAG        1684
Arg Asp Ile Val Leu Lys Arg Glu Leu Gly Glu Gly Ala Phe Gly Lys
            540             545             550

GTC TTC CTG GCC GAG TGC TAC AAC CTC AGC CCC ACC AAG GTC AAG ATG        1732
Val Phe Leu Ala Glu Cys Tyr Asn Leu Ser Pro Thr Lys Val Lys Met
            555             560             565

CTC GTG GCT GTG AAG GCC CTG AAG GAT CCC ACC CTG GCC GCC CGG AAG        1780
Leu Val Ala Val Lys Ala Leu Lys Asp Pro Thr Leu Ala Ala Arg Lys
            570             575             580

GAT TTC CAG AGG GAG GCT GAG CTG CTC ACC AAC CTG CAG CAT GAG CAC        1828
Asp Phe Gln Arg Glu Ala Glu Leu Leu Thr Asn Leu Gln His Glu His
        585             590             595

ATT GTC AAG TTC TAT GGG GTG TGC GGC GAC GGG GAC CCA CTC ATC ATG        1876
Ile Val Lys Phe Tyr Gly Val Cys Gly Asp Gly Asp Pro Leu Ile Met
600             605             610             615
```

```
GTT TTT GAG TAC ATG AAA CAC GGG GAT CTG AAC AAG TTC CTC AGG GCC        1924
Val Phe Glu Tyr Met Lys His Gly Asp Leu Asn Lys Phe Leu Arg Ala
                620                 625                 630

CAT GGG CCA GAT GCC ATG ATC CTC GTG GAC GGC CAG CCA CGC CAG GCA        1972
His Gly Pro Asp Ala Met Ile Leu Val Asp Gly Gln Pro Arg Gln Ala
                635                 640                 645

AAA GGC GAG CTG GGG CTC TCC CAG ATG CTG CAC ATT GCC AGT CAG ATC        2020
Lys Gly Glu Leu Gly Leu Ser Gln Met Leu His Ile Ala Ser Gln Ile
            650                 655                 660

TGC TCT GGC ATG GTG TAC CTG GCC TCC CAG CAT TTT GTG CAC CGG GAC        2068
Cys Ser Gly Met Val Tyr Leu Ala Ser Gln His Phe Val His Arg Asp
        665                 670                 675

CTG GCC ACC AGG AAC TGC CTG GTT GGA GCC AAC CTG CTG GTG AAG ATT        2116
Leu Ala Thr Arg Asn Cys Leu Val Gly Ala Asn Leu Leu Val Lys Ile
680                 685                 690                 695

GGC GAT TTC GGC ATG TCC AGA GAT GTC TAC AGC ACG GAT TAC TAC AGG        2164
Gly Asp Phe Gly Met Ser Arg Asp Val Tyr Ser Thr Asp Tyr Tyr Arg
                700                 705                 710

GTA GGA GGA CAC ACC ATG CTC CCA ATT CGC TGG ATG CCT CCT GAA AGC        2212
Val Gly Gly His Thr Met Leu Pro Ile Arg Trp Met Pro Pro Glu Ser
                715                 720                 725

ATC ATG TAC CGG AAG TTC ACT ACT GAG AGT GAC GTG TGG AGC TTC GGG        2260
Ile Met Tyr Arg Lys Phe Thr Thr Glu Ser Asp Val Trp Ser Phe Gly
            730                 735                 740

GTG ATC CTC TGG GAC ATC TTC ACC TAC GGA AAG CAG CCA TGG TTC CAA        2308
Val Ile Leu Trp Asp Ile Phe Thr Tyr Gly Lys Gln Pro Trp Phe Gln
        745                 750                 755

CTC TCA AAC ACA GAG GTC ATT GAG TGC ATC ACC CAA GGT CGC GTT TTG        2356
Leu Ser Asn Thr Glu Val Ile Glu Cys Ile Thr Gln Gly Arg Val Leu
760                 765                 770                 775

GAA CGG CCC CGG GTC TGC CCC AAA GAG GTG TAT GAT GTC ATG CTG GGG        2404
Glu Arg Pro Arg Val Cys Pro Lys Glu Val Tyr Asp Val Met Leu Gly
                780                 785                 790

TGC TGG CAG AGG GAA CCG CAG CAG CGG CTG AAC ATC AAG GAA ATC TAC        2452
Cys Trp Gln Arg Glu Pro Gln Gln Arg Leu Asn Ile Lys Glu Ile Tyr
            795                 800                 805

AAA ATC CTC CAT GCT TTG GGG AAA GCC ACC CCC ATC TAC CTG GAC ATC        2500
Lys Ile Leu His Ala Leu Gly Lys Ala Thr Pro Ile Tyr Leu Asp Ile
            810                 815                 820

CTT GGC TAGCGGTGGC CGGTGGTCAC                                          2526
Leu Gly
825
```

(2) INFORMATION FOR SEQ ID NO:2:

    (i) SEQUENCE CHARACTERISTICS:
        (A) LENGTH: 825 amino acids
        (B) TYPE: amino acid
        (D) TOPOLOGY: linear

    (ii) MOLECULE TYPE: protein

    (xi) SEQUENCE DESCRIPTION: SEQ ID NO:2:

```
Met Asp Val Ser Leu Cys Pro Ala Lys Cys Ser Phe Trp Arg Ile Phe
 1               5                  10                  15

Leu Leu Gly Ser Val Trp Leu Asp Tyr Val Gly Ser Val Leu Ala Cys
            20                  25                  30

Pro Ala Asn Cys Val Cys Ser Lys Thr Glu Ile Asn Cys Arg Arg Pro
            35                  40                  45

Asp Asp Gly Asn Leu Phe Pro Leu Leu Glu Gly Gln Asp Ser Gly Asn
        50                  55                  60

Ser Asn Gly Asn Ala Ser Ile Asn Ile Thr Asp Ile Ser Arg Asn Ile
65                  70                  75                  80

Thr Ser Ile His Ile Glu Asn Trp Arg Gly Leu His Thr Leu Asn Ala
                85                  90                  95

Val Asp Met Glu Leu Tyr Thr Gly Leu Gln Lys Leu Thr Ile Lys Asn
            100                 105                 110

Ser Gly Leu Arg Ser Ile Gln Pro Arg Ala Phe Ala Lys Asn Pro His
        115                 120                 125

Leu Arg Tyr Ile Asn Leu Ser Ser Asn Arg Leu Thr Thr Leu Ser Trp
        130                 135                 140

Gln Leu Phe Gln Thr Leu Ser Leu Arg Glu Leu Arg Leu Glu Gln Asn
145                 150                 155                 160

Phe Phe Asn Cys Ser Cys Asp Ile Arg Trp Met Gln Leu Trp Gln Glu
            165                 170                 175

Gln Gly Glu Ala Lys Leu Asn Ser Gln Ser Leu Tyr Cys Ile Ser Ala
            180                 185                 190

Asp Gly Ser Gln Leu Pro Leu Phe Arg Met Asn Ile Ser Gln Cys Asp
        195                 200                 205
```

```
Leu Pro Glu Ile Ser Val Ser His Val Asn Leu Thr Val Arg Glu Gly
    210             215             220

Asp Asn Ala Val Val Thr Cys Asn Gly Ser Gly Ser Pro Leu Pro Asp
225             230             235                 240

Val Asp Trp Ile Val Thr Gly Leu Gln Ser Ile Asn Thr His Gln Thr
            245             250             255

Asn Leu Asn Trp Thr Asn Val His Ala Ile Asn Leu Thr Leu Val Asn
            260             265             270

Val Thr Ser Glu Asp Asn Gly Phe Thr Leu Thr Cys Ile Ala Glu Asn
    275             280             285

Val Val Gly Met Ser Asn Ala Ser Val Ala Leu Thr Val His Tyr Pro
    290             295             300

Pro Arg Val Val Ser Leu Glu Glu Pro Glu Leu Arg Leu Glu His Cys
305             310             315             320

Ile Glu Phe Val Val Arg Gly Asn Pro Pro Pro Thr Leu His Trp Leu
            325             330             335

His Asn Gly Gln Pro Leu Arg Glu Ser Lys Ile Thr His Val Glu Tyr
            340             345             350

Tyr Gln Glu Gly Glu Val Ser Glu Gly Cys Leu Leu Phe Asn Lys Pro
            355             360             365

Thr His Tyr Asn Asn Gly Asn Tyr Thr Leu Asn Arg Gln Glu Pro Leu
    370             375             380

Gly Thr Ala Asn Gln Thr Ile Asn Gly His Phe Leu Lys Glu Pro Phe
385             390             395             400

Pro Glu Ser Thr Asp Asn Phe Val Ser Phe Tyr Glu Val Ser Pro Thr
            405             410             415

Pro Pro Ile Thr Val Thr His Lys Pro Glu Glu Asp Thr Phe Gly Val
            420             425             430

Ser Ile Ala Val Gly Leu Ala Ala Phe Ala Cys Val Leu Leu Val Val
        435             440             445

Leu Phe Ile Met Ile Asn Lys Tyr Gly Arg Arg Ser Lys Phe Gly Met
    450             455             460

Lys Gly Pro Val Ala Val Ile Ser Gly Glu Glu Asp Ser Ala Ser Pro
465             470             475             480
```

```
Leu His His Asp Gln Pro Trp His His His Thr Leu Ile Thr Gly Arg
            485             490                 495

Arg Ala Gly His Ser Val Ile Gly Met Thr Arg Ile Pro Val Ile Glu
            500             505                 510

Asn Pro Gln Tyr Phe Arg Gln Gly His Asn Cys His Lys Pro Asp Thr
            515             520                 525

Tyr Val Gln His Ile Lys Arg Arg Asp Ile Val Leu Lys Arg Glu Leu
            530             535                 540

Gly Glu Gly Ala Phe Gly Lys Val Phe Leu Ala Glu Cys Tyr Asn Leu
545             550             555                 560

Ser Pro Thr Lys Val Lys Met Leu Val Ala Val Lys Ala Leu Lys Asp
                565             570                 575

Pro Thr Leu Ala Ala Arg Lys Asp Phe Gln Arg Glu Ala Glu Leu Leu
            580             585                 590

Thr Asn Leu Gln His Glu His Ile Val Lys Phe Tyr Gly Val Cys Gly
            595             600                 605

Asp Gly Asp Pro Leu Ile Met Val Phe Glu Tyr Met Lys His Gly Asp
610             615             620

Leu Asn Lys Phe Leu Arg Ala His Gly Pro Asp Ala Met Ile Leu Val
625             630             635                 640

Asp Gly Gln Pro Arg Gln Ala Lys Gly Glu Leu Gly Leu Ser Gln Met
            645             650                 655

Leu His Ile Ala Ser Gln Ile Cys Ser Gly Met Val Tyr Leu Ala Ser
            660             665                 670

Gln His Phe Val His Arg Asp Leu Ala Thr Arg Asn Cys Leu Val Gly
            675             680                 685

Ala Asn Leu Leu Val Lys Ile Gly Asp Phe Gly Met Ser Arg Asp Val
            690             695             700

Tyr Ser Thr Asp Tyr Tyr Arg Val Gly Gly His Thr Met Leu Pro Ile
705             710             715                 720

Arg Trp Met Pro Pro Glu Ser Ile Met Tyr Arg Lys Phe Thr Thr Glu
            725             730                 735

Ser Asp Val Trp Ser Phe Gly Val Ile Leu Trp Asp Ile Phe Thr Tyr
            740             745             750

Gly Lys Gln Pro Trp Phe Gln Leu Ser Asn Thr Glu Val Ile Glu Cys
            755             760             765

Ile Thr Gln Gly Arg Val Leu Glu Arg Pro Arg Val Cys Pro Lys Glu
            770             775             780

Val Tyr Asp Val Met Leu Gly Cys Trp Gln Arg Glu Pro Gln Gln Arg
785             790             795                 800

Leu Asn Ile Lys Glu Ile Tyr Lys Ile Leu His Ala Leu Gly Lys Ala
            805             810             815

Thr Pro Ile Tyr Leu Asp Ile Leu Gly
            820             825
```

53

(2) INFORMATION FOR SEQ ID NO:3:

    (i) SEQUENCE CHARACTERISTICS:
        (A) LENGTH: 2376 base pairs
        (B) TYPE: nucleic acid
        (C) STRANDEDNESS: double
        (D) TOPOLOGY: linear

    (ii) MOLECULE TYPE: cDNA

   (iii) HYPOTHETICAL: NO

    (ix) FEATURE:
        (A) NAME/KEY: CDS
        (B) LOCATION: 1..2184

    (xi) SEQUENCE DESCRIPTION: SEQ ID NO:3:

```
AAT TCT GGA CTC CGG AAC ATC CAG CCC AGA GCC TTC GCC AAG AAC CCC          48
Asn Ser Gly Leu Arg Asn Ile Gln Pro Arg Ala Phe Ala Lys Asn Pro
 1               5                  10                  15

CAC TTG CGT TAT ATA AAC TTG TCA AGT AAC CGG CTC ACC ACA CTC TCC          96
His Leu Arg Tyr Ile Asn Leu Ser Ser Asn Arg Leu Thr Thr Leu Ser
            20                  25                  30

TGG CAG CTC TTC CAG ACG CTG AGC CTT CGG GAA TTG AGA CTG GAG CAG         144
Trp Gln Leu Phe Gln Thr Leu Ser Leu Arg Glu Leu Arg Leu Glu Gln
        35                  40                  45

AAC TTC TTC AAC TGC AGC TGT GAC ATC CGC TGG ATG CAG CTG TGG CAG         192
Asn Phe Phe Asn Cys Ser Cys Asp Ile Arg Trp Met Gln Leu Trp Gln
    50                  55                  60

GAA CAG GGG GAG GCG CGG CTG GAC AGC CAG AGC CTT TAC TGC ATC AGT         240
Glu Gln Gly Glu Ala Arg Leu Asp Ser Gln Ser Leu Tyr Cys Ile Ser
65                  70                  75                  80

GCT GAT GGC TCC CAA CTC CCC CTC TTC CGC ATG AAC ATC AGT CAG TGT         288
Ala Asp Gly Ser Gln Leu Pro Leu Phe Arg Met Asn Ile Ser Gln Cys
                85                  90                  95

GAT CTC CCA GAG ATC AGT GTG AGC CAC GTC AAC CTG ACT GTC CGA GAA         336
Asp Leu Pro Glu Ile Ser Val Ser His Val Asn Leu Thr Val Arg Glu
                100                 105                 110

GGA GAC AAT GCC GTG ATC ACT TGC AAT GGC TCT GGC TCT CCT TTG CCT         384
Gly Asp Asn Ala Val Ile Thr Cys Asn Gly Ser Gly Ser Pro Leu Pro
            115                 120                 125
```

```
GAT GTG GAC TGG ATA GTC ACT GGG CTG CAG TCC ATC AAC ACC CAC CAG        432
Asp Val Asp Trp Ile Val Thr Gly Leu Gln Ser Ile Asn Thr His Gln
    130             135             140

ACC AAT CTG AAC TGG ACC AAT GTA CAT GCC ATC AAC TTG ACC CTG GTG        480
Thr Asn Leu Asn Trp Thr Asn Val His Ala Ile Asn Leu Thr Leu Val
145             150             155             160

AAC GTG ACG AGC GAG GAC AAT GGC TTC ACC CTG ACG TGC ATT GCA GAG        528
Asn Val Thr Ser Glu Asp Asn Gly Phe Thr Leu Thr Cys Ile Ala Glu
            165             170             175

AAC GTG GTG GGC ATG AGC AAT GCC AGT GTT GCT CTC ACT GTC TAC TAC        576
Asn Val Val Gly Met Ser Asn Ala Ser Val Ala Leu Thr Val Tyr Tyr
        180             185             190

CCT CCA CGT GTG GTG AGC CTG GTG GAG CCT GAG GTA CGC CTG GAA CAT        624
Pro Pro Arg Val Val Ser Leu Val Glu Pro Glu Val Arg Leu Glu His
        195             200             205

TGC ATT GAG TTT GTG GTG CGT GGC AAC CCG ACA CCC ACG CTC CAC TGG        672
Cys Ile Glu Phe Val Val Arg Gly Asn Pro Thr Pro Thr Leu His Trp
    210             215             220

CTG TAC AAT GGA CAG CCA TTG AGG GAG TCC AAG ATC ATT CAC ATG GAC        720
Leu Tyr Asn Gly Gln Pro Leu Arg Glu Ser Lys Ile Ile His Met Asp
225             230             235             240

TAC TAC CAG GAG GGG GAG GTC TCA GAG GGC TGC CTG CTC TTC AAC AAG        768
Tyr Tyr Gln Glu Gly Glu Val Ser Glu Gly Cys Leu Leu Phe Asn Lys
            245             250             255

CCC ACC CAC TAC AAC AAT GGC AAC TAC ACC CTC ATT GCT AAG AAT GCC        816
Pro Thr His Tyr Asn Asn Gly Asn Tyr Thr Leu Ile Ala Lys Asn Ala
            260             265             270

CTG GGC ACG GCC AAC CAG ACC ATC AAC GGC CAC TTC CTG AAG GAG CCC        864
Leu Gly Thr Ala Asn Gln Thr Ile Asn Gly His Phe Leu Lys Glu Pro
        275             280             285

TTT CCA GAG AGC ACA GAT TTC TTT GAC TTT GAG TCT GAT GCG AGC CCT        912
Phe Pro Glu Ser Thr Asp Phe Phe Asp Phe Glu Ser Asp Ala Ser Pro
        290             295             300

ACA CCT CCT ATC ACT GTG ACC CAC AAA CCA GAG GAA GAC ACT TTT GGG        960
Thr Pro Pro Ile Thr Val Thr His Lys Pro Glu Glu Asp Thr Phe Gly
305             310             315             320

GTG TCC ATA GCA GTC GGA CTT GCT GCC TTT GCC TGC GTC CTT CTG GTG       1008
Val Ser Ile Ala Val Gly Leu Ala Ala Phe Ala Cys Val Leu Leu Val
            325             330             335

GTT CTC TTT ATC ATG ATC AAC AAG TAT GGT CGC CGG TCC AAA TTT GGA       1056
Val Leu Phe Ile Met Ile Asn Lys Tyr Gly Arg Arg Ser Lys Phe Gly
            340             345             350
```

55

```
ATG AAG GGT CCT GTG GCT GTT ATC AGT GGA GAG GAG GAC TCA GCC AGC          1104
Met Lys Gly Pro Val Ala Val Ile Ser Gly Glu Glu Asp Ser Ala Ser
        355             360             365

CCA CTG CAT CAC GAT CAA CCA TGG CAT CAC TAC ACC ATC ATC GTT GGA          1152
Pro Leu His His Asp Gln Pro Trp His His Tyr Thr Ile Ile Val Gly
        370             375             380

TGC TGG GCC GTA CAC GTG GTC ATT GGC ATG ACC CGC ATC CCA GTC ATT          1200
Cys Trp Ala Val His Val Val Ile Gly Met Thr Arg Ile Pro Val Ile
385             390             395                 400

GAG AAC CCC CAG TAC TTC CGT CAG GGT CAC AAT TGC CAC AAG CCA GAC          1248
Glu Asn Pro Gln Tyr Phe Arg Gln Gly His Asn Cys His Lys Pro Asp
                405             410             415

ACA TAT GTT CAG CAC ATC AAG AGG AGA GAC ATC GTG TTG AAG AGA GAA          1296
Thr Tyr Val Gln His Ile Lys Arg Arg Asp Ile Val Leu Lys Arg Glu
                420             425             430

TTG GGT GAG GGA GCC TTT GGG AAG GTC TTC CTG GCT GAG TGC TAC AAT          1344
Leu Gly Glu Gly Ala Phe Gly Lys Val Phe Leu Ala Glu Cys Tyr Asn
        435             440             445

CTA AGC CCC ACC AAA GAC AAG ATG CTA GTG GCA GTG AAG GCC CTG AAG          1392
Leu Ser Pro Thr Lys Asp Lys Met Leu Val Ala Val Lys Ala Leu Lys
        450             455             460

GAT CCC ACC TTG GCT GCC AGG AAG GAT TTC CAG AGG GAG GCT GAG CTG          1440
Asp Pro Thr Leu Ala Ala Arg Lys Asp Phe Gln Arg Glu Ala Glu Leu
465             470             475                 480

CTC ACG AAC CTG CAG CAT GAG CAT ATT GTC AAG TTC TAT GGG GTG TGT          1488
Leu Thr Asn Leu Gln His Glu His Ile Val Lys Phe Tyr Gly Val Cys
                485             490             495

GGT GAT GGT GAC CCA CTC ATC ATG GTC TTT GAA TAC ATG AAG CAT GGA          1536
Gly Asp Gly Asp Pro Leu Ile Met Val Phe Glu Tyr Met Lys His Gly
            500             505             510

GAC CTT AAC AAG TTC CTC AGG GCC CAT GGG CCA GAT GCC ATG ATC CTC          1584
Asp Leu Asn Lys Phe Leu Arg Ala His Gly Pro Asp Ala Met Ile Leu
            515             520             525

GTG GAT GGA CAG CCA CGT CAG GCC AAG GGG GAG CTA GGG CTC TCT CAG          1632
Val Asp Gly Gln Pro Arg Gln Ala Lys Gly Glu Leu Gly Leu Ser Gln
        530             535             540

ATG CTC CAC ATC GCC AGT CAG ATA GCC TCG GGC ATG GTG TAC CTG GCT          1680
Met Leu His Ile Ala Ser Gln Ile Ala Ser Gly Met Val Tyr Leu Ala
545             550             555             560
```

56

```
TCC CAG CAC TTT GTA CAC CGG GAC CTG GCC ACG AGG AAC TGC CTG GTT          1728
Ser Gln His Phe Val His Arg Asp Leu Ala Thr Arg Asn Cys Leu Val
            565             570                 575

GGA GCC AAT CTA CTA GTG AAG ATT GGA GAT TTT GGC ATG TCC AGG GAC          1776
Gly Ala Asn Leu Leu Val Lys Ile Gly Asp Phe Gly Met Ser Arg Asp
            580             585                 590

GTC TAC AGT ACT GAT TAC TAC AGG CTC TTT AAT CCA TCT GGA AAT GAT          1824
Val Tyr Ser Thr Asp Tyr Tyr Arg Leu Phe Asn Pro Ser Gly Asn Asp
            595             600                 605

TTT TGT ATA TGG TGT GAG GTG GGA GGA CAC ACC ATG CTC CCC ATC CGC          1872
Phe Cys Ile Trp Cys Glu Val Gly Gly His Thr Met Leu Pro Ile Arg
            610             615                 620

TGG ATG CCC CCT GAA AGC ATA ATG TAC CGG AAG TTC ACC ACA GAG AGT          1920
Trp Met Pro Pro Glu Ser Ile Met Tyr Arg Lys Phe Thr Thr Glu Ser
625             630                 635                 640

GAT GTC TGG AGC TTC GGG GTT ATT CTT TGG GAG ATC TTT ACC TAT GGG          1968
Asp Val Trp Ser Phe Gly Val Ile Leu Trp Glu Ile Phe Thr Tyr Gly
            645             650                 655

AAG CAA CCA TGG TTC CAG CTT TCC AAC ACG GAG GTC ATT GAA TGC ATC          2016
Lys Gln Pro Trp Phe Gln Leu Ser Asn Thr Glu Val Ile Glu Cys Ile
            660             665                 670

ACC CAA GGC CGT GTC TTG GAG AGA CCC AGA GTC TGC CCT AAA GAA GTG          2064
Thr Gln Gly Arg Val Leu Glu Arg Pro Arg Val Cys Pro Lys Glu Val
            675             680                 685

TAT GAT GTC ATG CTG GGG TGC TGG CAG AGG GAA CCA CAG CAG CGG CTG          2112
Tyr Asp Val Met Leu Gly Cys Trp Gln Arg Glu Pro Gln Gln Arg Leu
            690             695                 700

AAT ATT AAG GAG ATC TAC AAA ATC CTC CAT GCT TTG GGG AAG GCC ACC          2160
Asn Ile Lys Glu Ile Tyr Lys Ile Leu His Ala Leu Gly Lys Ala Thr
705             710                 715                 720

CCG ATC TAC CTG GAC ATT CTT GGC TAGTGGTGAC TGGTGGCCAA GCATTTATAC        2214
Pro Ile Tyr Leu Asp Ile Leu Gly
            725

TCTGTTGCCT CCTCTCTCCC TGCTTCCTTT CCTCTTTTTC CTCATCTCAA CTCCTTTCTT       2274

CCATTTTTGA CGGAAACGAA CATCTTCATA TAAACTCAAG TGCCTGCTAC ACATACAACA       2334

CTGAATTTAA ACAAAACAAA ACAAAAAAAA AAAAGGAATT CC                          2376
```

(2) INFORMATION FOR SEQ ID NO:4:

   (i) SEQUENCE CHARACTERISTICS:
     (A) LENGTH: 728 amino acids
     (B) TYPE: amino acid
     (D) TOPOLOGY: linear

   (ii) MOLECULE TYPE: protein

   (xi) SEQUENCE DESCRIPTION: SEQ ID NO:4:

```
Asn Ser Gly Leu Arg Asn Ile Gln Pro Arg Ala Phe Ala Lys Asn Pro
 1               5                  10                  15

His Leu Arg Tyr Ile Asn Leu Ser Ser Asn Arg Leu Thr Thr Leu Ser
            20                  25                  30

Trp Gln Leu Phe Gln Thr Leu Ser Leu Arg Glu Leu Arg Leu Glu Gln
        35                  40                  45

Asn Phe Phe Asn Cys Ser Cys Asp Ile Arg Trp Met Gln Leu Trp Gln
    50                  55                  60

Glu Gln Gly Glu Ala Arg Leu Asp Ser Gln Ser Leu Tyr Cys Ile Ser
65                  70                  75                  80

Ala Asp Gly Ser Gln Leu Pro Leu Phe Arg Met Asn Ile Ser Gln Cys
                85                  90                  95

Asp Leu Pro Glu Ile Ser Val Ser His Val Asn Leu Thr Val Arg Glu
            100                 105                 110

Gly Asp Asn Ala Val Ile Thr Cys Asn Gly Ser Gly Ser Pro Leu Pro
            115                 120                 125

Asp Val Asp Trp Ile Val Thr Gly Leu Gln Ser Ile Asn Thr His Gln
    130                 135                 140

Thr Asn Leu Asn Trp Thr Asn Val His Ala Ile Asn Leu Thr Leu Val
145                 150                 155                 160

Asn Val Thr Ser Glu Asp Asn Gly Phe Thr Leu Thr Cys Ile Ala Glu
                165                 170                 175

Asn Val Val Gly Met Ser Asn Ala Ser Val Ala Leu Thr Val Tyr Tyr
            180                 185                 190

Pro Pro Arg Val Val Ser Leu Val Glu Pro Glu Val Arg Leu Glu His
        195                 200                 205

Cys Ile Glu Phe Val Val Arg Gly Asn Pro Thr Pro Thr Leu His Trp
    210                 215                 220
```

```
Leu Tyr Asn Gly Gln Pro Leu Arg Glu Ser Lys Ile Ile His Met Asp
225                 230             235                     240

Tyr Tyr Gln Glu Gly Glu Val Ser Glu Gly Cys Leu Leu Phe Asn Lys
                245             250                     255

Pro Thr His Tyr Asn Asn Gly Asn Tyr Thr Leu Ile Ala Lys Asn Ala
            260             265                     270

Leu Gly Thr Ala Asn Gln Thr Ile Asn Gly His Phe Leu Lys Glu Pro
            275             280                 285

Phe Pro Glu Ser Thr Asp Phe Phe Asp Phe Glu Ser Asp Ala Ser Pro
        290             295                 300

Thr Pro Pro Ile Thr Val Thr His Lys Pro Glu Glu Asp Thr Phe Gly
305                 310             315                     320

Val Ser Ile Ala Val Gly Leu Ala Ala Phe Ala Cys Val Leu Leu Val
                325             330                 335

Val Leu Phe Ile Met Ile Asn Lys Tyr Gly Arg Arg Ser Lys Phe Gly
            340             345                 350

Met Lys Gly Pro Val Ala Val Ile Ser Gly Glu Glu Asp Ser Ala Ser
            355                 360                 365

Pro Leu His His Asp Gln Pro Trp His His Tyr Thr Ile Ile Val Gly
        370             375                 380

Cys Trp Ala Val His Val Val Ile Gly Met Thr Arg Ile Pro Val Ile
385                 390             395                     400

Glu Asn Pro Gln Tyr Phe Arg Gln Gly His Asn Cys His Lys Pro Asp
                405             410                 415

Thr Tyr Val Gln His Ile Lys Arg Arg Asp Ile Val Leu Lys Arg Glu
            420             425                 430

Leu Gly Glu Gly Ala Phe Gly Lys Val Phe Leu Ala Glu Cys Tyr Asn
        435                 440             445

Leu Ser Pro Thr Lys Asp Lys Met Leu Val Ala Val Lys Ala Leu Lys
        450             455                 460

Asp Pro Thr Leu Ala Ala Arg Lys Asp Phe Gln Arg Glu Ala Glu Leu
465                 470                 475                     480

Leu Thr Asn Leu Gln His Glu His Ile Val Lys Phe Tyr Gly Val Cys
                485             490                 495
```

```
Gly Asp Gly Asp Pro Leu Ile Met Val Phe Glu Tyr Met Lys His Gly
        500                 505             510

Asp Leu Asn Lys Phe Leu Arg Ala His Gly Pro Asp Ala Met Ile Leu
        515                 520             525

Val Asp Gly Gln Pro Arg Gln Ala Lys Gly Glu Leu Gly Leu Ser Gln
    530                 535             540

Met Leu His Ile Ala Ser Gln Ile Ala Ser Gly Met Val Tyr Leu Ala
545                 550             555                 560

Ser Gln His Phe Val His Arg Asp Leu Ala Thr Arg Asn Cys Leu Val
            565                 570             575

Gly Ala Asn Leu Leu Val Lys Ile Gly Asp Phe Gly Met Ser Arg Asp
            580                 585             590

Val Tyr Ser Thr Asp Tyr Tyr Arg Leu Phe Asn Pro Ser Gly Asn Asp
        595                 600             605

Phe Cys Ile Trp Cys Glu Val Gly Gly His Thr Met Leu Pro Ile Arg
        610                 615             620

Trp Met Pro Pro Glu Ser Ile Met Tyr Arg Lys Phe Thr Thr Glu Ser
625                 630             635                 640

Asp Val Trp Ser Phe Gly Val Ile Leu Trp Glu Ile Phe Thr Tyr Gly
            645                 650             655

Lys Gln Pro Trp Phe Gln Leu Ser Asn Thr Glu Val Ile Glu Cys Ile
            660                 665             670

Thr Gln Gly Arg Val Leu Glu Arg Pro Arg Val Cys Pro Lys Glu Val
            675                 680             685

Tyr Asp Val Met Leu Gly Cys Trp Gln Arg Glu Pro Gln Gln Arg Leu
        690                 695             700

Asn Ile Lys Glu Ile Tyr Lys Ile Leu His Ala Leu Gly Lys Ala Thr
705                 710             715                 720

Pro Ile Tyr Leu Asp Ile Leu Gly
            725
```

EP 0 504 914 A2

(2) INFORMATION FOR SEQ ID NO:5:

    (i) SEQUENCE CHARACTERISTICS:
        (A) LENGTH: 34 base pairs
        (B) TYPE: nucleic acid
        (C) STRANDEDNESS: single
        (D) TOPOLOGY: linear

    (ii) MOLECULE TYPE: DNA

    (xi) SEQUENCE DESCRIPTION: SEQ ID NO:5:

GTGGTCTGCA GTCGACTAAG TAATGATCCT CCGC

(2) INFORMATION FOR SEQ ID NO:6:

    (i) SEQUENCE CHARACTERISTICS:
        (A) LENGTH: 39 base pairs
        (B) TYPE: nucleic acid
        (C) STRANDEDNESS: single
        (D) TOPOLOGY: linear

    (ii) MOLECULE TYPE: DNA

    (xi) SEQUENCE DESCRIPTION: SEQ ID NO:6:

CAGGTCTAGA GAGAATTCTC TTCTCTCTAT TTTGCTAAC

## Claims

1. A method for detecting an agonist or an antagonist of a neurotrophic factor comprising:
   (a) contacting a *trk* family receptor with the neurotrophic factor and a test substance which may contain the agonist or antagonist; and
   (b) determining whether the agonist or antagonist competes with the neurotrophic factor in binding to the *trk* family receptor.

2. A method for detecting an agonist or an antagonist of a neurotrophic factor comprising:
   (a) contacting a *trk* family receptor with a test substance which may contain the agonist or antagonist; and
   (b) determining whether the agonist or antagonist binds to the *trk* family receptor.

3. A method for detecting an agonist of a neurotrophic factor comprising:
   (a) contacting a *trk* family receptor with a test substance which may contain the agonist; and
   (b) determining whether the test substance activates one or more biological activities of the *trk* family receptor.

4. A method for detecting an antagonist of a neurotrophic factor comprising:
   (a) contacting a *trk* family receptor with a neurotrophic factor and a test substance which may contain the antagonist; and
   (b) determining whether the test substance affects the activation by the neurotropic factor of one or more of the biological activities of the *trk* receptor.

5. The method according to Claims 3 or 4 wherein the biological activity is tyrosine kinase activity,

61

phosphhorylation of tyrosine residues, the transient expression of c-Fos proteins, the induction of DNA synthesis, the stimulation of cells to enter the S phase, the growth of cells in semi-solid media or the ability to morphologically transform cells.

6. The method according to any one of Claims 1 to 5 wherein the *trk* family receptor is recombinantly produced.

7. The method according to any one of Claims 1 to 6 wherein the *trk* family receptor is gp140$^{trk}$, gp145$^{trkB}$ or gp145$^{trkC}$.

8. The method according to any one of Claims 1 to 7 wherein the neutrophic factor is nerve growth factor, neutrophin-3, neurotrophin-4 or brain derived neurotrophic factor.

9. A method for detecting an agonist or an antagonist of nerve growth factor comprising:
   (a) contacting a *trk* receptor with nerve growth factor and a test substance which contains the agonist or antagonist; and
   (b) determining whether the agonist or antagonist or antagonist competes with nerve growth factor in binding to the *trk* receptor.

10. A kit comprising at least a *trk* family receptor and/or a neutrophic factor for detecting an agonist or an antagonist of a neutrophic factor according to the method of any one of claims 1 to 9.

FIG. 1

FIG. 2

# FIG. 3

EP 0 504 914 A2

FIG. 4 A

PC12

NGF Bound/NGF Free

NGF Bound (x 10⁻⁴ nmol)

FIG. 4 B

E25-427

NGF Bound/NGF Free

NGF Bound (x 10⁻⁴ nmol)

FIG.4C

E25-48

NGF Bound/NGF Free

NGF Bound (x 10⁻⁴ nmol)

**FIG. 5**

**FIG. 6**

FIG. 7

FIG. 8

**FIG. 9A**

**FIG. 9B**

FIG.10A

FIG.10B

FIG.10C

FIG.10D

FIG.10E

FIG.10F

FIG. I I A

FIG. I I B

FIG. I I C

FIG. I I D

FIG. 12

NEUROTROPHIC FACTOR (ng/ml)

PERCENTAGE OF [$^{125}$I] NGF BOUND

FIG.13A　　FIG.13B　　FIG.13C

FIG.14A  FIG.14B  FIG.14C  FIG.14D

FIG. I 5A

FIG. I 5B

FIG. I 5C

FIG. I 5D

FIG. 16A          FIG. 16B

PC12          E25-48

EP 0 504 914 A2

FIG. I 7A

CONTROLS
(2 μg)

—     *trk*B     BDNF     NT-3

**BRAIN-DERIVED NEUROTROPHIC FACTOR**

2 μg     200 ng     20 ng     2 ng

FIG. I 7B

*trk*B +
(2 μg)

2 μg     200 ng     20 ng     2 ng

**NEUROTROPHIN - 3**

FIG. 18A    FIG. 18B    FIG. 18C    FIG. 18D

a  b       a  b  c  d       a  b  c  d       a  c  e

gp140 $^{trk}$ →

← gp145 $^{trkB}$

◁

EP 0 504 914 A2

## FIG. I 9A

## FIG. I 9B

FIG.20A

FIG.20B

FIG.20C

FIG.20D

FIG.20E

FIG.20F

FIG.20G

FIG.20H

FIG.20I

FIG.20J

FIG. 21

FIG. 22

SP      TM      TK

ATG                     TAG

amino acid      1          200          400          600          800
residues

kbp  0                    1                    2

FIG. 23 A

# FIG. 23B

```
   1  CGGGCTCCGATAACCGAAGCAGCGATCGGAGATGGATGTCTCTCTTTGCCCAGCCAAGTG    60
                                     M  D  V  S  L  C  P  A  K  C   10

  61  TAGTTTCTGGCGGATTTTCTTGCTGGGAAGCGTCTGGCTGGACTATGTGGGCTCCGTGCT   120
      S  F  W  R  I  F  L  L  G  S  V  W  L  D  Y  V  G  S  V  L    30

 121  GGCTTGCCCTGCAAATTGTGTCTGCAGCAAGACTGAGATCAATTGCCGGCGGCCGGACGA   180
      A  C  P  A  N  C  V  C  S  K  T  E  I  N  C  R  R  P  D  D    50

 181  TGGGAACCTCTTCCCCCTCCTGGAAGGGCAGGATTCAGGGAACAGCAATGGGAATGCCAG   240
      G  N  L  F  P  L  L  E  G  Q  D  S  G  N  S  N  G  N  A  S    70

 241  CATCAACATCACGGACATCTCAAGGAATATCACTTCCATACACATAGAGAACTGGCGCGG   300
      I  N  I  T  D  I  S  R  N  I  T  S  I  H  I  E  N  W  R  G    90

 301  TCTGCACACGCTCAACGCTGTGGACATGGAGCTCTACACCGGCCTCCAGAAGCTGACCAT   360
      L  H  T  L  N  A  V  D  M  E  L  Y  T  G  L  Q  K  L  T  I   110

 361  CAAGAACTCAGGACTTCGGAGCATCCAGCCCAGAGCCTTTGCCAAGAACCCCCACCTGCG   420
      K  N  S  G  L  R  S  I  Q  P  R  A  F  A  K  N  P  H  L  R   130

 421  CTACATAAACCTGTCGAGTAACCGGCTCACCACACTCTCATGGCAGCTCTTCCAGACGCT   480
      Y  I  N  L  S  S  N  R  L  T  T  L  S  W  Q  L  F  Q  T  L   150

 481  GAGTCTTCGGGAATTGAGATTGGAGCAGAACTTCTTCAACTGCAGCTGTGACATCCGCTG   540
      S  L  R  E  L  R  L  E  Q  N  F  F  N  C  S  C  D  I  R  W   170

 541  GATGCAGCTGTGGCAGGAGCAGGGGGGAGGCCAAGCTGAACAGCCAGAGCCTCTATTGCAT   600
      M  Q  L  W  Q  E  Q  G  E  A  K  L  N  S  Q  S  L  Y  C  I   190

 601  CAGTGCCGATGGCTCCCAGCTCCCCCTCTTCCGCATGAACATTAGCCAGTGTGACCTTCC   660
      S  A  D  G  S  Q  L  P  L  F  R  M  N  I  S  Q  C  D  L  P   210

 661  TGAGATCAGTGTGAGCCACGTCAATCTGACCGTTCGGGAGGGTGACAATGCTGTTGTCAC   720
      E  I  S  V  S  H  V  N  L  T  V  R  E  G  D  N  A  V  V  T   230

 721  CTGCAATGGCTCTGGATCACCCCTGCCCGACGTGGACTGGATCGTCACTGGACTGCAGTC   780
      C  N  G  S  G  S  P  L  P  D  V  D  W  I  V  T  G  L  Q  S   250

 781  CATCAACACCCACCAGACAAATCTGAATTGGACCAACGTACACGCCATCAACCTGACACT   840
      I  N  T  H  Q  T  N  L  N  W  T  N  V  H  A  I  N  L  T  L   270

 841  GGTCAATGTGACGAGTGAGGACAACGGCTTCACCCTGACGTGCATTGCAGAGAACGTGGT   900
      V  N  V  T  S  E  D  N  G  F  T  L  T  C  I  A  E  N  V  V   290

 901  GGGCATGAGCAATGCCAGCGTCGCCCTCACTGTTCACTACCCCCCACGAGTGGTGAGCCT   960
      G  M  S  N  A  S  V  A  L  T  V  H  Y  P  P  R  V  V  S  L   310

 961  GGAGGAGCCAGAGCTGCGCCTGGAACACTGCATCGAGTTTGTGGTGCGTGGCAACCCGCC  1020
      E  E  P  E  L  R  L  E  H  C  I  E  F  V  V  R  G  N  P  P   330

1021  GCCCACGCTGCACTGGCTGCACAACGGGCAGCCGCTGCGTGAGTCCAAGATCACCCACGT  1080
      P  T  L  H  W  L  H  N  G  Q  P  L  R  E  S  K  I  T  H  V   350

1081  GGAGTACTACCAGGAGGGCGAGGTCTCCGAGGGCTGCCTGCTCTTCAACAAGCCCACCCA  1140
      E  Y  Y  Q  E  G  E  V  S  E  G  C  L  L  F  N  K  P  T  H   370

1141  CTACAACAATGGCAACTACACACTCAATCGCCAAGAACCCCTTGGCACAGCCAACCAGAC  1200
      Y  N  N  G  N  Y  T  L  N  R  Q  E  P  L  G  T  A  N  Q  T   390

1201  CATCAATGGCCACTTCCTCAAGGAGCCTTTTCCAGAGAGCACGGATAACTTTGTCTCTTT  1260
      I  N  G  H  F  L  K  E  P  F  P  E  S  T  D  N  F  V  S  F   410

1261  CTATGAAGTGAGCCCCACCCCTCCCATCACTGTGACGCACAAGCCAGAGGAAGATACATT  1320
      Y  E  V  S  P  T  P  P  I  T  V  T  H  K  P  E  E  D  T  F   430
```

```
1321 TGGGGTATCCATAGCTGTTGGACTTGCCGCTTTTGCCTGTGTCCTTCTGGTGGTTCTCTT 1380
      G  V  S  I  A  V  G  L  A  A  F  A  C  V  L  L  V  V  L  F    450

1381 TATCATGATCAACAAGTATGGTCGACGGTCTAAATTTGGAATGAAGGGTCCTGTGGCTGT 1440
      I  M  I  N  K  Y  G  R  R  S  K  F  G  M  K  G  P  V  A  V    470

1441 CATCAGTGGTGAAGAGGACTCAGCCAGCCCACTGCATCACGATCAACCATGGCATCACCA 1500
      I  S  G  E  E  D  S  A  S  P  L  H  H  D  Q  P  W  H  H  H    490

1501 CACCCTCATCACTGGACGCCGGGCCGGACACAGTGTCATTGGCATGACCCGCATCCCAGT 1560
      T  L  I  T  G  R  R  A  G  H  S  V  I  G  M  T  R  I  P  V    510

1561 CATTGAGAACCCCCAGTACTTCCGCCAGGGACACAACTGCCACAAGCCAGACACGTATGT 1620
      I  E  N  P  Q  Y  F  R  Q  G  H  N  C  H  K  P  D  T  Y  V    530

1621 GCAGCACATTAAAAGGAGGGACATCGTGCTGAAGCGAGAACTGGGTGAGGGAGCCTTTGG 1680
      Q  H  I  K  R  R  D  I  V  L  K  R  E  L  G  E  G  A  F  G    550

1681 GAAGGTCTTCCTGGCCGAGTGCTACAACCTCAGCCCCACCAAGGTCAAGATGCTCGTGGC 1740
      K  V  F  L  A  E  C  Y  N  L  S  P  T  K  V  K  M  L  V  A    570

1741 TGTGAAGGCCCTGAAGGATCCCACCCTGGCCGCCCGGAAGGATTTCCAGAGGGAGGCTGA 1800
      V  K  A  L  K  D  P  T  L  A  A  R  K  D  F  Q  R  E  A  E

1801 GCTGCTCACCAACCTGCAGCATGAGCACATTGTCAAGTTCTATGGGGTGTGCGGCGACGG 1860
      L  L  T  N  L  Q  H  E  H  I  V  K  F  Y  G  V  C  G  D  G    610

1861 GGACCCACTCATCATGGTTTTTGAGTACATGAAACACGGGGATCTGAACAAGTTCCTCAG 1920
      D  P  L  I  M  V  F  E  Y  M  K  H  G  D  L  N  K  F  L  R    630

1921 GGCCCATGGGCCAGATGCCATGATCCTCGTGGACGGCCAGCCACGCCAGGCAAAAGGCGA 1980
      A  H  G  P  D  A  M  I  L  V  D  G  Q  P  R  Q  A  K  G  E    650

1981 GCTGGGGCTCTCCCAGATGCTGCACATTGCCAGTCAGATCTGCTCTGGCATGGTGTACCT 2040
      L  G  L  S  Q  M  L  H  I  A  S  Q  I  C  S  G  M  V  Y  L    670

2041 GGCCTCCCAGCATTTTGTGCACCGGGACCTGGCCACCAGGAACTGCCTGGTTGGAGCCAA 2100
      A  S  Q  H  F  V  H  R  D  L  A  T  R  N  C  L  V  G  A  N    690

2101 CCTGCTGGTGAAGATTGGCGATTTCGGCATGTCCAGAGATGTCTACAGCACGGATTACTA 2160
      L  L  V  K  I  G  D  F  G  M  S  R  D  V  Y  S  T  D  Y  Y    710

2161 CAGGGTAGGAGGACACACCATGCTCCCAATTCGCTGGATGCCTCCTGAAAGCATCATGTA 2220
      R  V  G  G  H  T  M  L  P  I  R  W  M  P  P  E  S  I  M  Y    730

2221 CCGGAAGTTCACTACTGAGAGTGACGTGTGGAGCTTCGGGGTGATCCTCTGGGAGATCTT 2280
      R  K  F  T  T  E  S  D  V  W  S  F  G  V  I  L  W  E  I  F    750

2281 CACCTACGGAAAGCAGCCATGGTTCCAACTCTCAAACACAGAGGTCATTGAGTGCATCAC 2340
      T  Y  G  K  Q  P  W  F  Q  L  S  N  T  E  V  I  E  C  I  T    770

2341 CCAAGGTCGCGTTTTGGAACGGCCCCGGGTCTGCCCCAAAGAGGTGTATGATGTCATGCT 2400
      Q  G  R  V  L  E  R  P  R  V  C  P  K  E  V  Y  D  V  M  L    790

2401 GGGGTGCTGGCAGAGGGAACCGCAGCAGCGGCTGAACATCAAGGAAATCTACAAAATCCT 2460
      G  C  W  Q  R  E  P  Q  Q  R  L  N  I  K  E  I  Y  K  I  L    810

2461 CCATGCTTTGGGGAAAGCCACCCCCATCTACCTGGACATCCTTGGCTAGCGGTGGCCGGT 2520
      H  A  L  G  K  A  T  P  I  Y  L  D  I  L  G  ...             825

2521 GGTCAC 2526
```

TK.

TK

**FIG. 23C**

85

FIG. 24

FIG. 25

FIG. 26

FIG. 27

FIG.28A

FIG.28B

FIG.28C

## FIG. 29 A

### $^{125}$I-NGF

**E25-42**

**(3T3/*trk*)**

## FIG. 29 B

### $^{125}$I-BDNF

**Z52-17**

**(3T3/*trk*B)**

## FIG. 29 C

### $^{125}$I-NT-3

**R4-31**

**(3T3/*trk*C)**

FIG.30A      FIG.30B      FIG.30C

Control (cos)   NGF   XNT-4 (cos)     Control (cos)   BDNF   XNT-4 (cos)     Control (cos)   NT-3   XNT-4 (cos)

200K —

gp140*trk* →

$\longleftarrow$ gp145*trk*B, gp145*trk*C

97K —

69K —

46K —

E25-48
(3T3/*trk*)      Z52-17
(3T3/*trk*B)      R4-31
(3T3/*trk*C)

EP 0 504 914 A2

FIG.31A     FIG.31B     FIG.31C

Control (bac)   NGF   XNT-4 (bac)    Control (bac)   BDNF   BDNF (bac)   XNT-4 (bac)    Control (bac)   NT-3   XNT-4 (bac)

200K —

gp140 *trk* →

97K —

69K —

46K —

← gp145*trk*B, gp145*trk*C

E25-42
(3T3/*trk*)

Z52-17
(3T3/*trk*B)

R4-31
(3T3/*trk*C)

EP 0 504 914 A2

FIG. 32

FIG. 33

PC12            PC12 + XNT-4            PC12/trkB + Control sup

FIG.34A           FIG.34B           FIG.34C

PC12/trkB + NGF       PC12/trkB + BDNF       PC12/trkB + XNT-4

FIG.34D           FIG.34E           FIG.34F

## FIG.35A    FIG.35B

P   I    P   I

200K —

gp145 *trkB* ➝

97K —

69K —

46K —

gp145 *trkB/S345*

FIG. 36